(19) Europäisches Patentamt
European Patent Office
Office européen des brevets

(11) EP 1 364 026 B1

(12) # EUROPEAN PATENT SPECIFICATION

(45) Date of publication and mention of the grant of the patent:
**07.05.2008 Bulletin 2008/19**

(21) Application number: **02719843.1**

(22) Date of filing: **15.02.2002**

(51) Int Cl.:
***C12N 15/12*** *(2006.01)* ***C07K 14/705*** *(2006.01)*
***C12N 15/63*** *(2006.01)* ***C12Q 1/68*** *(2006.01)*
***G01N 33/50*** *(2006.01)* ***C12N 15/62*** *(2006.01)*
***G01N 33/53*** *(2006.01)* ***C07K 16/28*** *(2006.01)*
***C12N 15/11*** *(2006.01)* ***A61K 31/7088*** *(2006.01)*
***A61K 39/395*** *(2006.01)*

(86) International application number:
**PCT/EP2002/001606**

(87) International publication number:
**WO 2002/072823 (19.09.2002 Gazette 2002/38)**

(54) **HUMAN G PROTEIN-COUPLED RECEPTOR**

MENSCHLICHER G-PROTEIN GEKOPPELTER REZEPTOR

RECEPTEUR COMPLEXE A LA PROTEINE G HUMAIN

(84) Designated Contracting States:
**AT BE CH CY DE DK ES FI FR GB GR IE IT LI LU MC NL PT SE TR**

(30) Priority: **20.02.2001 US 269410 P**
**19.09.2001 US 323809 P**

(43) Date of publication of application:
**26.11.2003 Bulletin 2003/48**

(73) Proprietor: **Bayer HealthCare AG**
**51368 Leverkusen (DE)**

(72) Inventor: **SMOLYAR, Alex**
**Brookline, MA 02467 (US)**

(56) References cited:
**WO-A-00/64942** **WO-A-01/77325**

- **THE SANGER CENTRE (UK) AND THE WASHINGTON UNIVERSITY GENOME SEQUENCING CENTER (USA): "Toward a complete human genome sequence" GENOME RESEARCH, COLD SPRING HARBOR LABORATORY PRESS, US, vol. 8, no. 11, November 1998 (1998-11), pages 1097-1108, XP002169000 ISSN: 1088-9051 -& DATABASE NCBI [Online] 8 November 2000 (2000-11-08) Database accession no. AC021016 XP002232674**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 364 026 B1

## Description

This application claims the benefit of and incorporates by reference co-pending provisional application Serial No. 60/274,233 filed March 9, 2001.

## TECHNICAL FIELD OF THE INVENTION

The invention relates to the area of G protein-coupled receptors. More particularly, it relates to the area of human G protein-coupled receptor and its regulation.

## BACKGROUND OF THE INVENTION

### *G Protein-Coupled Receptors*

Many medically significant biological processes are mediated by signal transduction pathways that involve G-proteins (Lefkowitz, Nature 351, 353-354, 1991). The family of G protein-coupled receptors (GPCR) includes receptors for hormones, neurotransmitters, growth factors, and viruses. Specific examples of GPCRs include receptors for such diverse agents as calcitonin, adrenergic hormones, endothelin, cAMP, adenosine, acetylcholine, serotonin, dopamine, histamine, thrombin, kinin, follicle stimulating hormone, opsins, endothelial differentiation gene-1, rhodopsins, odorants, cytomegalovirus, G proteins themselves, effector proteins such as phospholipase C, adenyl cyclase, and phosphodiesterase, and actuator proteins such as protein kinase A and protein kinase C.

The GPCR protein superfamily now contains over 250 types of paralogues, receptors that represent variants generated by gene duplications (or other processes), as opposed to orthologues, the same receptor from different species. The superfamily can be broken down into five families: Family I, receptors typified by rhodopsin and currently represented by over 200 unique members (reviewed by Dohlman et al., Ann. Rev. Biochem. 60, 653-88, 1991, and references therein); Family II, the recently characterized parathyroid hormone/calcitonin/secretin receptor family (Juppner et al., Science 254, 1024-26, 1991; Lin et al., Science 254, 1022-24, 1991); Family III, the metabotropic glutamate receptor family in mammals (Nakanishi, Science 258, 597-603, 1992); Family IV, the cAMP receptor family, important in the chemotaxis and development of *D. discoideum* (Klein et al., Science 241, 1467-72, 1988; and Family V, the fingal mating pheromone receptors such as STE2 (reviewed by Kurjan, Ann. Rev. Biochem. 61, 1097-1129, 1992).

WO 00/64942 discloses the human G-protein coupled receptor AXOR-27 having structural similarity with the human 5-HT6 serotonin receptor and methods for isolating agonists and/or antagonists thereto.

GPCRs possess seven conserved membrane-spanning domains connecting at least eight divergent hydrophilic loops. GPCRs (also known as 7TM receptors) have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 has been implicated in signal transduction.

Phosphorylation and lipidation (palmitylation or farnesylation) of cysteine residues can influence signal transduction of some GPCRs. Most GPCRs contain potential phosphorylation sites within the third cytoplasmic loop and/or the carboxy terminus. For several GPCRs, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 has been implicated in several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G-proteins to various intracellular enzymes, ion channels, and transporters (*see* Johnson et al., Endoc. Rev. 10, 317-331, 1989). Different G-protein alpha-subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation inside the cell of the enzyme, adenylate cyclase. Enzyme activation by hormones is dependent on the presence of the nucleotide GTP. GTP also influences hormone binding. A G protein connects the hormone receptor to adenylate cyclase. G protein exchanges GTP for bound GDP when activated by a hormone receptor. The GTP-carrying form then binds to activated adenylate cyclase. Hydrolysis of GTP to GDP, catalyzed by the G protein itself, returns the G protein to its basal, inactive form. Thus, the G protein serves a dual role, as an intermediate that relays the signal from receptor to effector, and as a clock that controls the duration of the signal.

Over the past 15 years, nearly 350 therapeutic agents targeting GPCRs receptors have been successfully introduced onto the market. This indicates that these receptors have an established, proven history as therapeutic targets. Clearly, there is an ongoing need for identification and characterization of further GPCRs which can play a role in preventing, ameliorating, or correcting dysfunctions or diseases including, but not limited to, infections such as bacterial, fungal, protozoan, and viral infections, particularly those caused by HIV viruses, pain, cancers, anorexia, bulimia, asthma, Parkinson's diseases, acute heart failure, hypotension, hypertension, urinary retention, osteoporosis, angina pectoris, myocardial infarction, ulcers, asthma, allergies, benign prostatic hypertrophy, and psychotic and neurological disorders, including anxiety, schizophrenia, manic depression, delirium, dementia, several mental retardation, and dyskinesias, such as Huntington's disease and Tourett's syndrome.
Because of the diverse biological effects, there is a need in the art to identify additional GPCRs whose activity can be regulated to provide therapeutic effects.

## SUMMARY OF THE INVENTION

It is an object of the invention to provide reagents and methods of regulating a human GPCR. This and other objects of the invention are provided by one or more of the embodiments described below.
Herein described is a G protein-coupled receptor polypeptide comprising an amino acid sequence selected from the group consisting of:

amino acid sequences which are at least about 50% identical to
the amino acid sequence shown in SEQ ID NO: 2 and;
the amino acid sequence shown in SEQ ID NO: 2;

Yet another embodiment of the invention is a method of screening for agents which decrease extracellular matrix degradation. A test compound is contacted with a G protein-coupled receptor polypeptide comprising an amino acid sequence selected from the group consisting of:

amino acid sequences which are at least about 50% identical to
the amino acid sequence shown in SEQ ID NO: 2 and;
the amino acid sequence shown in SEQ ID NO: 2;

Binding between the test compound and the G protein-coupled receptor polypeptide is detected. A test compound which binds to the G protein-coupled receptor polypeptide is thereby identified as a potential agent for decreasing extracellular matrix degradation. The agent can work by decreasing the activity of the G protein-coupled receptor.
Another embodiment of the invention is a method of screening for agents which decrease extracellular matrix degradation. A test compound is contacted with a polynucleotide encoding a G protein-coupled receptor polypeptide, wherein the polynucleotide comprises a nucleotide sequence selected from the group consisting of:

nucleotide sequences which are at least about 50% identical to
the nucleotide sequence shown in SEQ ID NO: 1 and;
the nucleotide sequence shown in SEQ ID NO: 1;

Binding of the test compound to the polynucleotide is detected. A test compound which binds to the polynucleotide is identified as a potential agent for decreasing extracellular matrix degradation. The agent can work by decreasing the amount of the G protein-coupled receptor through interacting with the G protein-coupled receptor mRNA.
Another embodiment of the invention is a method of screening for agents which regulate extracellular matrix degradation. A test compound is contacted with a G protein-coupled receptor polypeptide comprising an amino acid sequence selected from the group consisting of:

amino acid sequences which are at least about 50% identical to
the amino acid sequence shown in SEQ ID NO: 2 and;
the amino acid sequence shown in SEQ ID NO: 2;

A G protein-coupled receptor activity of the polypeptide is detected. A test compound which increases G protein-coupled receptor activity of the polypeptide relative to G protein-coupled receptor activity in the absence of the test compound is thereby identified as a potential agent for increasing extracellular matrix degradation. A test compound which decreases G protein-coupled receptor activity of the polypeptide relative to G protein-coupled receptor activity in the absence of the test compound is thereby identified as a potential agent for decreasing extracellular matrix degradation.

Even another embodiment of the invention is a method of screening for agents which decrease extracellular matrix degradation. A test compound is contacted with a G protein-coupled receptor product of a polynucleotide which comprises a nucleotide sequence selected from the group consisting of:

nucleotide sequences which are at least about 50% identical to
the nucleotide sequence shown in SEQ ID NO: 1 and;
the nucleotide sequence shown in SEQ ID NO: 1;

Binding of the test compound to the G protein-coupled receptor product is detected. A test compound which binds to the G protein-coupled receptor product is thereby identified as a potential agent for decreasing extracellular matrix degradation.

Still another embodiment of the invention is a method of reducing extracellular matrix degradation. A cell is contacted with a reagent which specifically binds to a polynucleotide encoding a G protein-coupled receptor polypeptide or the product encoded by the polynucleotide, wherein the polynucleotide comprises a nucleotide sequence selected from the group consisting of:

nucleotide sequences which are at least about 50% identical to
the nucleotide sequence shown in SEQ ID NO: 1 and;
the nucleotide sequence shown in SEQ ID NO: 1;

G protein-coupled receptor activity in the cell is thereby decreased.

The invention thus provides a human GPCR which can be used to identify test compounds which may act as agonists or antagonists at the receptor site. The human GPCR and fragments thereof also are useful in raising specific antibodies which can block the receptor and effectively prevent ligand binding.

## BRIEF DESCRIPTION OF THE DRAWINGS

Fig. 1 shows the DNA-sequence encoding a G protein-coupled receptor Polypeptide (SEQ ID NO: 1).

Fig. 2 shows the amino acid sequence deduced from the DNA-sequence of Fig. 1 (SEQ ID NO: 2).

Fig. 3 shows the DNA-sequence encoding a G protein-coupled receptor Polypeptide (SEQ ID NO: 3).

Fig. 4 shows the DNA-sequence encoding a G protein-coupled receptor Polypeptide (SEQ ID NO: 4).

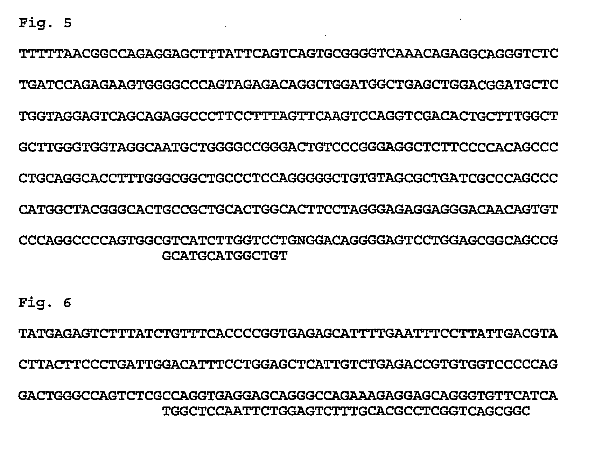

Fig. 5 shows the DNA-sequence encoding a G protein-coupled receptor Polypeptide (SEQ ID NO: 5).

Fig. 6 shows the DNA-sequence encoding a G protein-coupled receptor Polypeptide (SEQ ID NO: 6).

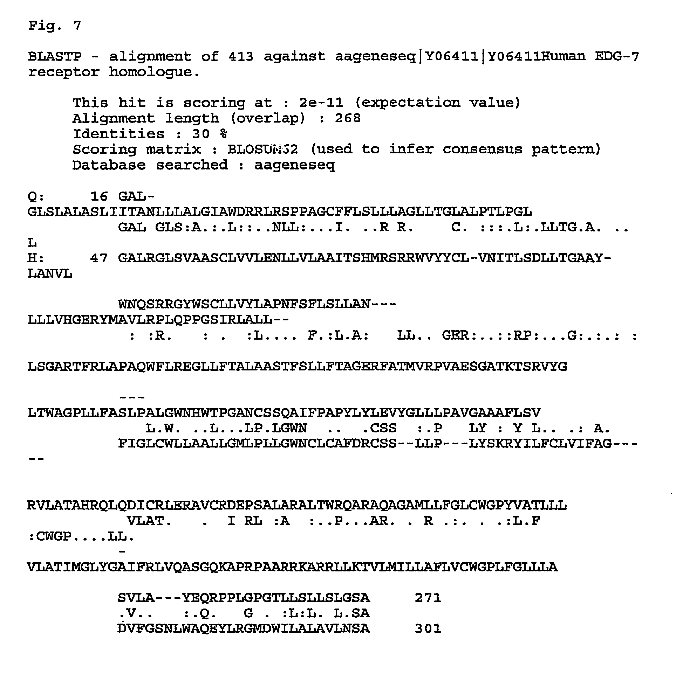

Fig. 7 shows the BLASTP - alignment of 413 (SEQ ID NO: 2) against aageneseq|Y06411|Y06411Human EDG-7 receptor homologue.

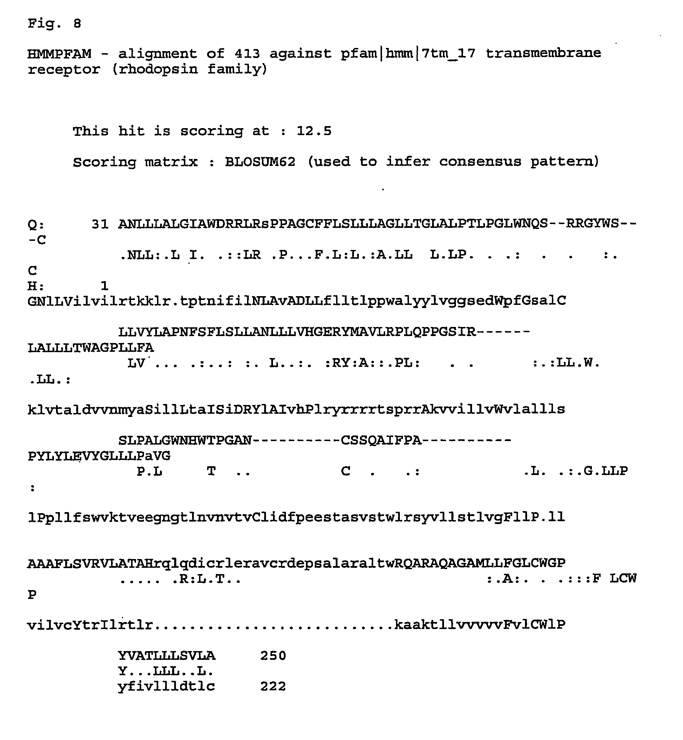

Fig. 8 shows the HMMPFAM - alignment of 413 (SEQ ID NO: 2) against pfam|hmm|7tm_17 transmembrane receptor (rhodopsin family).

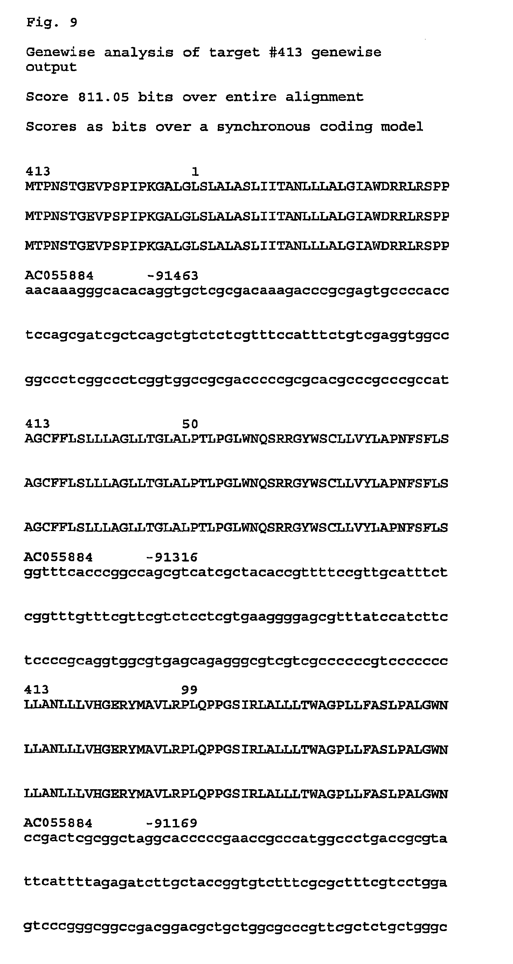

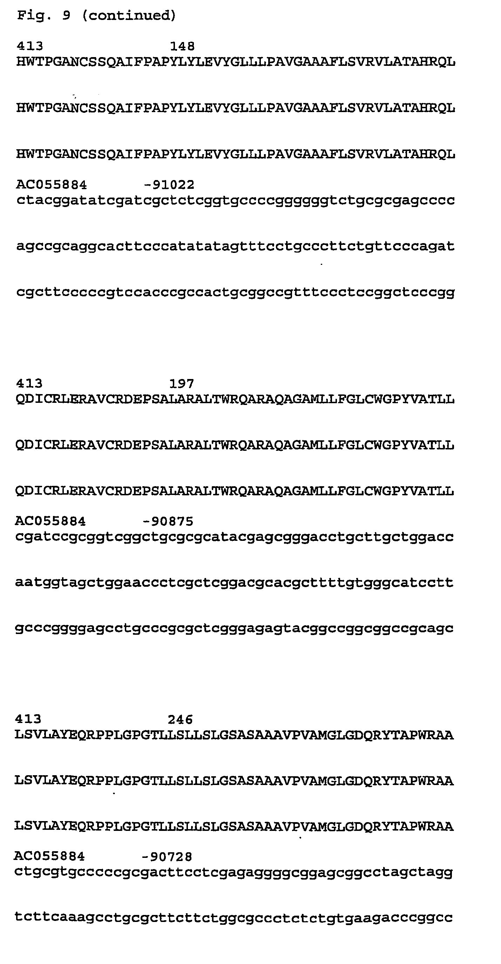

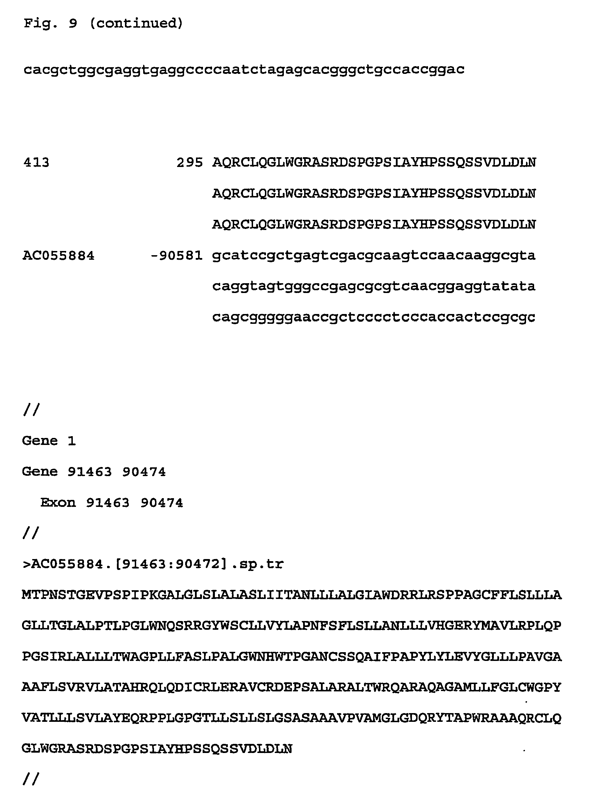

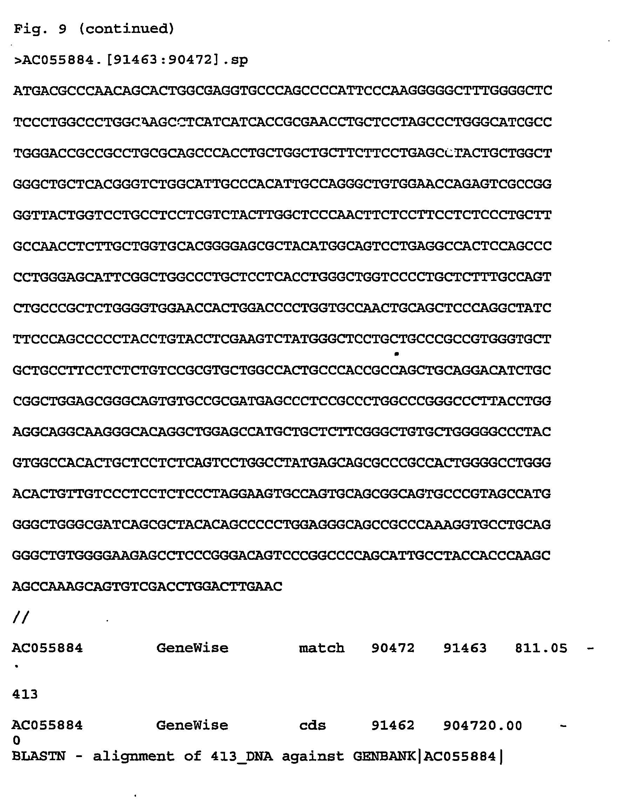

Fig. 9 shows the Genewise analysis.

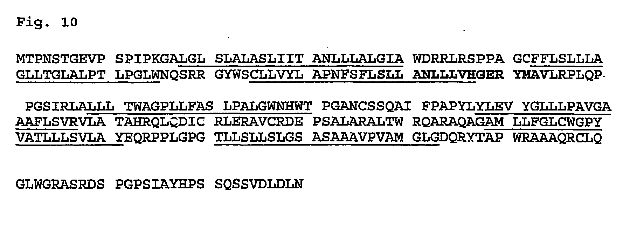

Fig. 10 Amino acid sequence of human GPCR Transmembrane domains are underlined, and the Prosite consensus pattern is shown in bold.

Fig. 11 shows the Expression profile.

Fig. 12 shows the Expression profile.

Fig. 13 shows the Expression profile.

## DETAILED DESCRIPTION OF THE INVENTION

The invention relates to an isolated polynucleotide from the group consisting of:

a) a polynucleotide encoding a G protein-coupled receptor polypeptide comprising an amino acid sequence selected from the group consisting of:

amino acid sequences which are at least about 50% identical to
the amino acid sequence shown in SEQ ID NO: 2 and;
the amino acid sequence shown in SEQ ID NO: 2;

b) a polynucleotide comprising the sequence of SEQ ID NO: 1;

c) a polynucleotide which hybridizes under stringent conditions to a polynucleotide specified in (a) and (b) and encodes a G protein-coupled receptor polypeptide;

d) a polynucleotide the sequence of which deviates from the polynucleotide sequences specified in (a) to (c) due to the degeneration of the genetic code and encodes a G protein-coupled receptor polypeptide; and

e) a polynucleotide which represents a fragment, derivative or allelic variation of a polynucleotide sequence specified in (a) to (d) and encodes a G protein-coupled receptor polypeptide.

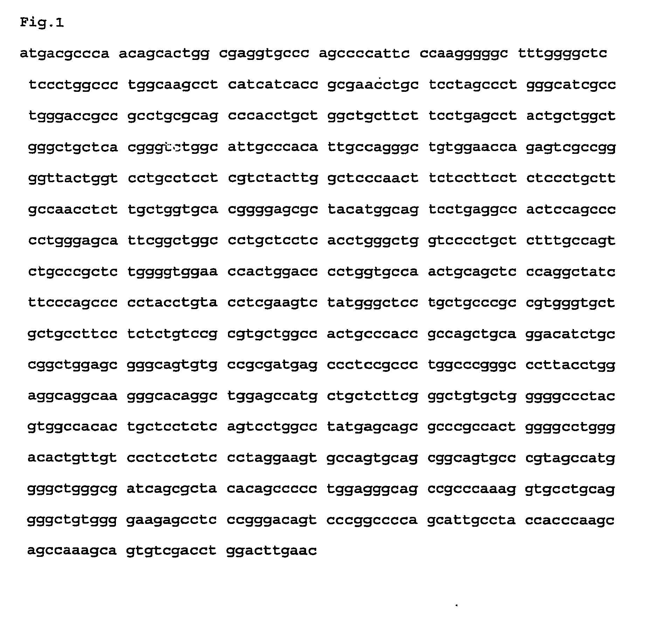

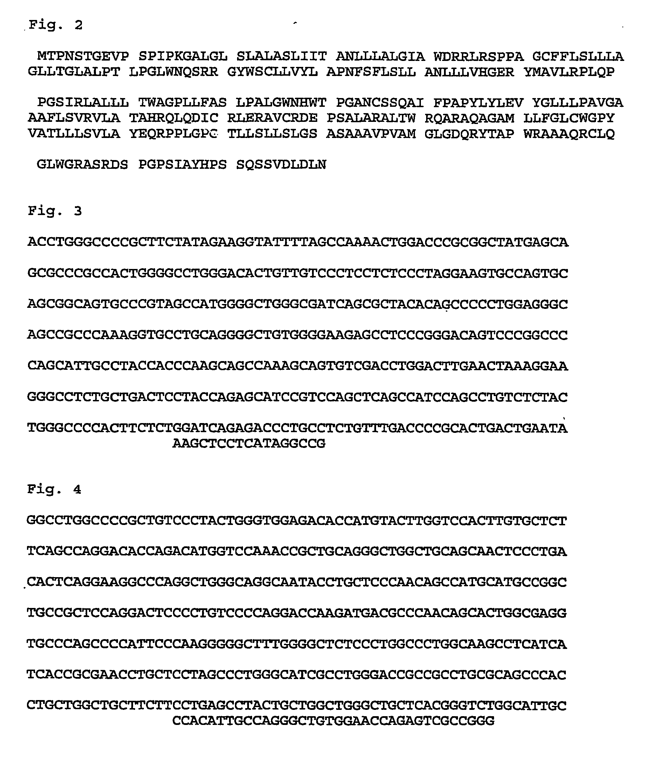

Furthermore, it has been discovered by the present applicant that a new GPCR, particularly a human GPCR, can be used in therapeutic methods to treat COPD, a cardiovascular disorder, cancer, a urinary disorder, obesity, diabetes, a CNS disorder, a liver disorder, a kidney disorder, a disorder of the secretory organs, asthma, a metabolic disease or a hematological disorder comprising the amino acid sequence shown in SEQ ID NO: 2 has been identified. A coding sequence for human GPCR is shown in SEQ ID NO: 1. This sequence is located on chromosome 2. The coding sequence for SEQ ID NO: 2 was assembled from the genomic sequence identified with GenBank Accession No. AC055884, using geneid and genewise. Alignments are provided in FIGS. 1 and 2.

SEQ ID NO: 2 contains seven transmembrane domains and hits several known GPCRs with good e-value. SEQ ID NO: 2 is approximately 30% identical to several EDG receptors, with about 25% identity several human histamine H2, beta-adrenergic and thyrotropin releasing hormone (TRH) receptor. Three dimensional structure was inferred by unlikely homology from residues 4 to 178 in 1F88-B (rhodopsin). The Prosite consensus pattern is highlighted in FIG. 4. Related ESTs (SEQ ID NOS: 3-6) are expressed in neuroblastoma, B cells, chronic lymphatic leukemiaa, brain, and normal prostate.

The human GPCR of the invention can be used in therapeutic methods to treat disorders such as COPD, cardiovascular disorders, cancer, urinary disorders, obesity, diabetes, CNS disorders, liver disorders, kidney disorders, disorders of the secretory organs, asthma, and hematological disorders. The human GPCR also can be used to screen for human GPCR activators and inhibitors.

### *Polypeptides*

GPCR polypeptides according to the invention comprise at least 6, 8, 10, 15, 20, 25, 50, 75, 100, 125, 150, 175, 200, 225, 250, 275, 300, 325, or 330 contiguous amino acids selected from the amino acid sequence shown in SEQ ID NO: 2 or a biologically active variant thereof, as defined below. A GPCR polypeptide of the invention therefore can be a portion of a GPCR protein, a full-length GPCR protein, or a fusion protein comprising all or a portion of a GPCR protein.

### *Biologically Active Variants*

GPCR polypeptide variants which are biologically active, *i.e.*, retain the ability to bind a ligand to produce a biological effect, such as cyclic AMP formation, mobilization of intracellular calcium, or phosphoinositide metabolism, also are GPCR polypeptides. Preferably, naturally or non-naturally occurring GPCR polypeptide variants have amino acid sequences which are at least about 50, preferably at least about 55, 65, 70, 75, 90, 96, or 98% identical to the amino acid sequence shown in SEQ ID NO: 2 or a fragment thereof. Percent identity between a putative GPCR polypeptide variant and an amino acid sequence of SEQ ID NO: 2 is determined using the Blast2 alignment program (Blosum62, Expect 10, standard genetic codes).

Variations in percent identity can be due, for example, to amino acid substitutions, insertions, or deletions.

Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a GPCR polypeptide can be found using computer programs well known in the art, such as DNASTAR software. Whether an amino acid change results in a biologically active GPCR polypeptide can readily be determined by assaying for binding to a ligand or by conducting a functional assay, as described for example, in the specific Examples, below.

*Fusion Proteins*

Fusion proteins are useful for generating antibodies against GPCR polypeptide amino acid sequences and for use in various assay systems. For example, fusion proteins can be used to identify proteins which interact with portions of a GPCR polypeptide. Protein affinity chromatography or library-based assays for protein-protein interactions, such as the yeast two-hybrid or phage display systems, can be used for this purpose. Such methods are well known in the art and also can be used as drug screens.

A GPCR polypeptide fusion protein comprises two polypeptide segments fused together by means of a peptide bond. The first polypeptide segment comprises at least 6, 8, 10, 15, 20, 25, 50, 75, 100, 125, 150, 175, 200, 225, 250, 275, 300, 325, or 330 contiguous amino acids of SEQ ID NO: 2 or of a biologically active variant, such as those described above. The first polypeptide segment also can comprise full-length GPCR protein.

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the GPCR polypeptide-encoding sequence and the heterologous protein sequence, so that the GPCR polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from SEQ ID NO: 1 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

*Identification of Species Homologs*

Species homologs of human GPCR polypeptide can be obtained using GPCR polypeptide polynucleotides (described below) to make suitable probes or primers for screening cDNA expression libraries from other species, such as mice, monkeys, or yeast, identifying cDNAs which encode homologs of GPCR polypeptide, and expressing the cDNAs as is known in the art.

*Polynucleotides*

A GPCR polynucleotide can be single- or double-stranded and comprises a coding sequence or the complement of a coding sequence for a GPCR polypeptide. A coding sequence for human GPCR is shown in SEQ ID NO: 1.

Degenerate nucleotide sequences encoding human GPCR polypeptides, as well as homologous nucleotide sequences which are at least about 50, preferably about 75, 90, 96, or 98% identical to the nucleotide sequence shown in SEQ ID NO: 1 also are GPCR polynucleotides. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologs, and variants of GPCR polynucleotides which encode biologically active GPCR polypeptides also are GPCR polynucleotides. Polynucleotide fragments comprising at least 8, 9, 10, 11, 12, 15, 20, or 25 contiguous nucleotides of SEQ ID NO: 1 or its complement also are GPCR polynucleotides. These fragments can be used, for example, as hybridization probes or as antisense oligonucleotides.

*Identification of Polynucleotide Variants and Homologs*

Variants and homologs of the GPCR polynucleotides described above also are GPCR polynucleotides. Typically, homologous GPCR polynucleotide sequences can be identified by hybridization of candidate polynucleotides to known GPCR polynucleotides under stringent conditions, as is known in the art. For example, using the following wash conditions-2X SSC (0.3 M NaCl, 0.03 M sodium citrate, pH 7.0), 0.1% SDS, room temperature twice, 30 minutes each; then 2X SSC, 0.1% SDS, 50°C once, 30 minutes; then 2X SSC, room temperature twice, 10 minutes each--homologous sequences can be identified which contain at most about 25-30% basepair mismatches. More preferably, homologous nucleic acid strands contain 15-25% basepair mismatches, even more preferably 5-15% basepair mismatches.

Species homologs of the GPCR polynucleotides disclosed herein also can be identified by making suitable probes or primers and screening cDNA expression libraries from other species, such as mice, monkeys, or yeast. Human variants of GPCR polynucleotides can be identified, for example, by screening human cDNA expression libraries. It is well known that the $T_m$ of a double-stranded DNA decreases by 1-1.5°C with every 1% decrease in homology (Bonner et al., J. Mol. Biol. 81, 123 (1973). Variants of human GPCR polynucleotides or GPCR polynucleotides of other species can therefore be identified by hybridizing a putative homologous GPCR polynucleotide with a polynucleotide having a nucleotide sequence of SEQ ID NO: 1 or the complement thereof to form a test hybrid. The melting temperature of the test hybrid is compared with the melting temperature of a hybrid comprising polynucleotides having perfectly complementary nucleotide sequences, and the number or percent of basepair mismatches within the test hybrid is calculated.

Nucleotide sequences which hybridize to GPCR polynucleotides or their complements following stringent hybridization and/or wash conditions also are GPCR polynucleotides. Stringent wash conditions are well known and understood in the art and are disclosed, for example, in Sambrook et al., MOLECULAR CLONING: A LABORATORY MANUAL, 2d ed., 1989, at pages 9.50-9.51.

Typically, for stringent hybridization conditions a combination of temperature and salt concentration should be chosen that is approximately 12-20°C below the calculated $T_m$ of the hybrid under study. The $T_m$ of a hybrid between a GPCR polynucleotide having a nucleotide sequence shown in SEQ ID NO: 1 or the complement thereof and a polynucleotide sequence which is at least about 50, preferably about 75, 90, 96, or 98% identical to one of those nucleotide sequences can be calculated, for example, using the equation of Bolton and McCarthy, Proc. Natl. Acad. Sci. U.S.A. 48, 1390 (1962):

$$T_m = 81.5\ ^\circ C - 16.6(\log_{10}[Na^+]) + 0.41(\%G + C) - 0.63(\%formamide) - 600/l,$$

where *l* = the length of the hybrid in basepairs.

Stringent wash conditions include, for example, 4X SSC at 65°C, or 50% formamide, 4X SSC at 42°C, or 0.5X SSC, 0.1% SDS at 65°C. Highly stringent wash conditions include, for example, 0.2X SSC at 65°C.

*Preparation of Polynucleotides*

A naturally occurring GPCR polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating poly nucleotides are routine and are known in the art. Any such technique for obtaining a polynucleotide can be used to obtain isolated GPCR polynucleotides. For example, restriction enzymes and probes can be used to isolate polynucleotide fragments which comprise GPCR nucleotide sequences. Isolated polynucleotides are in preparations which are free or at least 70, 80, or 90% free of other molecules.

GPCR cDNA molecules can be made with standard molecular biology techniques, using GPCR mRNA as a template. GPCR cDNA molecules can thereafter be replicated using molecular biology techniques known in the art and disclosed in manuals such as Sambrook *et al.* (1989). An amplification technique, such as PCR, can be used to obtain additional copies of polynucleotides of the invention, using either human genomic DNA or cDNA as a template.

Alternatively, synthetic chemistry techniques can be used to synthesize GPCR poly nucleotides. The degeneracy of the genetic code allows alternate nucleotide sequences to be synthesized which will encode a GPCR polypeptide having, for example, an amino acid sequence shown in SEQ ID NO: 2 or a biologically active variant thereof.

*Extending Polynucleotides*

Various PCR-based methods can be used to extend the nucleic acid sequences disclosed herein to detect upstream sequences such as promoters and regulatory elements. For example, restriction-site PCR uses universal primers to retrieve unknown sequence adjacent to a known locus (Sarkar, PCR Methods Applic. 2, 318-322, 1993). Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region (Triglia et al., Nucleic Acids Res. 16, 8186, 1988). Primers can be designed using commercially available software, such as OUGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA (Lagerstrom et al., PCR Methods Applic. 1, 111-119, 1991). In this method, multiple restriction enzyme digestions and ligations also can be used to place an engineered double-stranded sequence into an unknown fragment of the DNA molecule before performing PCR.

Another method which can be used to retrieve unknown sequences is that of Parker et al., Nucleic Acids Res. 19, 3055-3060, 1991). Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software *(e.g.* GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA, which might be present in limited amounts in a particular sample.

*Obtaining Polypeptides*

GPCR polypeptides can be obtained, for example, by purification from human cells, by expression of GPCR polynucleotides, or by direct chemical synthesis.

*Protein Purification*

GPCR polypeptides can be purified from any human cell which expresses the receptor, including host cells which have been transfected with GPCR polynucleotides. A purified GPCR polypeptide is separated from other compounds which normally associate with the GPCR polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

GPCR polypeptide can be conveniently isolated as a complex with its associated G protein, as described in the specific examples, below. A preparation of purified GPCR polypeptides is at least 80% pure; preferably, the preparations are 90%, 95%, or 99% pure. Purity of the preparations can be assessed by any means known in the art, such as SDS-polyacrylamide gel electrophoresis.

*Expression of Polynucleotides*

To express a GPCR polynucleotide, the polynucleotide can be inserted into an expression vector which contains the necessary elements for the transcription and translation of the inserted coding sequence. Methods which are well known to those skilled in the art can be used to construct expression vectors containing sequences encoding GPCR polypeptides and appropriate transcriptional and translational control elements. These methods include *in vitro* recombinant DNA techniques, synthetic techniques, and *in vivo* genetic recombination. Such techniques are described, for example, in Sambrook et al. (1989) and in Ausubel et al., CURRENT PROTOCOLS IN MOLECULAR BIOLOGY, John Wiley & Sons, New York, N.Y., 1989.

A variety of expression vector/host systems can be utilized to contain and express sequences encoding a GPCR polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e.g.,* baculovirus), plant cell systems transformed with virus expression vectors (*e.g.*, cauliflower mosaic virus, CaMV; tobacco mosaic virus, TMV) or with bacterial expression vectors (*e.g.*, Ti or pBR322 plasmids), or animal cell systems.

The control elements or regulatory sequences are those non-translated regions of the vector - enhancers, promoters, 5' and 3' untranslated regions - which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells *(e.g.,* heat shock, RUBISCO, and storage protein genes) or from plant viruses *(e.g.,* viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding an GPCR polypeptide, vectors based on SV40 or EBV can be used with an appropriate selectable marker.

*Bacterial and Yeast Expression Systems*

In bacterial systems, a number of expression vectors can be selected depending upon the use intended for the GPCR polypeptide. For example, when a large quantity of an GPCR polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the GPCR polypeptide can be ligated into the vector in frame with sequences for the amino-terminal Met and the subsequent 7 residues of β-galactosidase so that a hybrid protein is produced. pIN vectors (Van Heeke & Schuster, J. Biol. Chem. 264, 5503-5509, 1989) or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast *Saccharomyces cerevisiae,* a number of vectors containing constitutive or inducible promoters such as alpha factor, alcohol oxidase, and PGH can be used. For reviews, see Ausubel et al. (1989) and Grant et al., Methods Enzymol. 153, 516-544, 1987.

*Plant and Insect Expression Systems*

If plant expression vectors are used, the expression of sequences encoding GPCR polypeptides can be driven by any of a number of promoters. For example, viral promoters such as the 35S and 19S promoters of CaMV can be used alone or in combination with the omega leader sequence from TMV (Takamatsu, EMBO J. 6, 307-311, 1987). Alternatively, plant promoters such as the small subunit of RUBISCO or heat shock promoters can be used (Coruzzi et al., EMBO J. 3, 1671-1680, 1984; Broglie et al., Science 224, 838-843, 1984; Winter et al., Results Probl. Cell Differ. 17, 85-105, 1991). These constructs can be introduced into plant cells by direct DNA transformation or by pathogen-mediated transfection. Such techniques are described in a number of generally available reviews (*e.g.*, Hobbs or Murray, in MCGRAW HILL YEARBOOK OF SCIENCE AND TECHNOLOGY, McGraw Hill, New York. N.Y., pp. 191-196, 1992).

An insect system also can be used to express a GPCR polypeptide. For example, in one such system *Autographa californica* nuclear polyhedrosis virus (AcNPV) is used as a vector to express foreign genes in *Spodoptera frugiperda* cells or in *Trichoplusia* larvae. Sequences encoding GPCR polypeptides can be cloned into a non-essential region of the virus, such as the polyhedrin gene, and placed under control of the polyhedrin promoter. Successful insertion of GPCR polypeptides will render the polyhedrin gene inactive and produce recombinant virus lacking coat protein. The recombinant viruses can then be used to infect *S. frugiperda cells* or *Trichoplusia* larvae in which GPCR polypeptides can be expressed (Engelhard et al., Proc. Nat. Acad. Sci. 91, 3224-3227, 1994).

*Mammalian Expression Systems*

A number of viral-based expression systems can be used to express GPCR polypeptides in mammalian host cells. For example, if an adenovirus is used as an expression vector, sequences encoding GPCR polypeptides can be ligated into an adenovirus transcription/translation complex comprising the late promoter and tripartite leader sequence. Insertion in a non-essential E1 or E3 region of the viral genome can be used to obtain a viable virus which is capable of expressing an GPCR polypeptide in infected host cells (Logan & Shenk, Proc. Natl. Acad. Sci. 81, 3655-3659, 1984). If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (*e.g.*, liposomes, polycationic amino polymers, or vesicles).

Specific initiation signals also can be used to achieve more efficient translation of sequences encoding GPCR polypeptides. Such signals include the ATG initiation codon and adjacent sequences. In cases where sequences encoding an GPCR polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used (see Scharf et al., Results Probl. Cell Differ. 20, 125-162, 1994).

*Host Cells*

A host cell strain can be chosen for its ability to modulate the expression of the inserted sequences or to process the expressed GPCR polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g.*, CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express GPCR polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced GPCR sequences. Resistant clones of stably transformed cells can be proliferated using tissue culture techniques appropriate to the cell type. See, for example, ANIMAL CELL CULTURE, R.I. Freshney, ed., 1986.

Any number of selection systems can be used to recover transformed cell lines.

These include, but are not limited to, the herpes simplex virus thymidine kinase (Wigler et al., Cell 11, 223-32, 1977) and adenine phosphoribosyltransferase (Lowy et al., Cell 22, 817-23, 1980) genes which can be employed in $tk^-$ or $aprt^-$ cells, respectively. Also, antimetabolite, antibiotic, or herbicide resistance can be used as the basis for selection. For example, *dhfr* confers resistance to methotrexate (Wigler et al., Proc. Natl. Acad. Sci. 77, 3567-70, 1980), *npt* confers resistance to the aminoglycosides, neomycin and G-418 (Colbere-Garapin et al., J. Mol. Biol. 150, 1-14, 1981), and *als* and *pat* confer resistance to chlorsulfuron and phosphinotricin acetyltransferase, respectively (Murray, 1992, *supra*). Additional selectable genes have been described. For example, *trpB* allows cells to utilize indole in place of tryptophan, or *hisD,* which allows cells to utilize histinol in place of histidine (Hartman & Mulligan, Proc. Natl. Acad. Sci. 85, 8047-51, 1988). Visible markers such as anthocyanins, β-glucuronidase and its substrate GUS, and luciferase and its substrate luciferin, can be used to identify transformants and to quantify the amount of transient or stable protein expression attributable to a specific vector system (Rhodes et al., Methods Mol. Biol. 55, 121-131, 1995).

*Detecting Expression*

Although the presence of marker gene expression suggests that the GPCR poly nucleotide is also present, its presence and expression may need to be confirmed. For example, if a sequence encoding an GPCR polypeptide is inserted within a marker gene sequence, transformed cells containing sequences which encode an GPCR polypeptide can be identified by the absence of marker gene function. Alternatively, a marker gene can be placed in tandem with a sequence encoding an GPCR poly peptide under the control of a single promoter. Expression of the marker gene in response to induction or selection usually indicates expression of the GPCR poly nucleotide.

Alternatively, host cells which contain a GPCR polynucleotide and which express an GPCR polypeptide can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding a GPCR polypeptide can be detected by DNA-DNA or DNA-RNA hybridization or amplification using probes or fragments or fragments of polynucleotides encoding an GPCR polypeptide. Nucleic acid amplification-based assays involve the use of oligonucleotides selected from sequences encoding an GPCR polypeptide to detect transformants which contain a GPCR polynucleotide.

A variety of protocols for detecting and measuring the expression of a GPCR polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a GPCR polypeptide can be used, or a competitive binding assay can be employed. These and other assays are described in Hampton et al., SEROLOGICAL METHODS: A LABORATORY MANUAL, APS Press, St. Paul, Minn., 1990) and Maddox et al., J. Exp. Med. 158, 1211-1216, 1983).

A wide variety of labels and conjugation techniques are known by those skilled in the art and can be used in various nucleic acid and amino acid assays. Means for producing labeled hybridization or PCR probes for detecting sequences related to polynucleotides encoding GPCR polypeptides include oligolabeling, nick translation, end-labeling, or PCR amplification using a labeled nucleotide. Alternatively, sequences encoding a GPCR polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with nucleotide sequences encoding a GPCR polypeptide can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing polynucleotides which encode GPCR polypeptides can be designed to contain signal sequences which direct secretion of soluble GPCR polypeptides through a prokaryotic or eukaryotic cell membrane or which direct the membrane insertion of membrane-bound GPCR polypeptide.

As discussed above, other constructions can be used to join a sequence encoding a GPCR polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the GPCR poly peptide also can be used to facilitate purification. One such expression vector provides for expression of a fusion protein containing a GPCR polypeptide and 6 histidine residues preceding a thioredoxin or an enterokinase cleavage site. The histidine residues facilitate purification by IMAC (immobilized metal ion affinity chromatography, as described in Porath et al., Prot. Exp. Purif. 3, 263-281, 1992), while the enterokinase cleavage site provides a means for purifying the GPCR polypeptide from the fusion protein. Vectors which contain fusion proteins are disclosed in Kroll et al., DNA Cell Biol. 12, 441-453, 1993.

*Chemical Synthesis*

Sequences encoding a GPCR polypeptide can be synthesized, in whole or in part, using chemical methods well known in the art (see Caruthers et al., Nucl. Acids Res. Symp. Ser. 215-223, 1980; Horn et al. Nucl. Acids Res. Symp. Ser. 225-232, 1980). Alternatively, a GPCR polypeptide itself can be produced using chemical methods to synthesize its amino acid sequence, such as by direct peptide synthesis using solid-phase techniques (Merrifield, J. Am. Chem. Soc. 85, 2149-2154, 1963; Roberge et al., Science 269, 202-204, 1995). Protein synthesis can be performed using manual techniques or by automation. Automated synthesis can be achieved, for example, using Applied Biosystems 431A Peptide Synthesizer (Perkin Elmer). Optionally, fragments of GPCR polypeptides can be separately synthesized and combined using chemical methods to produce a full-length molecule.

The newly synthesized peptide can be substantially purified by preparative high performance liquid chromatography (*e.g.*, Creighton, PROTEINS: STRUCTURES AND MOLECULAR PRINCIPLES, WH Freeman and Co., New York, N.Y., 1983). The composition of a synthetic GPCR polypeptide can be confirmed by amino acid analysis or sequencing *(e.g.,* the Edman degradation procedure; *see* Creighton, *supra*). Additionally, any portion of the amino acid sequence of the GPCR polypeptide can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce GPCR polypeptide-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter GPCR polypeptide-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Antibodies*

Any type of antibody known in the art can be generated to bind specifically to an epitope of a GPCR polypeptide. "Antibody" as used herein includes intact immunoglobulin molecules, as well as fragments thereof, such as Fab, $F(ab')_2$, and Fv, which are capable of binding an epitope of a GPCR polypeptide. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to an epitope of a GPCR polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to a GPCR polypeptide provides a detection signal at least 5-, 10-, or 20-fold higher than a detection signal provided with other proteins when used in an immunochemical assay. Preferably, antibodies which specifically bind to GPCR polypeptides do not detect other proteins in immunochemical assays and can immunoprecipitate a GPCR polypeptide from solution.

GPCR polypeptides can be used to immunize a mammal, such as a mouse, rat, rabbit, guinea pig, monkey, or human, to produce polyclonal antibodies. If desired, a GPCR polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (*e.g.*, aluminum hydroxide), and surface active substances (*e.g.* lysolecithin, pluronic polyols, polyanions, peptides, oil emulsions, keyhole limpet hemocyanin, and dinitrophenol). Among adjuvants used in humans, BCG (*bacilli Calmette-Guerin*) and *Corynebacterium parvum* are especially useful.

Monoclonal antibodies which specifically bind to a GPCR polypeptide can be prepared using any technique which provides for the production of antibody molecules by continuous cell lines in culture. These techniques include, but are not limited to, the hybridoma technique, the human B-cell hybridoma technique, and the EBV-hybridoma technique (Kohler et al., Nature 256, 495-497, 1985; Kozbor et al., J. Immunol. Methods 81, 31-42, 1985; Cote et al., Proc. Natl. Acad. Sci. 80, 2026-2030, 1983; Cole et al., Mol. Cell Biol. 62, 109-120, 1984).

In addition, techniques developed for the production of "chimeric antibodies," the splicing of mouse antibody genes to human antibody genes to obtain a molecule with appropriate antigen specificity and biological activity, can be used (Morrison et al., Proc. Natl. Acad. Sci. 81, 6851-6855, 1984; Neuberger et al., Nature 312, 604-608, 1984; Takeda et al., Nature 314, 452-454, 1985). Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to a GPCR polypeptide can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to GPCR polypeptides. Antibodies with related specificity, but of distinct idiotypic composition, can be generated by chain shuffling from random combinatorial immunoglobin libraries (Burton, Proc. Natl. Acad. Sci. 88, 11120-23, 1991).

Single-chain antibodies also can be constructed using a DNA amplification method, such as PCR, using hybridoma cDNA as a template (Thirion et al., 1996, Eur. J. Cancer Prev. 5, 507-11). Single-chain antibodies can be mono- or bispecific, and can be bivalent or tetravalent. Construction of tetravalent, bispecific single-chain antibodies is taught, for example, in Coloma & Morrison, 1997, Nat. Biotechnol. 15, 159-63. Construction of bivalent, bispecific single-chain antibodies is taught in Mallender & Voss, 1994, J. Biol. Chem. 269, 199-206.

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology (Verhaar et al., 1995, Int. J. Cancer 61, 497-501; Nicholls et al., 1993, J. Immunol. Meth. 165, 81-91).

Antibodies which specifically bind to GPCR polypeptides also can be produced by inducing *in vivo* production in the lymphocyte population or by screening immunoglobulin libraries or panels of highly specific binding reagents as disclosed in the literature (Orlandi et al., Proc. Natl. Acad. Sci. 86, 3833-3837, 1989; Winter et al., Nature 349, 293-299, 1991).

Other types of antibodies can be constructed and used therapeutically in methods of the invention For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which a GPCR polypeptide is bound. The bound antibodies can then be eluted from the column using a buffer with a high salt concentration.

*Antisense Oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of GPCR gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters. *See* Brown, Meth. Mol. Biol. 20, 1-8, 1994; Sonveaux, Meth. Mol. Biol. 26, 1-72, 1994; Uhlmann et al., Chem. Rev. 90, 543-583, 1990.

Modifications of GPCR gene expression can be obtained by designing antisense oligonucleotides which will form duplexes to the control, 5', or regulatory regions of the GPCR gene. Oligonucleotides derived from the transcription initiation site, *e.g.,* between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons.

Therapeutic advances using triplex DNA have been described in the literature (*e.g.*, Gee et al., in Huber & Carr, MOLECULAR AND IMMUNOLOGIC APPROACHES, Futura Publishing Co., Mt. Kisco, N.Y., 1994). An antisense oligonucleotide also can be designed to block translation of mRNA by preventing the transcript from binding to ribosomes.

Precise complementarity is not required for successful complex formation between an antisense oligonucleotide and the complementary sequence of a GPCR polynucleotide. Antisense oligonucleotides which comprise, for example, 2, 3, 4, or 5 or more stretches of contiguous nucleotides which are precisely complementary to a GPCR polynucleotide, each separated by a stretch of contiguous nucleotides which are not complementary to adjacent GPCR nucleotides, can provide sufficient targeting specificity for GPCR mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular GPCR polynucleotide sequence.

Antisense oligonucleotides can be modified without affecting their ability to hybridize to a GPCR polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art. *See, e.g.,* Agrawal et al., Trends Biotechnol. 10, 152-158, 1992; Uhlmann et al., Chem. Rev. 90, 543-584, 1990; Uhlmann et al., Tetrahedron. Lett. 215, 3539-3542, 1987.

*Ribozymes*

Ribozymes are RNA molecules with catalytic activity. *See, e.g.,* Cech, Science 236, 1532-1539; 1987; Cech, Ann. Rev. Biochem. 59, 543-568; 1990, Cech, Curr. Opin. Struct. Biol. 2, 605-609; 1992, Couture & Stinchcomb, Trends Genet. 12, 510-515, 1996. Ribozymes can be used to inhibit gene function by cleaving an RNA sequence, as is known in the art (*e.g.,* Haseloff et al., U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The coding sequence of a GPCR polynucleotide can be used to generate ribozymes which will specifically bind to mRNA transcribed from the GPCR polynucleotide. Methods of designing and constructing ribozymes which can cleave other RNA molecules in trans in a highly sequence specific manner have been developed and described in the art (*see* Haseloff et al. Nature 334, 585-591, 1988). For example, the cleavage activity of ribozymes can be targeted to specific RNAs by engineering a discrete "hybridization" region into the ribozyme. The hybridization region contains a sequence complementary to the target RNA and thus specifically hybridizes with the target (see, for example, Gerlach et al., EP 321,201).

Specific ribozyme cleavage sites within a GPCR RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate GPCR RNA targets also can be evaluated by testing accessibility to hybridization with complementary oligonucleotides using ribonuclease protection assays. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease GPCR expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff et al., U.S. Patent 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

*Differentially Expressed Genes*

Described herein are methods for the identification of genes whose products interact with human GPCR. Such genes may represent genes which are differentially expressed in disorders including, but not limited to, COPD, cardiovascular disorders, cancer, urinary disorders, obesity, diabetes, CNS disorders, liver disorders, kidney disorders, disorders of the secretory organs, asthma, and hematological disorders. Further, such genes may represent genes which are differentially regulated in response to manipulations relevant to the progression or treatment of such diseases. Additionally, such genes may have a temporally modulated expression, increased or decreased at different stages of tissue or organism development. A differentially expressed gene may also have its expression modulated under control versus experimental conditions. In addition, the human GPCR gene or gene product may itself be tested for differential expression.

The degree to which expression differs in a normal versus a diseased state need only be large enough to be visualized via standard characterization techniques such as differential display techniques. Other such standard characterization techniques by which expression differences may be visualized include but are not limited to, quantitative RT (reverse transcriptase), PCR, and Northern analysis.

*Identification of Differentially Expressed Genes*

To identify differentially expressed genes total RNA or, preferably, mRNA is isolated from tissues of interest. For example, RNA samples are obtained from tissues of experimental subjects and from corresponding tissues of control subjects. Any RNA isolation technique which does not select against the isolation of mRNA may be utilized for the purification of such RNA samples. See, for example, Ausubel et al., ed.,, CURRENT PROTOCOLS IN MOLECULAR BIOLOGY, John Wiley & Sons, Inc. New York, 1987-1993. Large numbers of tissue samples may readily be processed using techniques well known to those of skill in the art, such as, for example, the single-step RNA isolation process of Chomczynski, U.S. Patent 4,843,155.

Transcripts within the collected RNA samples which represent RNA produced by differentially expressed genes are identified by methods well known to those of skill in the art. They include, for example, differential screening (Tedder et al., Proc. Natl. Acad. Sci. U.S.A. 85, 208-12, 1988), subtractive hybridization (Hedrick et al., Nature 308, 149-53; Lee et al., Proc. Natl. Acad. Sci. U.S.A. 88, 2825, 1984), differential display (Liang & Pardee, Science 257, 967-71, 1992; U.S. Patent 5,262,311), and microarray analysis.

The differential expression information may itself suggest relevant methods for the treatment of disorders involving the human GPCR. For example, treatment may include a modulation of expression of the differentially expressed genes and/or the gene encoding the human GPCR. The differential expression information may indicate whether the expression or activity of the differentially expressed gene or gene product or the human GPCR gene or gene product are up-regulated or downregulated.

*Screening Methods*

The invention provides assays for screening test compounds which bind to or modulate the activity of a GPCR polypeptide or a GPCR polynucleotide. A test compound preferably binds to a GPCR polypeptide or polynucleotide. More preferably, a test compound decreases or increases the biological effect of a ligand on the receptor by at least about 10, preferably about 50, more preferably about 75, 90, or 100% relative to the absence of the test compound.

*Test Compounds*

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. *See* Lam, Anticancer Drug Des. 12, 145, 1997.

Methods for the synthesis of molecular libraries are well known in the art (*see,* for example, DeWitt et al., Proc. Natl. Acad. Sci. U.S.A. 90, 6909, 1993; Erb et al. Proc. Natl. Acad. Sci. U.S.A. 91, 11422, 1994; Zuckermann et al., J. Med. Chem. 37, 2678, 1994; Cho et al., Science 261, 1303, 1993; Carell et al., Angew. Chem. Int. Ed. Engl. 33, 2059, 1994; Carell et al., Angew. Chem. Int. Ed. Engl. 33, 2061; Gallop et al., J. Med. Chem. 37, 1233, 1994). Libraries of compounds can be presented in solution (*see, e.g.,* Houghten, BioTechniques 13, 412-421, 1992), or on beads (Lam, Nature 354, 82-84, 1991), chips (Fodor, Nature 364, 555-556, 1993), bacteria or spores (Ladner, U.S. Patent 5,223,409), plasmids (Cull et al., Proc. Natl. Acad. Sci. U.S.A. 89, 1865-1869, 1992), or phage (Scott & Smith, Science 249, 386-390, 1990; Devlin, Science 249, 404-406, 1990); Cwirla et al., Proc. Natl. Acad. Sci. 97, 6378-6382, 1990; Felici, J. Mol. Biol. 222, 301-310, 1991; and Ladner, U.S. Patent 5,223,409).

*High Throughput Screening*

Test compounds can be screened for the ability to bind to GPCR polypeptides or polynucleotides or to affect GPCR activity or GPCR gene expression using high throughput screening. Using high throughput screening, many discrete compounds can be tested in parallel so that large numbers of test compounds can be quickly screened. The most widely established techniques utilize 96-well microtiter plates. The wells of the microtiter plates typically require assay volumes that range from 50 to 500 μl. In addition to the plates, many instruments, materials, pipettors, robotics, plate washers, and plate readers are commercially available to fit the 96-well format.

Alternatively, "free format assays," or assays that have no physical barrier between samples, can be used. For example, an assay using pigment cells (melanocytes) in a simple homogeneous assay for combinatorial peptide libraries is described by Jayawickreme et al., Proc. Natl. Acad. Sci. U.S.A. 19, 1614-18 (1994). The cells are placed under agarose in petri dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is described by Chelsky, "Strategies for Screening Combinatorial Libraries: Novel and Traditional Approaches," reported at the First Annual Conference of The Society for Biomolecular Screening in Philadelphia, Pa. (Nov. 7-10, 1995). Chelsky placed a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV-light. Compounds that inhibited the enzyme were observed as local zones of inhibition having less color change.

Yet another example is described by Salmon et al., Molecular Diversity 2, 57-63 (1996). In this example, combinatorial libraries were screened for compounds that had cytotoxic effects on cancer cells growing in agar.

Another high throughput screening method is described in Beutel et al., U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule which binds to and occupies the active site of the GPCR polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known GPCRs and analogues or derivatives thereof.

In binding assays, either the test compound or the GPCR polypeptide can comprise a detectable label, such as a fluorescent, radioisotopic, chemiluminescent, or enzymatic label, such as horseradish peroxidase, alkaline phosphatase, or luciferase. Detection of a test compound which is bound to the GPCR polypeptide can then be accomplished, for example, by direct counting of radioemmission, by scintillation counting, or by determining conversion of an appropriate substrate to a detectable product.

Alternatively, binding of a test compound to a GPCR polypeptide can be determined without labeling either of the interactants. For example, a microphysiometer can be used to detect binding of a test compound with a GPCR polypeptide. A microphysiometer (*e.g.*, Cytosensor™) is an analytical instrument that measures the rate at which a cell acidifies its environment using a light-addressable potentiometric sensor (LAPS). Changes in this acidification rate can be used as an indicator of the interaction between a test compound and a GPCR polypeptide (McConnell et al., Science 257, 1906-1912, 1992).

Determining the ability of a test compound to bind to a GPCR polypeptide also can be accomplished using a technology such as real-time Bimolecular Interaction Analysis (BIA) (Sjolander & Urbaniczky, Anal. Chem. 63, 2338-2345, 1991, and Szabo et al., Curr. Opin. Struct. Biol. 5, 699-705, 1995). BIA is a technology for studying biospecific interactions in real time, without labeling any of the interactants (*e.g.*, BIAcore™). Changes in the optical phenomenon surface plasmon resonance (SPR) can be used as an indication of real-time reactions between biological molecules.

In yet another aspect of the invention, a GPCR polypeptide can be used as a "bait protein" in a two-hybrid assay or three-hybrid assay (see, *e.g.*, U.S. Patent 5,283,317; Zervos et al., Cell 72, 223-232, 1993; Madura et al., J.

Biol. Chem. 268, 12046-12054, 1993; Bartel et al., BioTechniques 14, 920-924, 1993; Iwabuchi et al., Oncogene 8, 1693-1696, 1993; and Brent W094/10300), to identify other proteins which bind to or interact with the GPCR polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a GPCR polypeptide can be fused to a polynucleotide encoding the DNA binding domain of a known transcription factor (*e.g.*, GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene *(e.g.,* LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the GPCR polypeptide.

It may be desirable to immobilize either the GPCR polypeptide (or polynucleotide) or the test compound to facilitate separation of bound from unbound forms of one or both of the interactants, as well as to accommodate automation of the assay. Thus, either the GPCR polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach the GPCR polypeptide (or poly nucleotide) or test compound to a solid support, including use of covalent and noncovalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a GPCR polypeptide (or polynucleotide) can be accomplished in any vessel suitable for containing the reactants. Examples of such vessels include microtiter plates, test tubes, and microcentrifuge tubes.

In one embodiment, the GPCR polypeptide is a fusion protein comprising a domain that allows the GPCR polypeptide to be bound to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed GPCR polypeptide; the mixture is then incubated under conditions conducive to complex formation *(e.g.,* at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either a GPCR polypeptide (or polynucleotide) or a test compound can be immobilized utilizing conjugation of biotin and streptavidin. Biotinylated GPCR polypeptides (or polynucleotides) or test compounds can be prepared from biotin-NHS(N-hydroxysuccinimide) using techniques well known in the art (*e.g.*, biotinylation kit, Pierce Chemicals, Rockford, Ill.) and immobilized in the wells of streptavidin-coated 96 well plates (Pierce Chemical). Alternatively, antibodies which specifically bind to a GPCR polypeptide, polynucleotide, or a test compound, but which do not interfere with a desired binding site, such as the active site of the GPCR polypeptide, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to the GPCR polypeptide or test compound, enzyme-linked assays which rely on detecting an activity of the GPCR polypeptide, and SDS gel electrophoresis under non-reducing conditions.

Screening for test compounds which bind to a GPCR polypeptide or polynucleotide also can be carried out in an intact cell. Any cell which comprises a GPCR poly peptide or polynucleotide can be used in a cell-based assay system. A GPCR polynucleotide can be naturally occurring in the cell or can be introduced using techniques such as those described above. Binding of the test compound to a GPCR polypeptide or polynucleotide is determined as described above.

*Functional Assays*

Test compounds can be tested for the ability to increase or decrease a biological effect of a GPCR polypeptide. Such biological effects can be determined using the functional assays described in the specific examples, below. Functional assays can be carried out after contacting either a purified GPCR polypeptide, a cell membrane preparation, or an intact cell with a test compound. A test compound which decreases a functional activity of a GPCR by at least about 10, preferably about 50, more preferably about 75, 90, or 100% is identified as a potential agent for decreasing GPCR activity. A test compound which increases GPCR activity by at least about 10, preferably about 50, more preferably about 75, 90, or 100% is identified as a potential agent for increasing GPCR activity.

One such screening procedure involves the use of melanophores which are transfected to express a GPCR polypeptide. Such a screening technique is described in WO 92/01810 published Feb. 6, 1992. Thus, for example, such an assay may be employed for screening for a compound which inhibits activation of the receptor polypeptide by contacting the melanophore cells which comprise the receptor with both the receptor ligand and a test compound to be screened. Inhibition of the signal generated by the ligand indicates that a test compound is a potential antagonist for the receptor, *i.e.,* inhibits activation of the receptor. The screen may be employed for identifying a test compound which activates the receptor by contacting such cells with compounds to be screened and determining whether each test compound generates a signal, *i.e.,* activates the receptor.

Other screening techniques include the use of cells which express a human GPCR polypeptide (for example, transfected CHO cells) in a system which measures extracellular pH changes caused by receptor activation (*see, e.g.,* Science 246, 181-296, 1989). For example, test compounds may be contacted with a cell which expresses a human GPCR polypeptide and a second messenger response, e.g., signal transduction or pH changes, can be measured to determine whether the test compound activates or inhibits the receptor.

Another such screening technique involves introducing RNA encoding a human GPCR polypeptide into *Xenopus* oocytes to transiently express the receptor. The transfected oocytes can then be contacted with the receptor ligand and a test compound to be screened, followed by detection of inhibition or activation of a calcium signal in the case of screening for test compounds which are thought to inhibit activation of the receptor.

Another screening technique involves expressing a human GPCR polypeptide in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

Details of functional assays such as those described above are provided in the specific examples, below.

*Gene Expression*

In another embodiment, test compounds which increase or decrease GPCR gene expression are identified. A GPCR polynucleotide is contacted with a test compound, and the expression of an RNA or polypeptide product of the GPCR polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of GPCR mRNA or polypeptide expression in the cells can be determined by methods well known in the art for detecting mRNA or polypeptide. Either qualitative or quantitative methods can be used. The presence of polypeptide products of a GPCR polynucleotide can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labeled amino acids into a GPCR polypeptide.

Such screening can be carried out either in a cell-free assay system or in an intact cell. Any cell which expresses a GPCR polynucleotide can be used in a cell-based assay system. The GPCR polynucleotide can be naturally occurring in the cell or can be introduced using techniques such as those described above. Either a primary culture or an established cell line, such as CHO or human embryonic kidney 293 cells, can be used.

*Pharmaceutical Compositions*

The invention also provides pharmaceutical compositions which can be administered to a patient to achieve a therapeutic effect. Pharmaceutical compositions of the invention can comprise, for example, a GPCR polypeptide, GPCR polynucleotide, antibodies which specifically bind to a GPCR polypeptide, or mimetics, agonists, antagonists, or inhibitors of a GPCR polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically-acceptable carriers comprising excipients and auxiliaries which facilitate processing of the active compounds into preparations which can be used pharmaceutically. Pharmaceutical compositions of the invention can be administered by any number of routes including, but not limited to, oral, intravenous, intramuscular, intra-arterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, *i.e.,* dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents which increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions of the present invention can be manufactured in a manner that is known in the art, *e.g.*, by means of conventional mixing, dissolving, granulating, dragee-making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilized powder which can contain any or all of the following: 1-50 mM histidine, 0.1%-2% sucrose, and 2-7% mannitol, at a pH range of 4.5 to 5.5, that is combined with buffer prior to use.

Further details on techniques for formulation and administration can be found in the latest edition of REMINGTON'S PHARMACEUTICAL SCIENCES (Maack Publishing Co., Easton, Pa.). After pharmaceutical compositions have been prepared, they can be placed in an appropriate container and labeled for treatment of an indicated condition. Such labeling would include amount, frequency, and method of administration.

*Therapeutic Indications and Methods*

GPCRs are ubiquitous in the mammalian host and are responsible for many biological functions, including many pathologies. Accordingly, it is desirable to find compounds and drugs which stimulate a GPCR on the one hand and which can inhibit the function of a GPCR on the other hand. For example, compounds which activate a GPCR may be employed for therapeutic purposes, such as the treatment of COPD, cardiovascular disorders, cancer, urinary disorders, obesity, diabetes, CNS disorders, liver disorders, kidney disorders, disorders of the secretory organs, asthma, and hematological disorders. In particular, compounds which activate GPCRs are useful in treating various cardiovascular ailments such as caused by the lack of pulmonary blood flow or hypertension. In addition these compounds may also be used in treating various physiological disorders relating to abnormal control of fluid and electrolyte homeostasis and in diseases associated with abnormal angiotensin-induced aldosterone secretion.

In general, compounds which inhibit activation of a GPCR can be used for a variety of therapeutic purposes, for example, for the treatment of hypotension and/or hypertension, angina pectoris, myocardial infarction, ulcers, asthma, allergies, benign prostatic hypertrophy, and psychotic and neurological disorders including schizophrenia, manic excitement, depression, delirium, dementia or severe mental retardation, dyskinesias, such as Huntington's disease or Tourett's syndrome, among others. Compounds which inhibit GPCRs also are useful in reversing endogenous anorexia, in the control of bulimia, and in treating various cardiovascular ailments such as caused by excessive pulmonary blood flow or hypotension. In particular, regulation of GPCR can be used to treat anxiety, depression, hypertension, migraine, compulsive disorders, schizophrenia, autism, neurodegenerative disorders, such as Alzheimer's disease, Parkinsonism, and Huntington's chorea, and cancer chemotherapy-induced vomiting, as well as sleep and eating disorders, pain control, disorders involving regulation of body temperature and blood pressure.

*Obesity*

This gene, translated proteins and agents which modulate this gene or portions of the gene or its products are useful for treating obesity, overweight, anorexia, cachexia, wasting disorders, appetite suppression, appetite enhancement, increases or decreases in satiety, modulation of body weight, and/or other eating disorders such as bulimia. Obesity and overweight are defined as an excess of body fat relative to lean body mass. An increase in caloric intake or a decrease in energy expenditure or both can bring about this imbalance leading to surplus energy being stored as fat. Obesity is associated with important medical morbidities and an increase in mortality. The causes of obesity are poorly understood and may be due to genetic factors, environmental factors or a combination of the two to cause a positive energy balance. In contrast, anorexia and cachexia are characterized by an imbalance in energy intake versus energy expenditure leading to a negative energy balance and weight loss. Agents that either increase energy expenditure and/or decrease energy intake, absorption or storage would be useful for treating obesity, overweight, and associated comorbidities. Agents that either increase energy intake and/or decrease energy expenditure or increase the amount of lean tissue would be useful for treating cachexia, anorexia and wasting disorders.

This gene, translated proteins and agents which modulate this gene or portions of the gene or its products also are useful for treating obesity/overweight-associated comorbidities including hypertension, type 2 diabetes, coronary artery disease, hyper-lipidemia, stroke, gallbladder disease, gout, osteoarthritis, sleep apnea and respiratory problems, some types of cancer including endometrial, breast, prostate and colon cancer, thrombolic disease, polycystic ovarian syndrome; reduced fertility, complications of pregnancy, menstrual irregularities, hirsutism, stress incontinence, and depression.

*Cancer*

Human GPCRs provide a potential target for treating cancer. Cancer is a disease fundamentally caused by oncogenic-cellular transformation. There are several hallmarks of transformed cells that distinguish them from their normal counterparts and underlie the pathophysiology of cancer. These include uncontrolled cellular proliferation, unresponsiveness to normal death-inducing signals (immortalization), increased cellular motility and invasiveness, increased ability to recruit blood supply through induction of new blood vessel formation (angiogenesis), genetic instability, and dysregulated gene expression. Various combinations of these aberrant physiologies, along with the acquisition of drug-resistance frequently lead to an intractable disease state in which organ failure and patient death ultimately ensue.

Most standard cancer therapies target cellular proliferation and rely on the differential proliferative capacities between transformed and normal cells for their efficacy. This approach is hindered by the facts that several important normal cell types are also highly proliferative and that cancer cells frequently become resistant to these agents. Thus, the therapeutic indices for traditional anti-cancer therapies rarely exceed 2.0.

The advent of genomics-driven molecular target identification has opened up the possibility of identifying new cancer-specific targets for therapeutic intervention that will provide safer, more effective treatments for cancer patients. Thus, newly discovered tumor-associated genes and their products can be tested for their role(s) in disease and used as tools to discover and develop innovative therapies. Genes playing important roles in any of the physiological processes outlined above can be characterized as cancer targets.

Genes or gene fragments identified through genomics can readily be expressed in one or more heterologous expression systems to produce functional recombinant proteins. These proteins are characterized *in vitro* for their biochemical properties and then used as tools in high-throughput molecular screening programs to identify chemical modulators of their biochemical activities. Agonists and/or antagonists of target protein activity can be identified in this manner and subsequently tested in cellular and *in vivo* disease models for anti-cancer activity. Optimization of lead compounds with iterative testing in biological models and detailed pharmacokinetic and toxicological analyses form the basis for drug development and subsequent testing in humans.

*Diabetes*

Diabetes also can be potentially treated by regulating the activity of human GPCR. Diabetes mellitus is a common metabolic disorder characterized by an abnormal elevation in blood glucose, alterations in lipids and abnormalities (complications) in the cardiovascular system, eye, kidney and nervous system. Diabetes is divided into two separate diseases: type 1 diabetes (juvenile onset) that results from a loss of cells which make and secrete insulin, and type 2 diabetes (adult onset) which is caused by a defect in insulin secretion and a defect in insulin action.

Type 1 diabetes is initiated by an autoimmune reaction that attacks the insulin secreting cells (beta cells) in the pancreatic islets. Agents that prevent this reaction from occurring or that stop the reaction before destruction of the beta cells has been accomplished are potential therapies for this disease. Other agents that induce beta cell proliferation and regeneration are also potential therapies.

Type II diabetes is the most common of the two diabetic conditions (6% of the population). The defect in insulin secretion is an important cause of the diabetic condition and results from an inability of the beta cell to properly detect and respond to rises in blood glucose levels with insulin release. Therapies that increase the response by the beta cell to glucose would offer an important new treatment for this disease.

The defect in insulin action in Type II diabetic subjects is another target for therapeutic intervention. Agents that increase the activity of the insulin receptor in muscle, liver and fat will cause a decrease in blood glucose and a normalization of plasma lipids. The receptor activity can be increased by agents that directly stimulate the receptor or that increase the intracellular signals from the receptor. Other therapies can directly activate the cellular end process, *i.e.* glucose transport or various enzyme systems, to generate an insulin-like effect and therefore a produce beneficial outcome. Because overweight subjects have a greater susceptibility to Type II diabetes, any agent that reduces body weight is a possible therapy.

Both Type I and Type diabetes can be treated with agents that mimic insulin action or that treat diabetic complications by reducing blood glucose levels. Likewise agents that reduces new blood vessel growth can be used to treat the eye complications that develop in both diseases.

*COPD*

Chronic obstructive pulmonary (or airways) disease (COPD) is a condition defined physiologically as airflow obstruction that generally results from a mixture of emphysema and peripheral airway obstruction due to chronic bronchitis (Senior & Shapiro, Pulmonary Diseases and Disorders, 3d ed., New York, McGraw-Hill, 1998, pp. 659-681, 1998; Barnes, Chest 117, 10S-14S, 2000). Emphysema is characterized by destruction of alveolar walls leading to abnormal enlargement of the air spaces of the lung. Chronic bronchitis is defined clinically as the presence of chronic productive cough for three months in each of two successive years. In COPD, airflow obstruction is usually progressive and is only partially reversible. By far the most important risk factor for development of COPD is cigarette smoking, although the disease does occur in non-smokers.

Chronic inflammation of the airways is a key pathological feature of COPD (Senior & Shapiro, 1998). The inflammatory cell population comprises increased numbers of macrophages, neutrophils, and $CD8^+$ lymphocytes. Inhaled irritants, such as cigarette smoke, activate macrophages which are resident in the respiratory tract, as well as epithelial cells leading to release of chemokines (*e.g.*, interleukin-8) and other chemotactic factors. These chemotactic factors act to increase the neutrophil/monocyte trafficking from the blood into the lung tissue and airways. Neutrophils and monocytes recruited into the airways can release a variety of potentially damaging mediators such as proteolytic enzymes and reactive oxygen species. Matrix degradation and emphysema, along with airway wall thickening, surfactant dysfunction, and mucus hypersecretion, all are potential sequelae of this inflammatory response that lead to impaired airflow and gas exchange.

Several GPCRs have been implicated in the pathology of COPD. For example, the chemokine IL-8 acts through CXCR1 and CXCR2, and antagonists for these receptors are under investigation as therapeutics for COPD. Members of the P2Y family of metabotropic receptors may play key roles in normal pulmonary function. In particular, the $P2Y_2$ receptor is believed to be involved in the regulation of mucociliary clearance mechanisms in the lung, and agonists of this receptor may stimulate airway mucus clearance in patients with chronic bronchitis (Yerxa Johnson, Drugs of the Future 24, 759-769, 1999). GPCRs, therefore, are therapeutic targets for COPD, and the identification of additional members of existing GPCR families or of novel GPCRs would yield further attractive targets.

*Cardiovascular disorders*

Cardiovascular diseases include the following disorders of the heart and the vascular system: congestive heart failure, myocardial infarction, ischemic diseases of the heart, all kinds of atrial and ventricular arrhythmias, hypertensive vascular diseases, and peripheral vascular diseases.

Heart failure is defined as a pathophysiologic state in which an abnormality of cardiac function is responsible for the failure of the heart to pump blood at a rate commensurate with the requirement of the metabolizing tissue. It includes all forms of pumping failure, such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included, as well as the acute treatment of MI and the prevention of complications.

Ischemic diseases are conditions in which the coronary flow is restricted resulting in a perfusion which is inadequate to meet the myocardial requirement for oxygen. This group of diseases includes stable angina, unstable angina, and asymptomatic ischemia.

Arrhythmias include all forms of atrial and ventricular tachyarrhythmias (atrial tachycardia, atrial flutter, atrial fibrillation, atrio-ventricular reentrant tachycardia, preexcitation syndrome, ventricular tachycardia, ventricular flutter, and ventricular fibrillation), as well as bradycardic forms of arrhythmias.

Vascular diseases include primary as well as all kinds of secondary arterial hypertension (renal, endocrine, neurogenic, others). The disclosed gene and its product may be used as drug targets for the treatment of hypertension as well as for the prevention of all complications. Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon, and venous disorders.

*CNS disorders*

Central and peripheral nervous system disorders also can be treated, such as primary and secondary disorders after brain injury, disorders of mood, anxiety disorders, disorders of thought and volition, disorders of sleep and wakefulness, diseases of the motor unit, such as neurogenic and myopathic disorders, neurodegenerative disorders such as Alzheimer's and Parkinson's disease, and processes of peripheral and chronic pain.

Pain that is associated with CNS disorders also can be treated by regulating the activity of human GPCR. Pain which can be treated includes that associated with central nervous system disorders, such as multiple sclerosis, spinal cord injury, sciatica, failed back surgery syndrome, traumatic brain injury, epilepsy, Parkinson's disease, post-stroke, and vascular lesions in the brain and spinal cord (*e.g.*, infarct, hemorrhage, vascular malformation). Non-central neuropathic pain includes that associated with post mastectomy pain, reflex sympathetic dystrophy (RSD), trigeminal neuralgiaradioculopathy, post-surgical pain, HIV/AIDS related pain, cancer pain, metabolic neuropathies (*e.g.*, diabetic neuropathy, vasculitic neuropathy secondary to connective tissue disease), paraneoplastic polyneuropathy associated, for example, with carcinoma of lung, or leukemia, or lymphoma, or carcinoma of prostate, colon or stomach, trigeminal neuralgia, cranial neuralgias, and post-herpetic neuralgia. Pain associated with cancer and cancer treatment also can be treated, as can headache pain (for example, migraine with aura, migraine without aura, and other migraine disorders), episodic and chronic tension-type headache, tension-type like headache, cluster headache, and chronic paroxysmal hemicrania

*Asthma*

Allergy is a complex process in which environmental antigens induce clinically adverse reactions. The inducing antigens, called allergens, typically elicit a specific IgE response and, although in most cases the allergens themselves have little or no intrinsic toxicity, they induce pathology when the IgE response in turn elicits an IgE-dependent or T cell-dependent hypersensitivity reaction. Hypersensitivity reactions can be local or systemic and typically occur within minutes of allergen exposure in individuals who have previously been sensitized to an allergen. The hypersensitivity reaction of allergy develops when the allergen is recognized by IgE antibodies bound to specific receptors on the surface of effector cells, such as mast cells, basophils, or eosinophils, which causes the activation of the effector cells and the release of mediators that produce the acute signs and symptoms of the reactions. Allergic diseases include asthma, allergic rhinitis (hay fever), atopic dermatitis, and anaphylaxis.

Asthma is though to arise as a result of interactions between multiple genetic and environmental factors and is characterized by three major features: 1) intermittent and reversible airway obstruction caused by bronchoconstriction, increased mucus production, and thickening of the walls of the airways that leads to a narrowing of the airways, 2) airway hyperresponsiveness caused by a decreased control of airway caliber, and 3) airway inflammation. Certain cells are critical to the inflammatory reaction of asthma and they include T cells and antigen presenting cells, B cells that produce IgE, and mast cells, basophils, eosinophils, and other cells that bind IgE. These effector cells accumulate at the site of allergic reaction in the airways and release toxic products that contribute to the acute pathology and eventually to the tissue destruction related to the disorder. Other resident cells, such as smooth muscle cells, lung epithelial cells, mucus-producing cells, and nerve cells may also be abnormal in individuals with asthma and may contribute to the pathology. While the airway obstruction of asthma, presenting clinically as an intermittent wheeze and shortness of breath, is generally the most pressing symptom of the disease requiring immediate treatment, the inflammation and tissue destruction associated with the disease can lead to irreversible changes that eventually make asthma a chronic disabling disorder requiring long-term management.

Despite recent important advances in our understanding of the pathophysiology of asthma, the disease appears to be increasing in prevalence and severity (Gergen and Weiss, Am. Rev. Respir. Dis. 146, 823-24, 1992). It is estimated that 30-40% of the population suffer with atopic allergy, and 15% of children and 5% of adults in the population suffer from asthma (Gergen and Weiss, 1992). Thus, an enormous burden is placed on our health care resources. However, both diagnosis and treatment of asthma are difficult. The severity of lung tissue inflammation is not easy to measure and the symptoms of the disease are often indistinguishable from those of respiratory infections, chronic respiratory inflammatory disorders, allergic rhinitis, or other respiratory disorders. Often, the inciting allergen cannot be determined, making removal of the causative environmental agent difficult. Current pharmacological treatments suffer their own set of disadvantages. Commonly used therapeutic agents, such as beta agonists, can act as symptom relievers to transiently improve pulmonary function, but do not affect the underlying inflammation. Agents that can reduce the underlying inflammation, such as anti-inflammatory steroids, can have major drawbacks that range from immunosuppression to bone loss (Goodman and Gilman's THE PHARMACOLOGIC BASIS OF THERAPEUTICS, Seventh Edition, MacMillan Publishing Company, NY, USA, 1985). In addition, many of the present therapies, such as inhaled corticosteroids, are short-lasting, inconvenient to use, and must be used often on a regular basis, in some cases for life, making failure of patients to comply with the treatment a major problem and thereby reducing their effectiveness as a treatment.

Because of the problems associated with conventional therapies, alternative treatment strategies have been evaluated. Glycophorin A (Chu and Sharom, Cell. Immunol. 145, 223-39, 1992), cyclosporin (Alexander et al., Lancet 339, 324-28, 1992), and a nonapeptide fragment of IL-2 (Zav'yalov et al., Immunol. Lett. 31, 285-88, 1992) all inhibit interleukin-2 dependent T lymphocyte proliferation; however, they are known to have many other effects. For example, cyclosporin is used as a immuno-suppressant after organ transplantation. While these agents may represent alternatives to steroids in the treatment of asthmatics, they inhibit interleukin-2 dependent T lymphocyte proliferation and potentially critical immune functions associated with homeostasis. Other treatments that block the release or activity of mediators of bronchochonstriction, such as cromones or anti-leukotrienes, have recently been introduced for the treatment of mild asthma, but they are expensive and not effective in all patients and it is unclear whether they have any effect on the chronic changes associated with asthmatic inflammation. What is needed in the art is the identification of a treatment that can act in pathways critical to the development of asthma that both blocks the episodic attacks of the disorder and preferentially dampens the hyperactive allergic immune response without immunocompromising the patient.

*Hematological disorders*

Guanin-nucleotide-binding (G-) protein coupled receptors (GPCRs) are involved in various hematopoietic processes, e.g. proliferation, differentiation, survival, migration and homing of precursor cells to hematopoietic and lymphoid tissues. Dysfunction of GPCRs may lead to inappropriate production of blood cells resulting in diseases like anemia, leukopenia, thrombocytopenia or different forms of leukemia.

GPCRs also play a role in diverse functions of circulating white blood cells, e.g. activation of immune response in lymphocytes, cytokine production by monocytes and chemotaxis of granulocytes. Dysregulated GPCR function may contribute to compromised immune function, allergy and other pathologic conditions of the host defense system.

In circulating platelets GPCRs mediate activation resulting in platelet aggregation and secretion of mediators eventually leading to hemostasis. Modulation of GPCR function in platelets by pharmacologic or molecular genetic methods has demonstrated key roles of GPCRs in thrombotic diseases and in bleeding disorders thus proving that GPCRs represent appropriate therapeutic drug targets.

GPCRs are activated by binding of various classes of ligands ranging from small molecules like serotonin to high molecular peptides like chemokines. Some GPCRs are activated by proteolytic cleavage, e.g. by thrombin. Upon ligand binding, signals from GPCRs are mediated via heterotrimeric G-proteins with the class of the $\alpha$-subunit determining the further pathway signal transduction.

It is conceivable that genes coding for "non-standard" GPCRs with unidentified ligands or unknown intracellular signal transduction pathways (e.g. novel G-proteins) or that GPCRs from classes as yet not associated with the hematopoietic and hemostatic systems will be identified. Therefore it is reasonable to assume that GPCRs specifically expressed in hematopoietic precursor or circulating blood cells represent good targets for therapeutic interventions in dysfunctions of hematopoiesis and hemostasis. Yang M., Srikaiatkhachom A, Antony M., Chong B.H.; Blood Coagul. Fibrinolysis 1996, 127-33; Arai H., Tsou C.L., Charo I.F.; Proc. Natl. Acad. Sci. USA 94, 14495-14499, 1997; Aragay A.M., Quick M.W.; J. Biol. Chem. 274, 4807-4815, 1999; Davignon I., Catalina M.D., Smith D., Montgomery J., Croy J., Siegelman M., Wilkie T.M.; Mol. Cell. Biol. 20, 797-804, 2000; Wiesmann A., Spangrude G.J.; Exp. Hematol. 27, 946-955, 1999;

Van Brocklyn J.R, Graler M.H., Bernhardt G., Hobson J.P., Lipp M., Spiegel S.; Blood 95, 2624-2629, 2000; Brass L.F.; J. Clin. Invest. 104, 1663-1665, 1999; Coughlin S.R; Proc. Natl. Acad. Sci. USA 96, 11023-11027, 1999.

*Urinary disorders*

*Urinary Incontinence.* Urinary incontinence (UI) is the involuntary loss of urine. Urge urinary incontinence (UUI) is one of the most common types of UI together with stress urinary incontinence (SUI), which is usually caused by a defect in the urethral closure mechanism. UUI is often associated with neurological disorders or diseases causing neuronal damage, such as dementia, Parkinson's disease, multiple sclerosis, stroke, and diabetes, although it also occurs in individuals with no such disorders. One of the usual causes of UUI is overactive bladder (OAB), which is a medical condition referring to the symptoms of frequency and urgency derived from abnormal contractions and instability of the detrusor muscle.

There are several medications for urinary incontinence on the market today, mainly to help treating UUI. Therapy for OAB is focused on drugs that affect peripheral neural control mechanisms or those that act directly on bladder detrusor smooth muscle contraction, with a major emphasis on development of anticholinergic agents. These agents can inhibit the parasympathetic nerves, which control bladder voiding, or can exert a direct spasmolytic effect on the detrusor muscle of the bladder. This results in a decrease in intravesicular pressure, an increase in capacity, and a reduction in the frequency of bladder contraction. Orally active anticholinergic drugs, such as propantheline (ProBanthine), tolterodine tartrate (Detrol), and oxybutynin (Ditropan), are the most commonly prescribed drugs. However, their most serious drawbacks are unacceptable side effects, such as dry mouth, abnormal visions, constipation, and central nervous system disturbances. These side effects lead to poor compliance. Dry mouth symptoms alone are responsible for a 70% non-compliance rate with oxybutynin. The inadequacies of present therapies highlight the need for novel, efficacious, safe, orally available drugs that have fewer side effects.

*Benign Prostatic Hyperplasia*

Benign prostatic hyperplasia (BPH) is the benign nodular hyperplasia of the periure-thral prostate gland commonly seen in men over the age of 50. The overgrowth occurs in the central area of the prostate called the transition zone, which wraps around the urethra. BPH causes variable degrees of bladder outlet obstruction, resulting in progressive lower urinary tract syndromes (LUTS) characterized by urinary frequency, urgency, and nocturia due to incomplete emptying and rapid refilling of the bladder. The actual cause of BPH is unknown but may involve age-related alterations in balance of steroidal sex hormones.

Selective α1-adrenoceptor antagonists, such as prazosin, indoramin, and tamsulosin, are used as an adjunct in the symptomatic treatment of urinary obstruction caused by BPH, although they do not affect on the underlying cause of BPH. In BPH, increased sympathetic tone exacerbates the degree of obstruction of the urethra through contraction of prostatic and urethral smooth muscle. These compounds inhibit sympathetic activity, thereby relaxing the smooth muscle of the urinary tract. Uroselective α1-antagonists and α1-antagonists with high tissue selectivity for lower urinary tract smooth muscle that do not provoke hypotensive side-effects should be developed for the treatment.

Drugs blocking dihydrotestosterone have been used to reduce the size of the prostate. 5α-reductase inhibitors such as finasteride are prescribed for BPH. These agents selectively inhibit 5α-reductase which mediates conversion of testosterone to dihydrotestosterone, thereby reducing plasma dihydrotestosterone levels and, thus, prostate growth. The 5a-reductase inhibitors do not bind to androgen receptors and do not affect testosterone levels, nor do they possess feminizing side-effects.

Androgen receptor antagonists are used for the treatment of prostatic hyperplasia due to excessive action or production of testosterone. Various antiandrogens are under investigation for BPH including chlormadione derivatives with no estrogenic activity, orally-active aromatase inhibitors, and luteinizing hormone-releasing hormone (LHRH) analogues.

*Liver disorders*

All chronic liver diseases cause the development of fibrosis in the liver. Fibrosis is a programmed uniform wound healing response. The most important chronic liver diseases are viral hepatitis B and C and alcohol-induced liver disease. Between 10 and 30 % of patients affected develop cirrhosis as a late complication. Liver cirrhosis has a 5-year survival rate of 50%. Deaths from liver cirrhosis occurrs at an average age of only 60 years. It is the 9th largest cause of death in the USA.

Toxic damage or injury caused by foreign proteins cause the deposition of extracellular matrix such as collagen, fibronectin and laminin. The common mechanism is the activation and transformation of vitamin-A storing hepatic stellate cells (Ito cells) into matrix producing myofibroblasts. These proliferate and fill the extracellular Space of Disse with extracellular matrix. This process contains para- and autocrine activation steps, which cause it to become auto-perpetuated, if the process of injury is sustained for a long period of time. It causes a slowly progressing shunt, which reduces the perfusion of the liver with portal and arterial blood. This results in a loss of liver function. Build-up of an increased diffusion barrier by a loss of fenestration in the sinusoidal endothelium. This corroborates the loss in function. Portal hypertension with the frequent complication of esophagal bleeding.
Causes of death:

30% hepatic coma
30% esophagal bleeding
30% primary hepatic carcinoma

Liver fibrosis and cirrhosis caused by chronic degenerative diseases of the liver such as viral hepatitis, alcohol hepatitis, autoimmune hepatitis, primary biliary cirrhosis, cystic fibrosis, hemochromatosis, Wilson's disease, non-alcoholic steato-hepatitis and others can be treated. Possible other indications are systemic sclerosis, pulmonary fibrosis, pancreatic fibrosis, myocardial fibrosis, and prostatic fibrosis.
Liver fibrosis pathogenesis involves altered proliferation and gene expression of multiple cell types such as hepatic stellate cells and cholangiocyte. These changes cause altered collagen content and altered connective tissue deposition, water retention, choleresis etc. Many ligand-receptor interaction processes are involved in these changes. Among the ligands are vasopressin, secretin, vasoactive intestinal peptide, etc., all using G protein-coupled receptor mediated signal transduction pathways. Endothelin, bradykinin, angiotensins and purines, all signaling via G protein coupled receptors, have been demonstrated to have the potential to modulate liver fibrosis and ensueing complications such as portal hypertension, cardiovascular and electrolyte dysregulation. It can be assumed that novel receptors of this class can be detected. G protein-coupled receptor is a proven target class for many indications. Therefore novel G protein coupled receptors will be good targets for therapeutic intervention of liver fibrosis. See Kojima et al., J Hepatol 2000 Jan;32(1):43-50; Pinzani et al., Semin Liver Dis 1999;19(4):397-410; Cai et al., Anticancer Res 1999 May-Jun;19 (3B):2243-7; Wirth et al., Eur J Pharmacol 1997 Oct 15;337(1):45-53; Gatta et al., Ital J Gastroenterol Hepatol 1999 May;31(4):326-45; Albrecht et al.; Drug Targeting 6 (2) (1998): 105-117; Ramos et al., Pathol Int 1994 Sep;44(9):655-61; Hidalgo et al., J. Autonom. Pharmacol. 18 (1) (1998): 31-37.
This invention further pertains to the use of novel agents identified by the screening assays described above. Accordingly, it is within the scope of this invention to use a test compound identified as described herein in an appropriate animal model. For example, an agent identified as described herein (*e.g.*, a modulating agent, an antisense nucleic acid molecule, a specific antibody, ribozyme, or a GPCR poly peptide binding molecule) can be used in an animal model to determine the efficacy, toxicity, or side effects of treatment with such an agent Alternatively, an agent identified as described herein can be used in an animal model to determine the mechanism of action of such an agent Furthermore, this invention pertains to uses of novel agents identified by the above-described screening assays for treatments as described herein.
A reagent which affects GPCR activity can be administered to a human cell, either *in vitro* or *in vivo,* to reduce GPCR activity. The reagent preferably binds to an expression product of a human GPCR gene. If the expression product is a protein, the reagent is preferably an antibody. For treatment of human cells *ex vivo,* an antibody can be added to a preparation of stem cells other than human embryonic stem cells which have been removed from the body. The cells can then be replaced in the same or another human body, with or without clonal propagation, as is known in the art.
In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.
A liposome useful in the present invention comprises a lipid composition that is capable of fusing with the plasma membrane of the targeted cell to deliver its contents to the cell. Preferably, the transfection efficiency of a liposome is about 0.5 $\mu$g of DNA per 16 nmole of liposome delivered to about $10^6$ cells, more preferably about 1.0 $\mu$g of DNA per 16 nmole of liposome delivered to about $10^6$ cells, and even more preferably about 2.0 $\mu$g of DNA per 16 nmol of liposome delivered to about $10^6$ cells. Preferably, a liposome is between about 100 and 500 nm, more preferably between about 150 and 450 nm, and even more preferably between about 200 and 400 nm in diameter.
Suitable liposomes for use in the present invention include those liposomes standardly used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a tumor cell, such as a tumor cell ligand exposed on the outer surface of the liposome.

Complexing a liposome with a reagent such as an antisense oligonucleotide or ribozyme can be achieved using methods which are standard in the art (see, for example, U.S. Patent 5,705,151). Preferably, from about 0.1 μg to about 10 μg of polynucleotide is combined with about 8 nmol of liposomes, more preferably from about 0.5 μg to about 5 μg of polynucleotides are combined with about 8 nmol liposomes, and even more preferably about 1.0 μg of polynucleotides is combined with about 8 nmol liposomes.

In another embodiment, antibodies can be delivered to specific tissues *in vivo* using receptor-mediated targeted delivery. Receptor-mediated DNA delivery techniques are taught in, for example, Findeis et al. Trends in Biotechnol. 11, 202-05 (1993); Chiou et al., GENE THERAPEUTICS: METHODS AND APPLICATIONS OF DIRECT GENE TRANSFER (J.A. Wolff, ed.) (1994); Wu & Wu, J. Biol. Chem. 263, 621-24 (1988); Wu et al., J. Biol. Chem. 269, 542-46 (1994) ; Zenke et al., Proc. Natl. Acad. Sci. U.S.A. 87, 3655-59 (1990); Wu et al., J. Biol. Chem. 266, 338-42 (1991).

*Determination of a Therapeutically Effective Dose*

The determination of a therapeutically effective dose is well within the capability of those skilled in the art. A therapeutically effective dose refers to that amount of active ingredient which increases or decreases GPCR activity relative to the GPCR activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, *e.g.*, $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of poly nucleotides or polypeptides will be specific to particular cells, conditions, locations, etc.

If the reagent is a single-chain antibody, polynucleotides encoding the antibody can be constructed and introduced into a cell either *ex vivo* or *in vivo* using well-established techniques including, but not limited to, transfcrrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun," and DEAE- or calcium phosphate-mediated transfection.

Effective *in vivo* dosages of an antibody are in the range of about 5 μg to about 50 μg/kg, about 50 μg to about 5 mg/kg, about 100 μg to about 500 μg/kg of patient body weight, and about 200 to about 250 μg/kg of patient body weight. For administration of polynucleotides encoding single-chain antibodies, effective *in vivo* dosages are in the range of about 100 ng to about 200 ng, 500 ng to about 50 mg, about 1μg to about 2 mg, about 5 μg to about 500 μg, and about 20 μg to about 100 μg of DNA.

If the expression product is mRNA, the reagent is preferably an antisense oligonucleotide or a ribozyme. Polynucleotides which express antisense oligonucleotides or ribozymes can be introduced into cells by a variety of methods, as described above.

Preferably, a reagent reduces expression of a GPCR gene or the activity of a GPCR polypeptide by at least about 10, preferably about 50, more preferably about 75, 90, or 100% relative to the absence of the reagent. The effectiveness of the mechanism chosen to decrease the level of expression of a GPCR gene or the activity of a GPCR polypeptide can be assessed using methods well known in the art, such as hybridization of nucleotide probes to GPCR-specific mRNA, quantitative RT-PCR, immunologic detection of a GPCR polypeptide, or measurement of GPCR activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, mammals such as dogs, cats, cows, horses, rabbits, monkeys, and most preferably, humans.

*Diagnostic Methods*

GPCRs also can be used in diagnostic assays for detecting diseases and abnormalities or susceptibility to diseases and abnormalities related to the presence of mutations in the nucleic acid sequences which encode a GPCR. Such diseases, by way of example, are related to cell transformation, such as tumors and cancers, and various cardiovascular disorders, including hypertension and hypotension, as well as diseases arising from abnormal blood flow, abnormal angiotensin-induced aldosterone secretion, and other abnormal control of fluid and electrolyte homeostasis.

Differences can be determined between the cDNA or genomic sequence encoding a GPCR in individuals afflicted with a disease and in normal individuals. If a mutation is observed in some or all of the afflicted individuals but not in normal individuals, then the mutation is likely to be the causative agent of the disease.

Sequence differences between a reference gene and a gene having mutations can be revealed by the direct DNA sequencing method. In addition, cloned DNA segments can be employed as probes to detect specific DNA segments. The sensitivity of this method is greatly enhanced when combined with PCR. For example, a sequencing primer can be used with a double-stranded PCR product or a single-stranded template molecule generated by a modified PCR. The sequence determination is performed by conventional procedures using radiolabeled nucleotides or by automatic sequencing procedures using fluorescent tags.

Genetic testing based on DNA sequence differences can be carried out by detection of alteration in electrophoretic mobility of DNA fragments in gels with or without denaturing agents. Small sequence deletions and insertions can be visualized, for example, by high resolution gel electrophoresis. DNA fragments of different sequences can be distinguished on denaturing formamide gradient gels in which the mobilities of different DNA fragments are retarded in the gel at different positions according to their specific melting or partial melting temperatures (*see, e.g.,* Myers et al., Science 230, 1242, 1985). Sequence changes at specific locations can also be revealed by nuclease protection assays, such as RNase and S 1 protection or the chemical cleavage method *(e.g.,* Cotton et al., Proc. Natl. Acad Sci. USA 85, 4397-4401, 1985). Thus, the detection of a specific DNA sequence can be performed by methods such as hybridization, RNase protection, chemical cleavage, direct DNA sequencing or the use of restriction enzymes and Southern blotting of genomic DNA. In addition to direct methods such as gel-electrophoresis and DNA sequencing, mutations can also be detected by *in situ* analysis.

Altered levels of a GPCR also can be detected in various tissues. Assays used to detect levels of the receptor polypeptides in a body sample, such as blood or a tissue biopsy, derived from a host are well known to those of skill in the art and include radioimmunoassays, competitive binding assays, Western blot analysis, and ELISA assays.

All patents and patent applications cited in this disclosure are expressly incorporated herein by reference. The above disclosure generally describes the present invention. A more complete understanding can be obtained by reference to the following specific examples which are provided for purposes of illustration only and are not intended to limit the scope of the invention.

## EXAMPLE 1

*Detection of dorsal root receptor-like GPCR activity*

The polynucleotide of SEQ ID NO: 1 is inserted into the expression vector pCEV4 and the expression vector pCEV4 dorsal root receptor-like GPCR polypeptide obtained is transfected into human embryonic kidney 293 cells. The cells are scraped from a culture flask into 5 ml of Tris HCl, 5 mM EDTA, pH 7.5, and lysed by sonication. Cell lysates are centrifuged at 1000 rpm for 5 minutes at 4°C. The supernatant is centrifuged at 30,000 x g for 20 minutes at 4°C. The pellet is suspended in binding buffer containing 50 mM Tris HCl, 5 mM $MgSO_4$, 1 mM EDTA, 100 mM NaCl, pH 7.5, supplemented with 0.1 % BSA, 2 mg/ml aprotinin, 0.5 mg/ml leupeptin, and 10 mg/ml phosphoramidon. Optimal membrane suspension dilutions, defined as the protein concentration required to bind less than 10 % of an added radioligand are added to 96-well polypropylene microtiter plates containing ligand, non-labeled peptides, and binding buffer to a final volume of 250 ml.

In equilibrium saturation binding assays, membrane preparations are incubated in the presence of increasing concentrations (0.1 nM to 4 nM) of $^{125}$I ligand.

Binding reaction mixtures are incubated for one hour at 30°C. The reaction is stopped by filtration through GF/B filters treated with 0.5% polyethyleneimine, using a cell harvester. Radioactivity is measured by scintillation counting, and data are analyzed by a computerized non-linear regression program. Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. The dorsal root receptor-like GPCR activity of the polypeptide comprising the amino acid sequence of SEQ ID NO: 2 is demonstrated.

## EXAMPLE 2

*Radioligand binding assays*

Human embryonic kidney 293 cells transfected with a polynucleotide which expresses human GPCR are scraped from a culture flask into 5 ml of Tris HCl, 5 mM EDTA, pH 7.5, and lysed by sonication. Cell lysates are centrifuged at 1000 rpm for 5 minutes at 4°C. The supernatant is centrifuged at 30,000 x g for 20 minutes at 4°C. The pellet is suspended in binding buffer containing 50 mM Tris HCl, 5 mM $MgSO_4$, 1 mM EDTA, 100 mM NaCl, pH 7.5, supplemented with 0.1% BSA, 2 μg/ml aprotinin, 0.5 mg/ml leupeptin, and 10 μg/ml phosphoramidon. Optimal membrane suspension dilutions, defined as the protein concentration required to bind less than 10% of the added radioligand, are added to 96-well polypropylene microtiter plates containing $^{125}$I-labeled ligand or test compound, non-labeled peptides, and binding buffer to a final volume of 250 μl.

In equilibrium saturation binding assays, membrane preparations are incubated in the presence of increasing concentrations (0.1 nM to 4 nM) of $^{125}$I-labeled ligand or test compound (specific activity 2200 Ci/mmol). The binding affinities of different test compounds are determined in equilibrium competition binding assays, using 0.1 nM $^{125}$I-peptide in the presence of twelve different concentrations of each test compound.

Binding reaction mixtures are incubated for one hour at 30°C. The reaction is stopped by filtration through GF/B filters treated with 0.5% polyethyleneimine, using a cell harvester. Radioactivity is measured by scintillation counting, and data are analyzed by a computerized non-linear regression program.

Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. A test compound which increases the radioactivity of membrane protein by at least 15% relative to radioactivity of membrane protein which was not incubated with a test compound is identified as a compound which binds to a human GPCR polypeptide.

## EXAMPLE 3

*Effect of a test compound on human GPCR -mediated cyclic AMP formation*

Receptor-mediated inhibition of cAMP formation can be assayed in host cells which express human GPCR. Cells are plated in 96-well plates and incubated in Dulbecco's phosphate buffered saline (PBS) supplemented with 10 mM HEPES, 5 mM theophylline, 2 μg/ml aprotinin, 0.5 mg/ml leupeptin, and 10 μg/ml phosphoramidon for 20 minutes at 37°C in 5% CO2. A test compound is added and incubated for an additional 10 minutes at 37°C. The medium is aspirated, and the reaction is stopped by the addition of 100 mM HCl. The plates are stored at 4 °C for 15 minutes. cAMP content in the stopping solution is measured by radioimmunoassay.

Radioactivity is quantified using a gamma counter equipped with data reduction software. A test compound which decreases radioactivity of the contents of a well relative to radioactivity of the contents of a well in the absence of the test compound is identified as a potential inhibitor of cAMP formation. A test compound which increases radioactivity of the contents of a well relative to radioactivity of the contents of a well in the absence of the test compound is identified as a potential enhancer of cAMP formation.

## EXAMPLE 4

*Effect of a test compound on the mobilization of intracellular calcium*

Intracellular free calcium concentration can be measured by microspectrofluorometry using the fluorescent indicator dye Fura-2/AM (Bush et al., J. Neurochem. 57, 562-74, 1991). Stably transfected cells are seeded onto a 35 mm culture dish containing a glass coverslip insert. Cells are washed with HBS , incubated with a test compound, and loaded with 100 μl of Fura-2/AM (10 μM) for 20-40 minutes. After washing with HBS to remove the Fura-2/AM solution, cells are equilibrated in HBS for 10-20 minutes. Cells are then visualized under the 40X objective of a Leitz Fluovert FS microscope.

Fluorescence emission is determined at 510 nM, with excitation wavelengths alternating between 340 nM and 380 nM. Raw fluorescence data are converted to calcium concentrations using standard calcium concentration curves and software analysis techniques. A test compound which increases the fluorescence by at least 15% relative to fluorescence in the absence of a test compound is identified as a compound which mobilizes intracellular calcium.

**EXAMPLE 5**

*Effect of a test compound on phosphoinositide metabolism*

Cells which stably express human GPCR cDNA are plated in 96-well plates and grown to confluence. The day before the assay, the growth medium is changed to 100 μl of medium containing 1% serum and 0.5 μCi $^3$H-myinositol. The plates are incubated overnight in a $CO_2$ incubator (5% $CO_2$ at 37°C). Immediately before the assay, the medium is removed and replaced by 200 μl of PBS containing 10 mM LiCl, and the cells are equilibrated with the new medium for 20 minutes. During this interval, cells also are equilibrated with antagonist, added as a 10 μl aliquot of a 20-fold concentrated solution in PBS.

The $^3$H-inositol phosphate accumulation from inositol phospholipid metabolism is started by adding 10 μml of a solution containing a test compound. To the first well 10 μl are added to measure basal accumulation. Eleven different concentrations of test compound are assayed in the following 11 wells of each plate row. All assays are performed in duplicate by repeating the same additions in two consecutive plate rows.

The plates are incubated in a $CO_2$ incubator for one hour. The reaction is terminated by adding 15 μl of 50% v/v trichloroacetic acid (TCA), followed by a 40 minute incubation at 4°C. After neutralizing TCA with 40 μl of 1 M Tris, the content of the wells is transferred to a Multiscreen HV filter plate (Millipore) containing Dowex AG1-X8 (200-400 mesh, formate form). The filter plates are prepared by adding 200 μl of Dowex AG1-X8 suspension (50% v/v, water: resin) to each well. The filter plates are placed on a vacuum manifold to wash or elute the resin bed. Each well is washed 2 times with 200 μl of water, followed by 2 x 200 μl of 5 mM sodium tetraborate/60 mM ammonium formate.

The $^3$H-IPs are eluted into empty 96-well plates with 200 μl of 1.2 M ammonium formate/0.1 formic acid. The content of the wells is added to 3 ml of scintillation cocktail, and radioactivity is determined by liquid scintillation counting.

**EXAMPLE 6**

*Receptor Binding Methods*

Standard Binding Assays. Binding assays are carried out in a binding buffer containing 50 mM HEPES, pH 7.4, 0.5% BSA, and 5 mM $MgCl_2$. The standard assay for radioligand binding to membrane fragments comprising GPCR polypeptides is carried out as follows in 96 well microtiter plates (*e.g.*, Dynatech Immulon II Removawell plates). Radioligand is diluted in binding buffer+ PMSF/Baci to the desired cpm per 50 μl, then 50 μl aliquots are added to the wells. For non-specific binding samples, 5 μl of 40 μM cold ligand also is added per well. Binding is initiated by adding 150 μl per well of membrane diluted to the desired concentration (10-30 μg membrane protein/well) in binding buffer+ PMSF/Baci. Plates are then covered with Linbro mylar plate sealers (Flow Labs) and placed on a Dynatech Microshaker II. Binding is allowed to proceed at room temperature for 1-2 hours and is stopped by centrifuging the plate for 15 minutes at 2,000 x g. The supernatants are decanted, and the membrane pellets are washed once by addition of 200 μl of ice cold binding buffer, brief shaking, and recentrifugation. The individual wells are placed in 12 x 75 mm tubes and counted in an LKB Gammamaster counter (78% efficiency). Specific binding by this method is identical to that measured when free ligand is removed by rapid (3-5 seconds) filtration and washing on polyethyleneimine-coated glass fiber filters.

Three variations of the standard binding assay are also used:

1. Competitive radioligand binding assays with a concentration range of cold ligand vs. $^{125}$I-labeled ligand are carried out as described above with one modification. All dilutions of ligands being assayed are made in 40X PMSF/Baci to a concentration 40X the final concentration in the assay. Samples of peptide (5 μl each) are then added per microtiter well. Membranes and radioligand are diluted in binding buffer without protease inhibitors. Radioligand is added and mixed with cold ligand, and then binding is initiated by addition of membranes.

2. Chemical cross-linking of radioligand with receptor is done after a binding step identical to the standard assay. However, the wash step is done with binding buffer minus BSA to reduce the possibility of non-specific cross-linking of radioligand with BSA. The cross-linking step is carried out as described below.

3. Larger scale binding assays to obtain membrane pellets for studies on solubilization of receptor:ligand complex and for receptor purification are also carried out. These are identical to the standard assays except that (a) binding is carried out in polypropylene tubes in volumes from 1-250 ml, (b) concentration of membrane protein is always 0.5 mg/ml, and (c) for receptor purification, BSA concentration in the binding buffer is reduced to 0.25%, and the wash step is done with binding buffer without BSA, which reduces BSA contamination of the purified receptor.

**EXAMPLE 7**

*Chemical Cross-Linking of Radioligand to Receptor*

After a radioligand binding step as described above, membrane pellets are resuspended in 200 μl per microtiter plate well of ice-cold binding buffer without BSA. Then 5 μl per well of 4 mM N-5-azido-2-nitrobenzoyloxysuccinimide (ANB-NOS, Pierce) in DMSO is added and mixed. The samples are held on ice and UV-irradiated for 10 minutes with a Mineralight R-52G lamp (UVP Inc., San Gabriel, Calif.) at a distance of 5-10 cm. Then the samples are transferred to Eppendorf microfuge tubes, the membranes pelleted by centrifugation, supernatants removed, and membranes solubilized in Laemmli SDS sample buffer for polyacrylamide gel electrophoresis (PAGE). PAGE is carried out as described below. Radiolabeled proteins are visualized by autoradiography of the dried gels with Kodak XAR film and DuPont image intensifier screens.

**EXAMPLE 8**

*Membrane Solubilization*

Membrane solubilization is carried out in buffer containing 25 mM Tris , pH 8, 10% glycerol (w/v) and 0.2 mM $CaCl_2$ (solubilization buffer). The highly soluble detergents including Triton X-100, deoxycholate, deoxycholate:lysolecithin, CHAPS, and zwittergent are made up in solubilization buffer at 10% concentrations and stored as frozen aliquots. Lysolecithin is made up fresh because of insolubility upon freeze-thawing and digitonin is made fresh at lower concentrations due to its more limited solubility.

To solubilize membranes, washed pellets after the binding step are resuspended free of visible particles by pipetting and vortexing in solubilization buffer at 100,000 x g for 30 minutes. The supernatants are removed and held on ice and the pellets are discarded.

**EXAMPLE 9**

*Assay of Solubilized Receptors*

After binding of $^{125}$I ligands and solubilization of the membranes with detergent, the intact R:L complex can be assayed by four different methods. All are carried out on ice or in a cold room at 4-10°C.).

1. Column chromatography (Knuhtsen et al., Biochem. J. 254, 641-647, 1988). Sephadex G-50 columns (8 x 250 mm) are equilibrated with solubilization buffer containing detergent at the concentration used to solubilize membranes and 1 mg/ml bovine serum albumin. Samples of solubilized membranes (0.2-0.5 ml) are applied to the columns and eluted at a flow rate of about 0.7 ml/minute. Samples (0.18 ml) are collected. Radioactivity is determined in a gamma counter. Void volumes of the columns are determined by the elution volume of blue dextran. Radioactivity eluting in the void volume is considered bound to protein. Radioactivity eluting later, at the same volume as free $^{125}$I ligands, is considered non-bound.

2. Polyethyleneglycol precipitation (Cuatrecasas, Proc. Natl. Acad. Sci. USA 69, 318-322, 1972). For a 100 μl sample of solubilized membranes in a 12 x 75 mm polypropylene tube, 0.5 ml of 1% (w/v) bovine gamma globulin (Sigma) in 0.1 M sodiumphosphate buffer is added, followed by 0.5 ml of 25% (w/v) polyethyleneglycol (Sigma) and mixing. The mixture is held on ice for 15 minutes. Then 3 ml of 0.1 M sodium phosphate, pH 7.4, is added per sample. The samples are rapidly (1-3 seconds) filtered over Whatman GF/B glass fiber filters and washed with 4 ml of the phosphate buffer. PEG-precipitated receptor : $^{125}$ I-ligand complex is determined by gamma counting of the filters.

3. GFB/PEI filter binding (Bruns et al., Analytical Biochem. 132, 74-81, 1983). Whatman GF/B glass fiber filters are soaked in 0.3% polyethyleneimine (PEI, Sigma) for 3 hours. Samples of solubilized membranes (25-100 μl) are replaced in 12 x 75 mm polypropylene tubes. Then 4 ml of solubilization buffer without detergent is added per sample and the samples are immediately filtered through the GFB/PEI filters (1-3 seconds) and washed with 4 ml of solubilization buffer. CPM of receptor: $^{125}$ I-ligand complex adsorbed to filters are determined by gamma counting.

4. Charcoal/Dextran (Paul and Said, Peptides 7[Suppl. 1],147-149, 1986). Dextran T70 (0.5 g, Pharmacia) is dissolved in 1 liter of water, then 5 g of activated charcoal (Norit A, alkaline; Fisher Scientific) is added. The suspension is stirred for 10 minutes at room temperature and then stored at 4°C. until use. To measure R:L complex, 4 parts by volume of charcoal/dextran suspension are added to 1 part by volume of solubilized membrane. The samples are mixed and held on ice for 2 minutes and then centrifuged for 2 minutes at 11,000 x g in a Beckman microfuge. Free radioligand is adsorbed charcoal/dextran and is discarded with the pellet. Receptor : $^{125}$I-ligand complexes remain in the supernatant and are determined by gamma counting.

## EXAMPLE 10

*Receptor Purification*

Binding of biotinyl-receptor to $GH_4$ Cl membranes is carried out as described above. Incubations are for 1 hour at room temperature. In the standard purification protocol, the binding incubations contain 10 nM Bio-S29. $^{125}$I ligand is added as a tracer at levels of 5,000-100,000 cpm per mg of membrane protein. Control incubations contain 10 μM cold ligand to saturate the receptor with non-biotinylated ligand.

Solubilization of receptor:ligand complex also is carried out as described above, with 0.15% deoxycholate: lysolecithin in solubilization buffer containing 0.2 mM $MgCl_2$, to obtain 100,000 x g supernatants containing solubilized R:L complex.

Immobilized streptavidin (streptavidin cross-linked to 6% beaded agarose, Pierce Chemical Co.; "SA-agarose") is washed in solubilization buffer and added to the solubilized membranes as 1/30 of the final volume. This mixture is incubated with constant stirring by end-over-end rotation for 4-5 hours at 4-10°C. Then the mixture is applied to a column and the non-bound material is washed through. Binding of radioligand to SA-agarose is determined by comparing cpm in the 100,000 x g supernatant with that in the column effluent after adsorption to SA-agarose. Finally, the column is washed with 12-15 column volumes of solubilization buffer+0.15% deoxycholate:lysolecithin +1/500 (vol/vol) 100 x 4pase.

The streptavidin column is eluted with solubilization buffer+0.1 mM EDTA+0.1 mM EGTA+O.L mM GTP-gamma-S (Sigma)+0.15% (wt/vol) deoxycholate:lysolecithin +1/1000 (vol/vol) 100.times.4pase. First, one column volume of elution buffer is passed through the column and flow is stopped for 20-30 minutes. Then 3-4 more column volumes of elution buffer are passed through. All the eluates are pooled.

Eluates from the streptavidin column are incubated overnight (12-15 hours) with immobilized wheat germ agglutinin (WGA agarose, Vector Labs) to adsorb the receptor via interaction of covalently bound carbohydrate with the WGA lectin. The ratio (vol/vol) of WGA-agarose to streptavidin column eluate is generally 1:400. A range from 1:1000 to 1:200 also can be used. After the tinning step, the resin is pelleted by centrifugation, the supernatant is removed and saved, and the resin is washed 3 times (about 2 minutes each) in buffer containing 50 mM HEPES, pH 8, 5 mM $MgCl_2$, and 0.15% deoxycholate:lysolecithin. To elute the WGA-bound receptor, the resin is extracted three times by repeated mixing (vortex mixer on low speed) over a 15-30 minute period on ice, with 3 resin columns each time, of 10 mM N-N'-N"-triacetylchitotriose in the same HEPES buffer used to wash the resin. After each elution step, the resin is centrifuged down and the supernatant is carefully removed, free of WGA-agarose pellets. The three, pooled eluates contain the final, purified receptor. The material non-bound to WGA contain G protein subunits specifically eluted from the streptavidin column, as well as non-specific contaminants. All these fractions are stored frozen at -90°C.

## EXAMPLE 11

*Identification of test compounds that bind to GPCR polypeptides*

Purified GPCR polypeptides comprising a glutathione-S-transferase protein and absorbed onto glutathione-derivatized wells of 96-well microtiter plates are contacted with test compounds from a small molecule library at pH 7.0 in a physiological buffer solution. GPCR polypeptides comprise an amino acid sequence shown in SEQ ID NO: 2. The test compounds comprise a fluorescent tag. The samples are incubated for 5 minutes to one hour. Control samples are incubated in the absence of a test compound.

The buffer solution containing the test compounds is washed from the wells. Binding of a test compound to a GPCR polypeptide is detected by fluorescence measurements of the contents of the wells. A test compound which increases the fluorescence in a well by at least 15% relative to fluorescence of a well in which a test compound is not incubated is identified as a compound which binds to a GPCR polypeptide.

**EXAMPLE 12**

*Identification of a test compound which decreases GPCR gene expression*

A test compound is administered to a culture of human gastric cells and incubated at 37°C for 10 to 45 minutes. A culture of the same type of cells incubated for the same time without the test compound provides a negative control.

RNA is isolated from the two cultures as described in Chirgwin et al., Biochem. 18, 5294-99, 1979). Northern blots are prepared using 20 to 30 μg total RNA and hybridized with a $^{32}P$-labeled GPCR-specific probe at 65°C in Express-hyb (CLONTECH). The probe comprises at least 11 contiguous nucleotides selected from the complement of SEQ ID NO: 1. A test compound which decreases the GPCR-specific signal relative to the signal obtained in the absence of the test compound is identified as an inhibitor of GPCR gene expression.

**EXAMPLE 13**

***Cellular test systems***

**$G_i$-coupled receptor screening**

Cells are stably transfected with the relevant receptor and with an inducible CRE-luciferase construct. Cells are grown in 50% Dulbecco's modified Eagle medium /50% F12 (DMEM/F12) supplemented with 10% FBS, at 37°C in a humidified atmosphere with 10% $CO_2$ and are routinely split at a ratio of 1:10 every 2 or 3 days.

Test cultures are seeded into 384 - well plates at an appropriate density (e.g. 2000 cells / well in 35 μl cell culture medium) in DMEM/F12 with FBS, and are grown for 48 hours (range: ~ 24 - 60 hours, depending on cell line). Growth medium is then exchanged against serum free medium (SFM; e.g. Ultra-CHO), containing 0,1% BSA. Test compounds dissolved in DMSO are diluted in SFM and transferred to the test cultures (maximal final concentration 10 μmolar), followed by addition of forskolin (~ 1 μmolar, final conc.) in SFM + 0,1% BSA 10 minutes later. In case of antagonist screening both, an appropriate concentration of agonist, and forskolin are added. The plates are incubated at 37°C in 10% $CO_2$ for 3 hours. Then the supernatant is removed, cells are lysed with lysis reagent (25 mmolar phosphate-buffer, pH 7,8 , containing 2 mmolar DDT, 10% glycerol and 3% Triton X100). The luciferase reaction is started by addition of substrate-buffer (e.g. luciferase assay reagent, Promega) and luminescence is immediately determined (e.g. Berthold luminometer or Hamamatzu camera system).

**$G_s$ -coupled receptor screening**

Cells are stably transfected with the relevant receptor and with an inducible CRE-luciferase construct. Cells are grown in 50% Dulbecco's modified Eagle medium /50% F12 (DMEM/F12) supplemented with 10% FBS, at 37°C in a humidified atmosphere with 10% $CO_2$ and are routinely split at a ratio of 1:10 every 2 or 3 days. Test cultures are seeded into 384 - well plates at an appropriate density (e.g. 1000 or 2000 cells / well in 35 μl cell culture medium) in DMEM/F12 with FBS, and are grown for 48 hours (range: ~ 24 - 60 hours, depending on cell line). The assay is started by addition of test-compounds in serum free medium (SFM; e.g. Ultra-CHO) containing 0,1% BSA: Test compounds are dissolved in DMSO, diluted in SFM and transferred to the test cultures (maximal final concentration 10 μmolar, DMSO conc. < 0,6%). In case of antagonist screening an appropriate concentration of agonist is added 5-10 minutes later. The plates are incubated at 37°C in 10% $CO_2$ for 3 hours. Then the cells are lysed with 10 μl lysis reagent per well (25 mmolar phosphate-buffer, pH 7,8 , containing 2 mmolar DDT, 10% glycerol and 3% Triton X100) and the luciferase reaction is started by addition of 20 μl substrate-buffer per well (e.g. luciferase assay reagent, Promega). Measurement of luminescence is started immediately (e.g. Berthold luminometer or Hamamatzu camera system).

**$G_g$ -coupled receptor screening**

Cells are stably transfected with the relevant receptor. Cells expressing functional receptor protein are grown in 50% Dulbecco's modified Eagle medium / 50% F12 (DMEM/F12) supplemented with 10% FBS, at 37°C in a humidified atmosphere with 5% $CO_2$ and are routinely split at a cell line dependent ratio every 3 or 4 days. Test cultures are seeded into 384 - well plates at an appropriate density (e.g. 2000 cells / well in 35 μl cell culture medium) in DMEM/F12 with FBS, and are grown for 48 hours (range: ~ 24 - 60 hours, depending on cell line). Growth medium is then exchanged against physiological salt solution (e.g. Tyrode's solution). Test compounds dissolved in DMSO are diluted in Tyrode's solution containing 0.1% BSA and transferred to the test cultures (maximal final concentration 10 μmolar). After addition of the receptor specific agonist the resulting Gq-mediated intracellular calcium increase is measured using appropriate read-out systems (e.g. calcium-sensitive dyes).

## EXAMPLE 14

*In vivo testing of compounds/target validation*

### 1. Acute Mechanistic Assays

#### *1.1. Reduction in Mitogenic Plasma Hormone Levels*

This non-tumor assay measures the ability of a compound to reduce either the endogenous level of a circulating hormone or the level of hormone produced in response to a biologic stimulus. Rodents are administered test compound (p.o., i.p., i.v., i.m., or s.c.). At a predetermined time after administration of test compound, blood plasma is collected. Plasma is assayed for levels of the hormone of interest. If the normal circulating levels of the hormone are too low and/or variable to provide consistent results, the level of the hormone may be elevated by a pre-treatment with a biologic stimulus (i.e., LHRH may be injected i.m. into mice at a dosage of 30 ng/mouse to induce a burst of testosterone synthesis). The timing of plasma collection would be adjusted to coincide with the peak of the induced hormone response. Compound effects are compared to a vehicle-treated control group. An F-test is preformed to determine if the variance is equal or unequal followed by a Student's t-test. Significance is p value $\leq 0.05$ compared to the vehicle control group.

#### *1.2. Hollow Fiber Mechanism of Action Assay*

Hollow fibers are prepared with desired cell line(s) and implanted intraperitoneally and/or subcutaneously in rodents. Compounds are administered p.o., i.p., i.v., i.m., or s.c. Fibers are harvested in accordance with specific readout assay protocol, these may include assays for gene expression (bDNA, PCR, or Taqman), or a specific biochemical activity (i.e., cAMP levels. Results are analyzed by Student's t-test or Rank Sum test after the variance between groups is compared by an F-test, with significance at $p \leq 0.05$ as compared to the vehicle control group.

### 2. Subacute Functional *In Vivo* Assays

#### *2.1. Reduction in Mass of Hormone Dependent Tissues*

This is another non-tumor assay that measures the ability of a compound to reduce the mass of a hormone dependent tissue (i.e., seminal vesicles in males and uteri in females). Rodents are administered test compound (p.o., i.p., i.v., i.m., or s.c.) according to a predetermined schedule and for a predetermined duration (i.e., 1 week). At termination of the study, animals are weighed, the target organ is excised, any fluid is expressed, and the weight of the organ is recorded. Blood plasma may also be collected. Plasma may be assayed for levels of a hormone of interest or for levels of test agent. Organ weights may be directly compared or they may be normalized for the body weight of the animal. Compound effects are compared to a vehicle-treated control group. An F-test is preformed to determine if the variance is equal or unequal followed by a Student's t-tesrt. Significance is p value $\leq 0.05$ compared to the vehicle control group.

#### *2.2. Hollow Fiber Proliferation Assay*

Hollow fibers are prepared with desired cell line(s) and implanted intraperitoneally and/or subcutaneously in rodents. Compounds are administered p.o., i.p., i.v., i.m., or s.c. Fibers are harvested in accordance with specific readout assay protocol. Cell proliferation is determined by measuring a marker of cell number (i.e., MTT or LDH). The cell number and change in cell number from the starting inoculum are analyzed by Student's t-test or Rank Sum test after the variance between groups is compared by an F-test, with significance at $p \leq 0.05$ as compared to the vehicle control group.

#### *2.3. Anti-angiogenesis Models*

*2.3.1. Corneal Angiogenesis*

Hydron pellets with or without growth factors or cells are implanted into a micro-pocket surgically created in the rodent cornea. Compound administration may be systemic or local (compound mixed with growth factors in the hydron pellet). Corneas are harvested at 7 days post implantation immediately following intracardiac infusion of colloidal carbon and are fixed in 10% formalin. Readout is qualitative scoring and/or image analysis. Qualitative scores are compared by Rank Sum test. Image analysis data is evaluated by measuring the area of neovascularization (in pixels) and group averages are compared by Student's t-test (2 tail). Significance is $p \leq 0.05$ as compared to the growth factor or cells only group.

*2.3.2. Matrigel Angiogenesis*

Matrigel, containing cells or growth factors, is injected subcutaneously. Compounds are administered p.o., i.p., i.v., i.m., or s.c. Matrigel plugs are harvested at predetermined time point(s) and prepared for readout. Readout is an ELISA-based assay for hemoglobin concentration and/or histological examination (i.e. vessel count, special staining for endothelial surface markers: CD31, factor-8). Readouts are analyzed by Student's t-test, after the variance between groups is compared by an F-test, with significance determined at $p \leq 0.05$ as compared to the vehicle control group.

## 3. Primary Antitumor Efficacy

### *3.1. Early Therapy Models*

*3.1.1. Subcutaneous Tumor*

Tumor cells or fragments are implanted subcutaneously on Day 0. Vehicle and/or compounds are administered p.o., i.p., i.v., i.m., or s.c. according to a predetermined schedule starting at a time, usually on Day 1, prior to the ability to measure the tumor burden. Body weights and tumor measurements are recorded 2-3 times weekly. Mean net body and tumor weights are calculated for each data collection day. Antitumor efficacy may be initially determined by comparing the size of treated (T) and control (C) tumors on a given day by a Student's t-test, after the variance between groups is compared by an F-test, with significance determined at $p \leq 0.05$. The experiment may also be continued past the end of dosing in which case tumor measurements would continue to be recorded to monitor tumor growth delay. Tumor growth delays are expressed as the difference in the median time for the treated and control groups to attain a predetermined size divided by the median time for the control group to attain that size. Growth delays are compared by generating Kaplan-Meier curves from the times for individual tumors to attain the evaluation size. Significance is $p \leq 0.05$.

*3.1.2. IntraperitonealIntracranial Tumor Models*

Tumor cells are injected intraperitoneally or intracranially on Day 0. Compounds are administered p.o., i.p., i.v., i.m., or s.c. according to a predetermined schedule starting on Day 1. Observations of morbidity and/or mortality are recorded twice daily. Body weights are measured and recorded twice weekly. Morbidity/mortality data is expressed in terms of the median time of survival and the number of long-term survivors is indicated separately. Survival times are used to generate Kaplan-Meier curves. Significance is $p \leq 0.05$ by a log-rank test compared to the control group in the experiment.

### *3.2. Established Disease Model*

Tumor cells or fragments are implanted subcutaneously and grown to the desired size for treatment to begin. Once at the predetermined size range, mice are randomized into treatment groups. Compounds are administered p.o., i.p., i.v., i.m., or s.c. according to a predetermined schedule. Tumor and body weights are measured and recorded 2-3 times weekly. Mean tumor weights of all groups over days post inoculation are graphed for comparison. An F-test is preformed to determine if the variance is equal or unequal followed by a Student's t-test to compare tumor sizes in the treated and control groups at the end of treatment. Significance is $p \leq 0.05$ as compared to the control group. Tumor measurements may be recorded after dosing has stopped to monitor tumor growth delay. Tumor growth delays are expressed as the difference in the median time for the treated and control groups to attain a predetermined size divided by the median time for the control group to attain that size. Growth delays are compared by generating Kaplan-Meier curves from the times for individual tumors to attain the evaluation size. Significance is p value$\leq$ 0.05 compared to the vehicle control group.

### *3.3. Orthotopic Disease Models*

*3.3.1. Mammary Fat Pad Assay*

Tumor cells or fragments, of mammary adenocarcinoma origin, are implanted directly into a surgically exposed and reflected mammary fat pad in rodents. The fat pad is placed back in its original position and the surgical site is closed. Hormones may also be administered to the rodents to support the growth of the tumors. Compounds are administered p.o., i.p., i.v., i.m., or s.c. according to a predetermined schedule. Tumor and body weights are measured and recorded 2-3 times weekly. Mean tumor weights of all groups over days post inoculation are graphed for comparison. An F-test is preformed to determine if the variance is equal or unequal followed by a Student's t- test to compare tumor sizes in the treated and control groups at the end of treatment. Significance is p ≤ 0.05 as compared to the control group.

Tumor measurements may be recorded after dosing has stopped to monitor tumor growth delay. Tumor growth delays are expressed as the difference in the median time for the treated and control groups to attain a predetermined size divided by the median time for the control group to attain that size. Growth delays are compared by generating Kaplan-Meier curves from the times for individual tumors to attain the evaluation size. Significance is p value≤ 0.05 compared to the vehicle control group. In addition, this model provides an opportunity to increase the rate of spontaneous metastasis of this type of tumor. Metastasis can be assessed at termination of the study by counting the number of visible foci per target organ, or measuring the target organ weight. The means of these endpoints are compared by Student's t-test after conducting an F-test, with significance determined at p ≤ 0.05 compared to the control group in the experiment.

*3.3.2. Intraprostatic Assay*

Tumor cells or fragments, of prostatic adenocarcinoma origin, are implanted directly into a surgically exposed dorsal lobe of the prostate in rodents. The prostate is externalized through an abdominal incision so that the tumor can be implanted specifically in the dorsal lobe while verifying that the implant does not enter the seminal vesicles. The successfully inoculated prostate is replaced in the abdomen and the incisions through the abdomen and skin are closed. Hormones may also be administered to the rodents to support the growth of the tumors. Compounds are administered p.o., i.p., i.v., i.m., or s.c. according to a predetermined schedule. Body weights are measured and recorded 2-3 times weekly. At a predetermined time, the experiment is terminated and the animal is dissected. The size of the primary tumor is measured in three dimensions using either a caliper or an ocular micrometer attached to a dissecting scope. An F-test is preformed to determine if the variance is equal or unequal followed by a Student's t-test to compare tumor sizes in the treated and control groups at the end of treatment Significance is p ≤ 0.05 as compared to the control group. This model provides an opportunity to increase the rate of spontaneous metastasis of this type of tumor. Metastasis can be assessed at termination of the study by counting the number of visible foci per target organ (i.e., the lungs), or measuring the target organ weight (i.e., the regional lymph nodes). The means of these endpoints are compared by Student's t-test after conducting an F-test, with significance determined at p ≤ 0.05 compared to the control group in the experiment.

*3.3.3. Intrabronchial Assay*

Tumor cells of pulmonary origin may be implanted intrabronchially by making an incision through the skin and exposing the trachea. The trachea is pierced with the beveled end of a 25 gauge needle and the tumor cells are inoculated into the main bronchus using a flat-ended 27 gauge needle with a 90° bend. Compounds are administered p.o., i.p., i.v., i.m., or s.c. according to a predetermined schedule. Body weights are measured and recorded 2-3 times weekly. At a predetermined time, the experiment is terminated and the animal is dissected. The size of the primary tumor is measured in three dimensions using either a caliper or an ocular micrometer attached to a dissecting scope. An F-test is preformed to determine if the variance is equal or unequal followed by a Student's t-test to compare tumor sizes in the treated and control groups at the end of treatment. Significance is p ≤ 0.05 as compared to the control group. This model provides an opportunity to increase the rate of spontaneous metastasis of this type of tumor. Metastasis can be assessed at termination of the study by counting the number of visible foci per target organ (i.e., the contralateral lung), or measuring the target organ weight. The means of these endpoints are compared by Student's t-test after conducting an F-test, with significance determined at p ≤ 0.05 compared to the control group in the experiment.

*3.3.4. Intracecal Assay*

Tumor cells of gastrointestinal origin may be implanted intracecally by making an abdominal incision through the skin and externalizing the intestine. Tumor cells are inoculated into the cecal wall without penetrating the lumen of the intestine using a 27 or 30 gauge needle. Compounds are administered p.o., i.p., i.v., i.m., or s.c. according to a predetermined schedule. Body weights are measured and recorded 2-3 times weekly. At a predetermined time, the experiment is terminated and the animal is dissected. The size of the primary tumor is measured in three dimensions using either a caliper or an ocular micrometer attached to a dissecting scope. An F-test is preformed to determine if the variance is equal or unequal followed by a Student's t-test to compare tumor sizes in the treated and control groups at the end of treatment. Significance is p ≤ 0.05 as compared to the control group. This model provides an opportunity to increase the rate of spontaneous metastasis of this type of tumor. Metastasis can be assessed at termination of the study by counting the number of visible foci per target organ (i.e., the liver), or measuring the target organ weight. The means of these endpoints are compared by Student's t-test after conducting an F-test, with significance determined at p ≤ 0.05 compared to the control group in the experiment.

**4. Secondary (Metastatic) Antitumor Efficacy**

***4.1. Spontaneous Metastasis***

Tumor cells are inoculated s.c. and the tumors allowed to grow to a predetermined range for spontaneous metastasis studies to the lung or liver. These primary tumors are then excised. Compounds are administered p.o., i.p., i.v., i.m., or s.c. according to a predetermined schedule which may include the period leading up to the excision of the primary tumor to evaluate therapies directed at inhibiting the early stages of tumor metastasis. Observations of morbidity and/or mortality are recorded daily. Body weights are measured and recorded twice weekly. Potential endpoints include survival time, numbers of visible foci per target organ, or target organ weight. When survival time is used as the endpoint the other values are not determined. Survival data is used to generate Kaplan-Meier curves. Significance is $p \leq 0.05$ by a log-rank test compared to the control group in the experiment. The mean number of visible tumor foci, as determined under a dissecting microscope, and the mean target organ weights are compared by Student's t-test after conducting an F-test, with significance determined at $p \leq 0.05$ compared to the control group in the experiment for both of these endpoints.

***4.2. Forced Metastasis***

Tumor cells are injected into the tail vein, portal vein, or the left ventricle of the heart in experimental (forced) lung, liver, and bone metastasis studies, respectively. Compounds are administered p.o., i.p., i.v., i.m., or s.c. according to a predetermined schedule. Observations of morbidity and/or mortality are recorded daily. Body weights are measured and recorded twice weekly. Potential endpoints include survival time, numbers of visible foci per target organ, or target organ weight. When survival time is used as the endpoint the other values are not determined. Survival data is used to generate Kaplan-Meier curves. Significance is $p \leq 0.05$ by a log-rank test compared to the control group in the experiment. The mean number of visible tumor foci, as determined under a dissecting microscope, and the mean target organ weights are compared by Student's t-test after conducting an F-test, with significance at $p \leq 0.05$ compared to the vehicle control group in the experiment for both endpoints.

**EXAMPLE 15**

*Proliferation inhibition assay: Antisense oligonucleotides suppress the growth of cancer cell lines*

The cell line used for testing is the human colon cancer cell line HCT116. Cells are cultured in RPMI-1640 with 10-15% fetal calf serum at a concentration of 10,000 cells per milliliter in a volume of 0.5 ml and kept at 37°C in a 95% air/5%$CO_2$ atmosphere.

Phosphorothioate oligoribonucleotides are synthesized on an Applied Biosystems Model 380B DNA synthesizer using phosphoroamidite chemistry. A sequence of 24 bases complementary to the nucleotides at position 1 to 24 of SEQ ID NO: 1 is used as the test oligonucleotide. As a control, another (random) sequence is used: 5'-TCA ACT GAC TAG ATG TAC ATG GAC-3'. Following assembly and deprotection, oligonucleotides are ethanol-precipitated twice, dried, and suspended in phosphate buffered saline at the desired concentration. Purity of the oligonucleotides is tested by capillary gel electrophoresis and ion exchange HPLC. The purified oligonucleotides are added to the culture medium at a concentration of 10 μM once per day for seven days.

The addition of the test oligonucleotide for seven days results in significantly reduced expression of human GPCR as determined by Western blotting. This effect is not observed with the control oligonucleotide. After 3 to 7 days, the number of cells in the cultures is counted using an automatic cell counter. The number of cells in cultures treated with the test oligonucleotide (expressed as 100%) is compared with the number of cells in cultures treated with the control oligonucleotide. The number of cells in cultures treated with the test oligonucleotide is not more than 30% of control, indicating that the inhibition of human GPCR has an anti-proliferative effect on cancer cells.

**EXAMPLE 16**

*Diabetes: In vivo testing of compounds/target validation*

1. Glucose Production

Over-production of glucose by the liver, due to an enhanced rate of gluconeogenesis, is the major cause of fasting hyperglycemia in diabetes. Overnight fasted normal rats or mice have elevated rates of gluconeogenesis as do streptozotocin-induced diabetic rats or mice fed ad libitum. Rats are made diabetic with a single intravenous injection of 40 mg/kg of streptozotocin while C57BL/KsJ mice are given 40-60 mg/kg i.p. for 5 consecutive days. Blood glucose is measured from tail-tip blood and then compounds are administered via different routes (p.o., i.p., i.v., s.c.). Blood is collected at various times thereafter and glucose measured. Alternatively, compounds are administered for several days, then the animals are fasted overnight, blood is collected and plasma glucose measured. Compounds that inhibit glucose production will decrease plasma glucose levels compared to the vehicle-treated control group.

2. Insulin Sensitivity

Both ob/ob and db/db mice as well as diabetic Zucker rats are hyperglycemic, hyperinsulinemic and insulin resistant. The animals are pre-bled, their glucose levels measured, and then they are grouped so that the mean glucose level is the same for each group. Compounds are administered daily either q.d. or b.i.d. by different routes (p.o., i.p., s.c.) for 7-28 days. Blood is collected at various times and plasma glucose and insulin levels determined. Compounds that improve insulin sensitivity in these models will decrease both plasma glucose and insulin levels when compared to the vehicle-treated control group.

3. Insulin Secretion

Compounds that enhance insulin secretion from the pancreas will increase plasma insulin levels and improve the disappearance of plasma glucose following the administration of a glucose load. When measuring insulin levels, compounds are administered by different routes (p.o., i.p., s.c. or i.v.) to overnight fasted normal rats or mice. At the appropriate time an intravenous glucose load (0.4g/kg) is given, blood is collected one minute later. Plasma insulin levels are determined. Compounds that enhance insulin secretion will increase plasma insulin levels compared to animals given only glucose. When measuring glucose disappearance, animals are bled at the appropriate time after compound administration, then given either an oral or intraperitoneal glucose load (1g/kg), bled again after 15, 30, 60 and 90 minutes and plasma glucose levels determined. Compounds that increase insulin levels will decrease glucose levels and the area-under-the glucose curve when compared to the vehicle-treated group given only glucose.

Compounds that enhance insulin secretion from the pancreas will increase plasma insulin levels and improve the disappearance of plasma glucose following the administration of a glucose load. When measuring insulin levels, test compounds which regulate GPCR are administered by different routes (p.o., i.p., s.c., or i.v.) to overnight fasted normal rats or mice. At the appropriate time an intravenous glucose load (0.4 g/kg) is given, blood is collected one minute later. Plasma insulin levels are determined. Test compounds that enhance insulin secretion will increase plasma insulin levels compared to animals given only glucose. When measuring glucose disappearance, animals are bled at the appropriate time after compound administration, then given either an oral or intraperitoneal glucose load (1 g/kg), bled again after 15, 30, 60, and 90 minutes and plasma glucose levels determined. Test compounds that increase insulin levels will decrease glucose levels and the area-under-the glucose curve when compared to the vehicle-treated group given only glucose.

4. Glucose Production

Over-production of glucose by the liver, due to an enhanced rate of gluconeogenesis, is the major cause of fasting hyperglycemia in diabetes. Overnight fasted normal rats or mice have elevated rates of gluconeogenesis as do streptozotocin-induced diabetic rats or mice fed ad libitum. Rats are made diabetic with a single intravenous injection of 40 mg/kg of streptozotocin while C57BL/KsJ mice are given 40-60 mg/kg i.p. for 5 consecutive days. Blood glucose is measured from tail-tip blood and then compounds are administered via different routes (p.o., i.p., i.v., s.c.). Blood is collected at various times thereafter and glucose measured. Alternatively, compounds are administered for several days, then the animals are fasted overnight, blood is collected and plasma glucose measured. Compounds that inhibit glucose production will decrease plasma glucose levels compared to the vehicle-treated control group.

5. Insulin Sensitivity

Both ob/ob and db/db mice as well as diabetic Zucker rats are hyperglycemic, hyperinsulinemic and insulin resistant. The animals are pre-bled, their glucose levels measured, and then they are grouped so that the mean glucose level is the same for each group. Compounds are administered daily either q.d. or b.i.d. by different routes (p.o., i.p., s.c.) for 7-28 days. Blood is collected at various times and plasma glucose and insulin levels determined. Compounds that improve insulin sensitivity in these models will decrease both plasma glucose and insulin levels when compared to the vehicle-treated control group.

6. Insulin Secretion

Compounds that enhance insulin secretion from the pancreas will increase plasma insulin levels and improve the disappearance of plasma glucose following the administration of a glucose load. When measuring insulin levels, compounds are administered by different routes (p.o., i.p., s.c. or i.v.) to overnight fasted normal rats or mice. At the appropriate time an intravenous glucose load (0.4 g/kg) is given, blood is collected one minute later. Plasma insulin levels are determined. Compounds that enhance insulin secretion will increase plasma insulin levels compared to animals given only glucose. When measuring glucose disappearance, animals are bled at the appropriate time after compound administration, then given either an oral or intraperitoneal glucose load (1 g/kg), bled again after 15, 30, 60 and 90 minutes and plasma glucose levels determined. Compounds that increase insulin levels will decrease glucose levels and the area-under-the glucose curve when compared to the vehicle-treated group given only glucose.

## EXAMPLE 17

***In vivo testing of compounds/taget validation***

**1. Pain**

***Acute Pain***

Acute pain is measured on a hot plate mainly in rats. Two variants of hot plate testing are used: In the classical variant animals are put on a hot surface (52 to 56°C) and the latency time is measured until the animals show nocifensive behavior, such as stepping or foot licking. The other variant is an increasing temperature hot plate where the experimental animals are put on a surface of neutral temperature. Subsequently this surface is slowly but constantly heated until the animals begin to lick a hind paw. The temperature which is reached when hind paw licking begins is a measure for pain threshold.

Compounds are tested against a vehicle treated control group. Substance application is performed at different time points via different application routes (i.v., i.p., p.o., i.t., i.c.v., s.c., intradermal, transdermal) prior to pain testing.

***Persistent Pain***

Persistent pain is measured with the formalin or capsaicin test, mainly in rats. A solution of 1 to 5% formalin or 10 to 100 μg capsaicin is injected into one hind paw of the experimental animal. After formalin or capsaicin application the animals show nocifensive reactions like flinching, licking and biting of the affected paw. The number of nocifensive reactions within a time frame of up to 90 minutes is a measure for intensity of pain.

Compounds are tested against a vehicle treated control group. Substance application is performed at different time points via different application routes (i.v., i.p., p.o., i.t., i.c.v., s.c., intradermal, transdermal) prior to formalin or capsaicin administration.

***Neuropathic Pain***

Neuropathic pain is induced by different variants of unilateral sciatic nerve injury mainly in rats. The operation is performed under anesthesia. The first variant of sciatic nerve injury is produced by placing loosely constrictive ligatures around the common sciatic nerve. The second variant is the tight ligation of about the half of the diameter of the common sciatic nerve. In the next variant, a group of models is used in which tight ligations or transections are made of either the L5 and L6 spinal nerves, or the L% spinal nerve only. The fourth variant involves an axotomy of two of the three terminal branches of the sciatic nerve (tibial and common peroneal nerves) leaving the remaining sural nerve intact whereas the last variant comprises the axotomy of only the tibial branch leaving the sural and common nerves uninjured. Control animals are treated with a sham operation.

Postoperatively, the nerve injured animals develop a chronic mechanical allodynia, cold allodynioa, as well as a thermal hyperalgesia. Mechanical allodynia is measured by means of a pressure transducer (electronic von Frey Anesthesiometer, IITC Inc.-Life Science Instruments, Woodland Hills, SA, USA; Electronic von Frey System, Somedic Sales AB, Hörby, Sweden). Thermal hyperalgesia is measured by means of a radiant heat source (Plantar Test, Ugo Basile, Comerio, Italy), or by means of a cold plate of 5 to 10°C where the nocifensive reactions of the affected hind paw are counted as a measure of pain intensity. A further test for cold induced pain is the counting of nocifensive reactions, or duration of nocifensive responses after plantar administration of acetone to the affected hind limb. Chronic pain in general is assessed by registering the circadanian rhythms in activity (Surjo and Arndt, Universität zu Köln, Cologne, Germany), and by scoring differences in gait (foot print patterns; FOOTPRINTS program, Klapdor et al., 1997. A low cost method to analyze footprint patterns. J. Neurosci. Methods 75, 49-54).

Compounds are tested against sham operated and vehicle treated control groups. Substance application is performed at different time points via different application routes (i.v., i.p., p.o., i.t., i.c.v., s.c., intradermal, transdermal) prior to pain testing.

***Inflammatory Pain***

Inflammatory pain is induced mainly in rats by injection of 0.75 mg carrageenan or complete Freund's adjuvant into one hind paw. The animals develop an edema with mechanical allodynia as well as thermal hyperalgesia. Mechanical allodynia is measured by means of a pressure transducer (electronic von Frey Anesthesiometer, ITTC Inc.-Life Science Instruments, Woodland Hills, SA, USA). Thermal hyperalgesia is measured by means of a radiant heat source (Plantar Test, Ugo Basile, Comerio, Italy, Paw thermal stimulator, G. Ozaki, University of California, USA). For edema measurement two methods are being used. In the first method, the animals are sacrificed and the affected hindpaws sectioned and weighed. The second method comprises differences in paw volume by measuring water displacement in a plethysmometer (Ugo Basile, Comerio, Italy).

Compounds are tested against uninflamed as well as vehicle treated control groups. Substance application is performed at different time points via different application routes (i.v., i.p., p.o., i.t., i.c.v., s.c., intradermal, transdermal) prior to pain testing.

***Diabetic Neuropathic Pain***

Rats treated with a single intraperitoneal injection of 50 to 80 mg/kg streptozotocin develop a profound hyperglycemia and mechanical allodynia within I to 3 weeks. Mechanical allodynia is measured by means of a pressure transducer (electronic von Frey Anesthesiometer, IITC Inc.-Life Science Instruments, Woodland Hills, SA, USA).

Compounds are tested against diabetic and non-diabetic vehicle treated control groups. Substance application is performed at different time points via different application routes (i.v., i.p., p.o., i.t., i.c.v., s.c., intradermal, transdermal) prior to pain testing.

**2. Parkinson's disease**

***6-Hydroxydopamine (6-OH-DA) Lesion***

Degeneration of the dopaminergic nigrostriatal and striatopallidal pathways is the central pathological event in Parkinson's disease. This disorder has been mimicked experimentally in rats using single/sequential unilateral stereotaxic injections of 6-OH-DA into the medium forebrain bundle (MFB).

Male Wistar rats (Harlan Winkelmann, Germany), weighing 200±250 g at the beginning of the experiment, are used. The rats are maintained in a temperature- and humidity-controlled environment under a 12 h light/dark cycle with free access to food and water when not in experimental sessions. The following in vivo protocols are approved by the governmental authorities. All efforts are made to minimize animal suffering, to reduce the number of animals used, and to utilize alternatives to in vivo techniques.

Animals are administered pargyline on the day of surgery (Sigma, St. Louis, MO, USA; 50 mg/kg i.p.) in order to inhibit metabolism of 6-OHDA by monoamine oxidase and desmethylimipramine HCl (Sigma; 25 mg/kg i.p.) in order to prevent uptake of 6-OHDA by noradrenergic terminals. Thirty minutes later the rats are anesthetized with sodium pentobarbital (50 mg/kg) and placed in a stereotaxic frame. In order to lesion the DA nigrostriatal pathway 4 μl of 0.01% ascorbic acid-saline containing 8 μg of 6-OHDA HBr (Sigma) are injected into the left medial fore-brain bundle at a rate of 1 μl/min (2.4 mm anterior, 1.49 mm lateral, -2.7 mm ventral to Bregma and the skull surface). The needle is left in place an additional 5 min to allow diffusion to occur.

<u>Stepping Test</u>

Forelimb akinesia is assessed three weeks following lesion placement using a modified stepping test protocol. In brief, the animals are held by the experimenter with one hand fixing the hindlimbs and slightly raising the hind part above the surface. One paw is touching the table, and is then moved slowly sideways (5 s for 1 m), first in the forehand and then in the backhand direction. The number of adjusting steps is counted for both paws in the backhand and forehand direction of movement. The sequence of testing is right paw forehand and backhand adjusting stepping, followed by left paw forehand and backhand directions. The test is repeated three times on three consecutive days, after an initial training period of three days prior to the first testing. Forehand adjusted stepping reveals no consistent differences between lesioned and healthy control animals. Analysis is therefore restricted to backhand adjusted stepping.

Balance Test

Balance adjustments following postural challenge are also measured during the stepping test sessions. The rats are held in the same position as described in the stepping test and, instead of being moved sideways, tilted by the experimenter towards the side of the paw touching the table. This maneuver results in loss of balance and the ability of the rats to regain balance by forelimb movements is scored on a scale ranging from 0 to 3. Score 0 is given for a normal forelimb placement. When the forelimb movement is delayed but recovery of postural balance detected, score 1 is given. Score 2 represents a clear, yet insufficient, forelimb reaction, as evidenced by muscle contraction, but lack of success in recovering balance, and score 3 is given for no reaction of movement. The test is repeated three times a day on each side for three consecutive days after an initial training period of three days prior to the first testing.

Staircase Test (Paw Reaching)

A modified version of the staircase test is used for evaluation of paw reaching behavior three weeks following primary and secondary lesion placement. Plexiglass test boxes with a central platform and a removable staircase on each side are used. The apparatus is designed such that only the paw on the same side at each staircase can be used, thus providing a measure of independent forelimb use. For each test the animals are left in the test boxes for 15 min. The double staircase is filled with 7 x 3 chow pellets (Precision food pellets, formula: P, purified rodent diet, size 45 mg; Sandown Scientific) on each side. After each test the number of pellets eaten (successfully retrieved pellets) and the number of pellets taken (touched but dropped) for each paw and the success rate (pellets eaten/pellets taken) are counted separately. After three days of food deprivation (12 g per animal per day) the animals are tested for 11 days. Full analysis is conducted only for the last five days.

***MPTP treatment***

The neurotoxin 1-methyl-4-phenyl-1,2,3,6-tetrahydro-pytidine (MPTP) causes degeneration of mesencephalic dopaminergic (DAergic) neurons in rodents, non-human primates, and humans and, in so doing, reproduces many of the symptoms of Parkinson's disease. MPTP leads to a marked decrease in the levels of dopamine and its metabolites, and in the number of dopaminergic terminals in the striatum as well as severe loss of the tyrosine hydroxylase (TH)-immunoreactive cell bodies in the substantia nigra, pars compacta.

In order to obtain severe and long-lasting lesions, and to reduce mortality, animals receive single injections of MPTP, and are then tested for severity of lesion 7-10 days later. Successive MPTP injections are administered on days 1, 2 and 3. Animals receive application of 4 mg/kg MPTP hydrochloride (Sigma) in saline once daily. All injections are intraperitoneal (i.p.) and the MPTP stock solution is frozen between injections. Animals are decapitated on day 11.

Immunohistology

At the completion of behavioral experiments, all animals are anaesthetized with 3 ml thiopental (1 g/40 ml i.p., Tyrol Pharma). The mice are perfused transcardially with 0.01 M PBS (pH 7.4) for 2 min, followed by 4% paraformaldehyde (Merck) in PBS for 15 min. The brains are removed and placed in 4% paraformaldehyde for 24 h at 4°C. For dehydration they are then transferred to a 20% sucrose (Merck) solution in 0.1 M PBS at 4°C until they sink. The brains are frozen in methylbutan at -20°C for 2 min and stored at -70°C. Using a sledge microtome (mod. 3800-Frigocut, Leica), 25 μm sections are taken from the genu of the corpus callosum (AP 1.7 mm) to the hippocampus (AP 21.8 mm) and from AP 24.16 to AP 26.72. Forty-six sections are cut and stored in assorters in 0.25 M Tris buffer (pH 7.4) for immunohistochemistry.

A series of sections is processed for free-floating tyrosine hydroxylase (TH) immunohistochemistry. Following three rinses in 0.1 M PBS, endogenous peroxidase activity is quenched for 10 min in 0.3% $H_2O_2$ ±PBS. After rinsing in PBS, sections are preincubated in 10% normal bovine serum (Sigma) for 5 min as blocking agent and transferred to either primary anti-rat TH rabbit antiserum (dilution 1:2000).

Following overnight incubation at room temperature, sections for TH immunoreactivity are rinsed in PBS (2 x10 min) and incubated in biotinylated anti-rabbit immunoglobulin G raised in goat (dilution 1:200) (Vector) for 90 min, rinsed repeatedly and transferred to Vectastain ABC (Vector) solution for 1 h. 3,.3' -Diaminobenzidine tetrahydrochloride (DAB; Sigma) in 0.1 M PBS, supplemented with 0.005% $H_2O_2$, serves as chromogen in the subsequent visualization reaction. Sections are mounted on to gelatin-coated slides, left to dry overnight, counter-stained with hematoxylin dehydrated in ascending alcohol concentrations and cleared in butylacetate. Coverslips are mounted on entellan.

Rotarod Test

We use a modification of the procedure described by Rozas and Labandeira-Garcia (1997), with a CR-1 Rotamex system (Columbus Instruments, Columbus, OH) comprising an IBM-compatible personal computer, a CIO-24 data acquisition card, a control unit, and a four-lane rotarod unit. The rotarod unit consists of a rotating spindle (diameter 7.3 cm) and individual compartments for each mouse. The system software allows preprogramming of session protocols with varying rotational speeds (0-80 rpm). Infrared beams are used to detect when a mouse has fallen onto the base grid beneath the rotarod. The system logs the fall as the end of the experiment for that mouse, and the total time on the rotarod, as well as the time of the fall and all the set-up parameters, are recorded. The system also allows a weak current to be passed through the base grid, to aid training.

**3. Dementia**

***The object recognition task***

The object recognition task has been designed to assess the effects of experimental manipulations on the cognitive performance of rodents. A rat is placed in an open field, in which two identical objects are present. The rats inspects both objects during the first trial of the object recognition task. In a second trial, after a retention interval of for example 24 hours, one of the two objects used in the first trial, the 'familiar' object, and a novel object are placed in the open field. The inspection time at each of the objects is registered. The basic measures in the OR task is the time spent by a rat exploring the two object the second trial. Good retention is reflected by higher exploration times towards the novel than the 'familiar' object

Administration of the putative cognition enhancer prior to the first trial predominantly allows assessment of the effects on acquisition, and eventually on consolidation processes. Administration of the testing compound after the first trial allows to assess the effects on consolidation processes, whereas administration before the second trial allows to measure effects on retrieval processes.

***The passive avoidance task***

The passive avoidance task assesses memory performance in rats and mice. The inhibitory avoidance apparatus consists of a two-compartment box with a light compartment and a dark compartment The two compartments are separated by a guillotine door that can be operated by the experimenter. A threshold of 2 cm separates the two compartments when the guillotine door is raised. When the door is open, the illumination in the dark compartment is about 2 lux. The light intensity is about 500 lux at the center of the floor of the light compartment.

Two habituation sessions, one shock session, and a retention session are given, separated by inter-session intervals of 24 hours. In the habituation sessions and the retention session the rat is allowed to explore the apparatus for 300 sec. The rat is placed in the light compartment, facing the wall opposite to the guillotine door. After an accommodation period of 15 sec. the guillotine door is opened so that all parts of the apparatus can be visited freely. Rats normally avoid brightly lit areas and will enter the dark compartment within a few seconds.

In the shock session the guillotine door between the compartments is lowered as soon as the rat has entered the dark compartment with its four paws, and a scrambled 1 mA footshock is administered for 2 sec. The rat is removed from the apparatus and put back into its home cage. The procedure during the retention session is identical to that of the habituation sessions.

The step-through latency, that is the first latency of entering the dark compartment (in sec.) during the retention session is an index of the memory performance of the animal; the longer the latency to enter the dark compartment, the better the retention is. A testing compound in given half an hour before the shock session, together with 1 $mg*kg^{-1}$ scopolamine. Scopolamine impairs the memory performance during the retention session 24 hours later. If the test compound increases the enter latency compared with the scopolamine-treated controls, is likely to possess cognition enhancing potential.

***The Morris water escape task***

The Morris water escape task measures spatial orientation learning in rodents. It is a test system that has extensively been used to investigate the effects of putative therapeutic on the cognitive functions of rats and mice. The performance of an animal is assessed in a circular water tank with an escape platform that is submerged about 1 cm below the surface of the water. The escape platform is not visible for an animal swimming in the water tank. Abundant extra-maze cues are provided by the furniture in the room, including desks, computer equipment, a second water tank, the presence of the experimenter, and by a radio on a shelf that is playing softly.

The animals receive four trials during five daily acquisition sessions. A trial is started by placing an animal into the pool, facing the wall of the tank. Each of four starting positions in the quadrants north, east, south, and west is used once in a series of four trials; their order is randomized. The escape platform is always in the same position. A trial is terminated as soon as the animal had climbs onto the escape platform or when 90 seconds have elapsed, whichever event occurs first. The animal is allowed to stay on the platform for 30 seconds. Then it is taken from the platform and the next trial is started. If an animal did not find the platform within 90 seconds it is put on the platform by the experimenter and is allowed to stay there for 30 seconds. After the fourth trial of the fifth daily session, an additional trial is given as a probe trial: the platform is removed, and the time the animal spends in the four quadrants is measured for 30 or 60 seconds. In the probe trial, all animals start from the same start position, opposite to the quadrant where the escape platform had been positioned during acquisition.

Four different measures are taken to evaluate the performance of an animal during acquisition training: escape latency, traveled distance, distance to platform, and swimming speed. The following measures are evaluated for the probe trial: time (s) in quadrants and traveled distance (cm) in the four quadrants. The probe trial provides additional information about how well an animal learned the position of the escape platform. If an animal spends more time and swims a longer distance in the quadrant where the platform had been positioned during the acquisition sessions than in any other quadrant, one concludes that the platform position has been learned well.

In order to assess the effects of putative cognition enhancing compounds, rats or mice with specific brain lesions which impair cognitive functions, or animals treated with compounds such as scopolamine or MK-801, which interfere with normal learning, or aged animals which suffer from cognitive deficits, are used.

***The T-maze spontaneous alternation task***

The T-maze spontaneous alternation task (TeMCAT) assesses the spatial memory performance in mice. The start arm and the two goal arms of the T-maze are provided with guillotine doors which can be operated manually by the experimenter. A mouse is put into the start arm at the beginning of training. The guillotine door is closed. In the first trial, the 'forced trial', either the left or right goal arm is blocked by lowering the guillotine door. After the mouse has been released from the start arm, it will negotiate the maze, eventually enter the open goal arm, and return to the start position, where it will be confined for 5 seconds, by lowering the guillotine door. Then, the animal can choose freely between the left and right goal arm (all guillotine-doors opened) during 14 'free choice' trials. As soon a the mouse has entered one goal arm, the other one is closed. The mouse eventually returns to the start arm and is free to visit whichever go alarm it wants after having been confined to the start arm for 5 seconds. After completion of 14 free choice trials in one session, the animal is removed from the maze. During training, the animal is never handled.

The percent alternations out of 14 trials is calculated. This percentage and the total time needed to complete the first forced trial and the subsequent 14 free choice trials (in s) is analyzed. Cognitive deficits are usually induced by an injection of scopolamine, 30 min before the start of the training session. Scopolamine reduced the per-cent alternations to chance level, or below. A cognition enhancer, which is always administered before the training session, will at least partially, antagonize the scopolamine-induced reduction in the spontaneous alternation rate.

**EXAMPLE 18**

*Expression of recombinant human GPCR*

The *Pichia pastoris* expression vector pPICZB (Invitrogen, San Diego, CA) is used to produce large quantities of recombinant human GPCR polypeptides in yeast. The GPCR-encoding DNA sequence is derived from SEQ ID NO: 1. Before insertion into vector pPICZB, the DNA sequence is modified by well known methods in such a way that it contains at its 5'-end an initiation codon and at its 3'-end an enterokinase cleavage site, a His6 reporter tag and a termination codon. Moreover, at both termini recognition sequences for restriction endonucleases are added and after digestion of the multiple cloning site of pPICZ B with the corresponding restriction enzymes the modified DNA sequence is ligated into pPICZB. This expression vector is designed for inducible expression in *Pichia pastoris,* driven by a yeast promoter. The resulting pPICZ/md-His6 vector is used to transform the yeast.

The yeast is cultivated under usual conditions in 5 liter shake flasks and the recombinantly produced protein isolated from the culture by affinity chromatography (Ni-NTA-Resin) in the presence of 8 M urea. The bound polypeptide is eluted with buffer, pH 3.5, and neutralized. Separation of the polypeptide from the His6 reporter tag is accomplished by site-specific proteolysis using enterokinase (Invitrogen, San Diego, CA) according to manufacturer's instructions. Purified human GPCR polypeptide is obtained.

## EXAMPLE 19

*Tissue-specific expression of GPCR*

The qualitative expression pattern of GPCR in various tissues is determined by Reverse Transcription-Polymerase Chain Reaction (RT-PCR).

To demonstrate that GPCR is involved in the disease process of COPD, the initial expression panel consists of RNA samples from respiratory tissues and inflammatory cells relevant to COPD: lung (adult and fetal), trachea, freshly isolated alveolar type II cells, cultured human bronchial epithelial cells, cultured small airway epithelial cells, cultured bronchial sooth muscle cells, cultured H441 cells (Clara-like), freshly isolated neutrophils and monocytes, and cultured monocytes (macrophage-like). Body map profiling also is carried out, using total RNA panels purchased from Clontech. The tissues are adrenal gland, bone marrow, brain, colon, heart, kidney, liver, lung, mammary gland, pancreas, prostate, salivary gland, skeletal muscle, small intestine, spleen, stomach, testis, thymus, trachea, thyroid, and uterus.

To demonstrate that GPCR is involved in cancer, expression is determined in the following tissues: adrenal gland, bone marrow, brain, cerebellum, colon, fetal brain, fetal liver, heart, kidney, liver, lung, mammary gland, pancreas, placenta, prostate, salivary gland, skeletal muscle, small intestine, spinal cord, spleen, stomach, testis, thymus, thyroid, trachea, uterus, and peripheral blood lymphocytes. Expression in the following cancer cell lines also is determined: DU-145 (prostate), NCI-H125 (lung), HT-29 (colon), COLO-205 (colon), A-549 (lung), NCI-H460 (lung), HT-116 (colon), DLD-1 (colon), MDA-MD-231 (breast), LS174T (colon), ZF-75 (breast), MDA-MN-435 (breast), HT-1080, MCF-7 (breast), and U87. Matched pairs of malignant and normal tissue from the same patient also are tested.

To demonstrate that GPCR is involved in the disease process of obesity, expression is determined in the following tissues: subcutaneous adipose tissue, mesenteric adipose tissue, adrenal gland, bone marrow, brain (cerebellum, spinal cord, cerebral cortex, caudate, medulla, substantia nigra, and putamen), colon, fetal brain, heart, kidney, liver, lung, mammary gland, pancreas, placenta, prostate, salivary gland, skeletal muscle small intestine, spleen, stomach, testes, thymus, thyroid trachea, and uterus. Neuroblastoma cell lines SK-Nr-Be (2), Hr, Sk-N-As, HTB-10, IMR-32, SNSY-5Y, T3, SK-N-D2, D283, DAOY, CHP-2, U87MG, BE(2)C, T986, KANTS, MO59K, CHP234, C6 (rat), SK-N-F1, SK-PU-DW, PFSK-1, BE(2)M17, and MCIXC also are tested for GPCR expression. As a final step, the expression of GPCR in cells derived from normal individuals with the expression of cells derived from obese individuals is compared.

To demonstrate that GPCR is involved in the disease process of diabetes, the following whole body panel is screened to show predominant or relatively high expression: subcutaneous and mesenteric adipose tissue, adrenal gland, bone marrow, brain, colon, fetal brain, heart, hypothalamus, kidney, liver, lung, mammary gland, pancreas, placenta, prostate, salivary gland, skeletal muscle, small intestine, spleen, stomach, testis, thymus, thyroid, trachea, and uterus. Human islet cells and an islet cell library also are tested. As a final step, the expression of GPCR in cells derived from normal individuals with the expression of cells derived from diabetic individuals is compared.

To demonstrate that GPCR is involved in CNS disorders, the following tissues are screened: fetal and adult brain, muscle, heart, lung, kidney, liver, thymus, testis, colon, placenta, trachea, pancreas, kidney, gastric mucosa, colon, liver, cerebellum, skin, cortex (Alzheimer's and normal), hypothalamus, cortex, amygdala, cerebellum, hippocampus, choroid, plexus, thalamus, and spinal cord.

To demonstrate that GPCR is involved in the disease process of asthma, the following whole body panel is screened to show predominant or relatively high expression in lung or immune tissues: brain, heart, kidney, liver, lung, trachea, bone marrow, colon, small intestine, spleen, stomach, thymus, mammary gland, skeletal muscle, prostate, testis, uterus, cerebellum, fetal brain, fetal liver, spinal cord, placenta, adrenal gland, pancreas, salivary gland, thyroid, peripheral blood leukocytes, lymph node, and tonsil. Once this is established, the following lung and immune system cells are screened to localize expression to particular cell subsets: lung microvascular endothelial cells, bronchial/tracheal epithelial cells, bronchial/tracheal smooth muscle cells, lung fibroblasts, T cells (T helper 1 subset, T helper 2 subset, NKT cell subset, and cytotoxic. T lymphocytes), B cells, mononuclear cells (monocytes and macrophages), mast cells, eosinophils, neutrophils, and dendritic cells. As a final step, the expression of GPCR in cells derived from normal individuals with the expression of cells derived from asthmatic individuals is compared.

*Quantitative expression profiling.* Quantitative expression profiling is performed by the form of quantitative PCR analysis called "kinetic analysis" firstly described in Higuchi et al., BioTechnology 10, 413-17, 1992, and Higuchi et al., BioTechnology 11, 1026-30, 1993. The principle is that at any given cycle within the exponential phase of PCR, the amount of product is proportional to the initial number of template copies.

If the amplification is performed in the presence of an internally quenched fluorescent oligonucleotide (TaqMan probe) complementary to the target sequence, the probe is cleaved by the 5'-3' endonuclease activity of Taq DNA polymerase and a fluorescent dye released in the medium (Holland et al., Proc. Natl. Acad. Sci. U.S.A. 88, 7276-80, 1991). Because the fluorescence emission will increase in direct proportion to the amount of the specific amplified product, the exponential growth phase of PCR product can be detected and used to determine the initial template concentration (Heid et al., Genome Res. 6, 986-94, 1996, and Gibson et al., Genome Res. 6, 995-1001, 1996).

The amplification of an endogenous control can be performed to standardize the amount of sample RNA added to a reaction. In this kind of experiment, the control of choice is the 18S ribosomal RNA. Because reporter dyes with differing emission spectra are available, the target and the endogenous control can be independently quantified in the same tube if probes labeled with different dyes are used.

All "real time PCR" measurements of fluorescence are made in the ABI Prism 7700.

*RNA extraction and cDNA preparation.* Total RNA from the tissues listed above are used for expression quantification. RNAs labeled "from autopsy" were extracted from autoptic tissues with the TRIzol reagent (Life Technologies, MD) according to the manufacturer's protocol.

Fifty μg of each RNA were treated with DNase I for 1 hour at 37°C in the following reaction mix: 0.2 U/μl RNase-free DNase I (Roche Diagnostics, Germany); 0.4 U/μl RNase inhibitor (PE Applied Biosystems, CA); 10 mM Tris-HCl pH 7.9; 10 mM $MgCl_2$; 50 mM NaCl; and 1 mM DTT.

After incubation, RNA is extracted once with 1 volume of phenol:chloroform:- isoamyl alcohol (24:24:1) and once with chloroform, and precipitated with 1/10 volume of 3 M NaAcetate, pH5.2, and 2 volumes of ethanol.

Fifty μg of each RNA from the autoptic tissues are DNase treated with the DNA-free kit purchased from Ambion (Ambion, TX). After resuspension and spectrophotometric quantification, each sample is reverse transcribed with the TaqMan Reverse Transcription Reagents (PE Applied Biosystems, CA) according to the manufacturer's protocol. The final concentration of RNA in the reaction mix is 200 ng/μL. Reverse transcription is carried out with 2.5μM of random hexamer primers.

*TaqMan quantitative analysis.* Specific primers and probe are designed according to the recommendations of PE Applied Biosystems; the probe can be labeled at the 5' end FAM (6-carboxy-fluorescein) and at the 3' end with TAMRA (6-carboxy tetramethyl-rhodamine). Quantification experiments are performed on 10 ng of reverse transcribed RNA from each sample. Each determination is done in triplicate.

Total cDNA content is normalized with the simultaneous quantification (multiplex PCR) of the 18S ribosomal RNA using the Pre-Developed TaqMan Assay Reagents (PDAR) Control Kit (PE Applied Biosystems, CA).

The assay reaction mix is as follows: 1X final TaqMan Universal PCR Master Mix (from 2X stock) (PE Applied Biosystems, CA); 1X PDAR control - 18S RNA (from 20X stock); 300 nM forward primer; 900 nM reverse primer; 200 nM probe; 10 ng cDNA; and water to 25 μl.

Each of the following steps are carried out once: pre PCR, 2 minutes at 50°C, and 10 minutes at 95°C. The following steps are carried out 40 times: denaturation, 15 seconds at 95°C, annealing/extension, 1 minute at 60°C.

The experiment is performed on an ABI Prism 7700 Sequence Detector (PE Applied Biosystems, CA). At the end of the run, fluorescence data acquired during PCR are processed as described in the ABI Prism 7700 user's manual in order to achieve better background subtraction as well as signal linearity with the starting target quantity.

## EXAMPLE 20

*Expression profiling*

Total cellular RNA was isolated from cells by one of two standard methods: (1) guanidine isothiocyanate/Cesium chloride density gradient centrifugation or (2) the Tri-Reagent protocol according to the manufacturer's specifications (Molecular Research Center, Inc., Cincinatti, Ohio). Total RNA prepared by the Tri-reagent protocol was treated with DNAse I to remove genomic DNA contamination. RNA was isolated from the following sources: coronary smooth muscle cells, brain, testis, pancreas, stomach, cerebellum, trachea, adrenal gland, skeletal muscle, salivary gland, small intestine, prostata, fetal liver, placenta, fetal brain, uterus, mammary gland, heart, spleen, lung, HeLa cells, liver, kidney, thymus, bone marrow, thyroid, colon, bladder, spinal cord, peripheral blood, liver liver cirrhosis, pancreas liver cirrhosis, spleen liver cirrhosis, total Alzheimer brain, fetal lung, breast tumor, colon tumor, lung tumor, HEK 293 cells, adipose, pericardium, fetal heart, thyroid tumor, MDA MB 231 cells, HEP G2 cells, HUVEC cells, fetal kidney, breast, Jurkat T-cells, Alzheimer brain cortex, cervix, esophagus, thalamus, precentral gyrus, hippocampus, occipital lobe, cerebral peduncles, postcentral gyrus, temporal lobe, parietal lobe, cerebellum (right), cerebellum (left), tonsilla cerebelli, cerebral meninges, pons, frontal lobe, cerebral cortex, corpus callosum, vermis cerebelli, Alzheimer brain frontal lobe, interventricular septum, heart atrium (right), heart atrium (left), and heart ventricle (left).

For relative quantitation of the mRNA distribution of human GPCR, total RNA from each cell or tissue source was first reverse transcribed. Eighty-five μg of total RNA was reverse transcribed using 1 μmole random hexamer primers, 0.5 mM each of dATP, dCTP, dGTP, and dTTP (Qiagen, Hilden, Germany), 3000 U RnaseQut (Invitrogen, Groningen, Netherlands) in a final volume of 680 μl. The first strand synthesis buffer and Omniscript (2 u/μl) reverse transcriptase were from (Qiagen, Hilden, Germany). The reaction was incubated at 37°C for 90 minutes and cooled on ice. The volume was adjusted to 6800 μl with water, yielding a final concentration of 12.5 ng/μl of starting RNA.

For relative quantitation of the distribution of human GPCR mRNA in cells and tissues the Perkin Elmer ABI Prism®. 7700 Sequence Detection system or Biorad iCycler was used according to the manufacturer's specifications and protocols. PCR reactions were set up to quantitate human GPCR and the housekeeping genes HPRT, GAPDH, beta-actin, and others. Forward and reverse primers and probe for the human GPCR were designed using the Perkin Elmer ABI Piimer Express™. software and were synthesized by TibMolBiol (Berlin, Germany). The X forward primer sequence was: GGGTTACTGGTCCTGCCTCC. The X reverse primer sequence was AGAGGTTGGCAAGCAGGGA. The fluorogenic probe, labeled with FAM as the reporter dye and TAMRA as the quencher, is CGTCTACTTGGCTCCCAACTTCTCCTTCC.

The following reactions in a final volume of 25 μl were set up: 1X TaqMan buffer A, 5.5 mM MgC12, 200 nM each of dATP, dCTP, dGTP, and dUTP, 0.025 U/μl AmpliTaq Gold.TM., 0.01 U/μl AmpErase UNG® and probe 1X, human GPCR forward and reverse primers each at 200 nM, 200 nM human GPCR FAM/TAMRA-labelled probe, and 5 μl of template cDNA. Thermal cycling parameters were 2 min HOLD at 50°C, 10 min HOLD at 95°C, followed by melting at 95°C for 15 sec and annealing/extending at 60°C for 1 min for each of 40 cycles.

Calculation of corrected CT values

The CT-value is calculated as described above. The CF-value is calculated as followed :

1. PCR reactions were set up to quantitate the houskeeping genes (HKG) for each cDNA sample.

2. $CT_{HKG}$-values were calculated as described above

3. CT-mean values of all HKG for each cDNA are calculated (n = number of HKG):

$$(CT_{HKG1}\text{-value} + CT_{HKG2}\text{-value} + CT_{HKG\text{-}X}\text{-value}) / n = CT_{cDNA\text{-}X}\text{-mean values}$$

$$(n = \text{number of HKG})$$

4.

$$(CT_{cDNA\text{-}1}\text{-mean value} + CT_{cDNA\text{-}X}\text{-mean value}) / y = CT_{pannel}\text{-mean value}$$

$$(y = \text{number of cDNAs})$$

5.

$$CT_{pannel}\text{-mean value} - CT_{cDNA\text{-}X}\text{-mean value} = CF_{cDNA\text{-}X}$$

6.

$$CT_{cDNA\text{-}X} + CF_{cDNA\text{-}X} = CT_{cor\text{-}cDNA\text{-}X}$$

Calculation of relative expression

Definition:

$$\text{highest } CT_{cor\text{-}cDNA\text{-}X} \neq 40 \text{ is defined as } CT_{cor\text{-}cDNA\text{-}X}\ [\text{high}]$$

$$\text{Relative Expression} = 2e(CT_{cor\text{-}cDNA\text{-}X}\ [high] - CT_{cor\text{-}cDNA\text{-}Y})$$

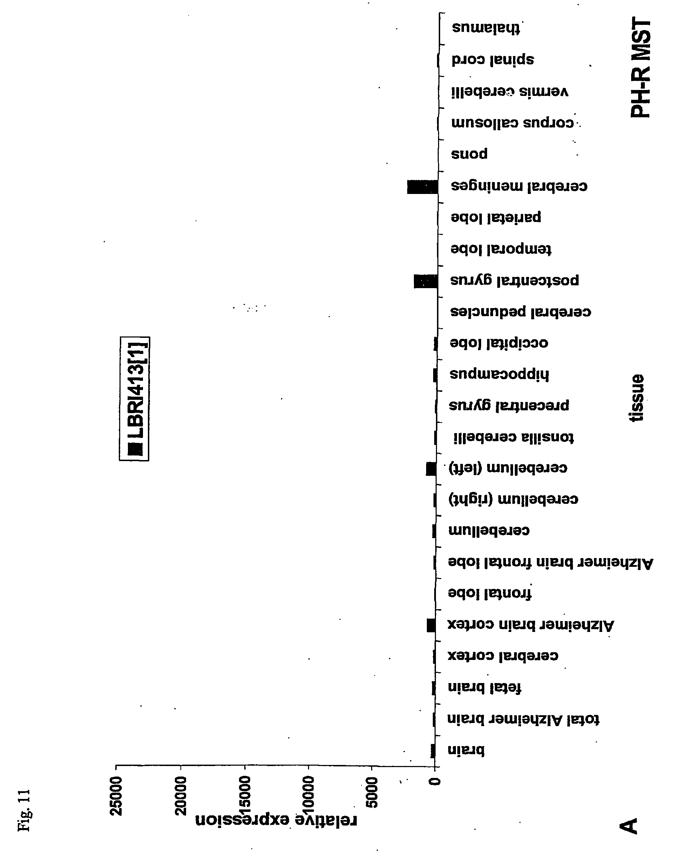

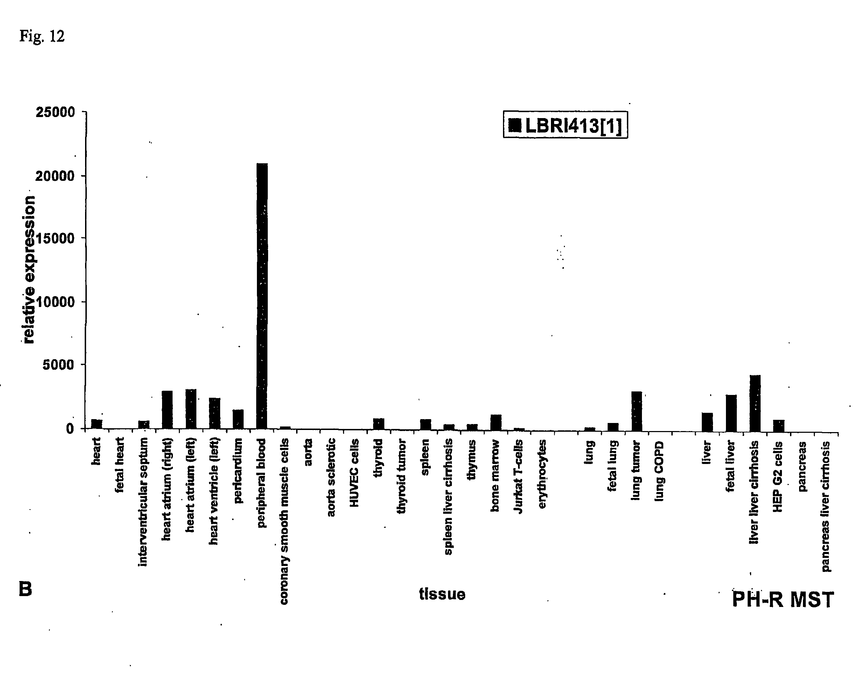

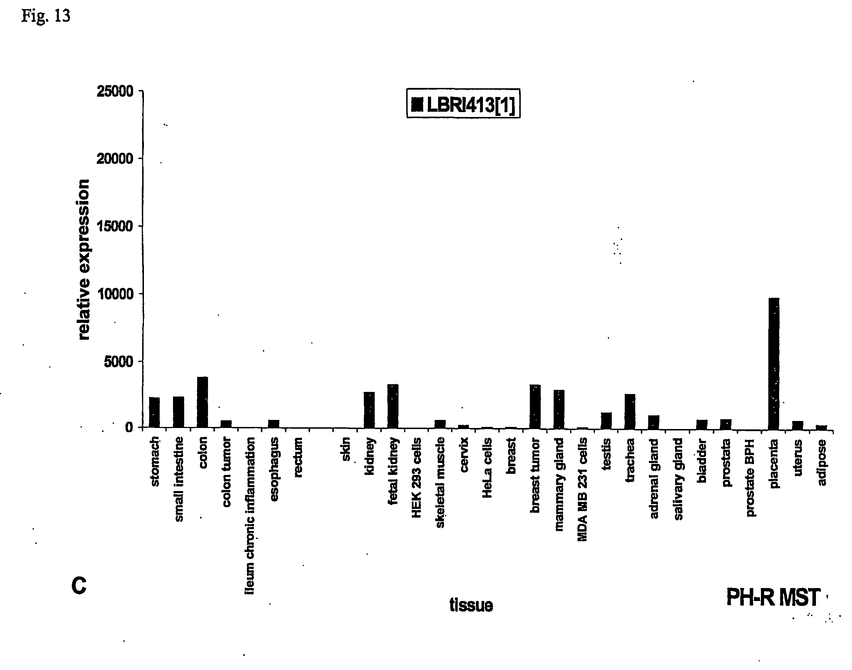

The results of the the mRNA-quantification (expression profiling) is shown in FIGS. 11-13.

Human GPCR mRNA is expressed in different human tissues. It is highly expressed in postcentral gyrus, cerebral meninges, heart (atrium :left + rigth), peripheral blood, lung tumor, fetal liver, liver (cirrhosis), stomach, small intestine, colon, kidney, fetal kidney, breast tumor, mammary gland, trachea, placenta.

Human GPCR mRNA is highly expressed in different brain tissues as postcentral gyrus and cerebral meninges. The expression in the above mentioned tissues suggests an association between human GPCR and peripheral and central nervous system diseases.

Human GPCR mRNA is is highly expressed in lung tumor and breast tumor. The expression in the above mentioned tissues suggests an association between human GPCR and cancer.

Human GPCR mRNA is highly expressed in liver, liver (liver cirrhosis), kidney, fetal kidney. The expression in the above mentioned tissues suggests an association between human GPCR and diseases of the liver and kidney.

Human GPCR mRNA is highly expressed in placenta. The expression in the above mentioned tissues suggests an association between human GPCR and genito-urinary diseases.

Human GPCR mRNA is highly expressed in mammary gland. The expression in the above mentioned tissues suggests an association between human GPCR and diseases of secretory organs.

Human GPCR mRNA is highly expressed in peripheral blood. The expression in the above mentioned tissues suggests an association between human GPCR and hematology, asthma and COPD.

Human GPCR mRNA is highly expressed in heart. The expression in the above mentioned tissues suggests an association between human GPCR and cardiovascular diseases.

## Claims

1. A method for detection of a polynucleotide encoding a G protein-coupled receptor polypeptide in a biological sample comprising the following steps:

I) hybridizing a polynucleotide selected from the group consisting of:

a) a polynucleotide encoding a G protein-coupted receptor polypeptide comprising an amino acid sequence selected from the group consisting of:

i. amino acid sequences which are at least 50% identical to the amino acid sequence shown in SEQ ID NO: 2; and
ii. the amino acid sequence shown in SEQ ID NO: 2.

b) a polynucleotide comprising the sequence of SEQ ID NO: 1;
c) a polynucleotide which hybridizes under stringent conditions to a polynucleotide specified in (a) and (b) and encodes a G protein-coupled receptor polypeptide;
d) a polynucleotide the sequence of which deviates from the polynucleotide sequences specified in (a) to (c) due to the degeneration of the genetic code and encodes a G protein-coupled receptor polypeptide; and
e) a polynucleotide which represents a fragment or allelic variation of a polynucleotide sequence specified in (a) to (d) and encodes a G protein-coupled receptor polypeptide to a nucleic acid material of a biological sample, thereby forming a hybridization complex; and

II) detecting said hybridization complex.

2. The method of claim 1, wherein before hybridization, the nucleic acid material of the biological sample is amplified.

3. A method for the detection of a polynucleotide of claim 1 or a G protein-coupled receptor polypeptide encoded by such polynucleotide comprising the steps of:

a) contacting a biological sample with an oligonucleotide which specifically interacts with the polynucleotide or with an antibody which specifically interacts with the G protein-coupled receptor polypeptide and
b) detecting the interaction.

4. A method of screening for agents useful for the treatment of breast or lung cancer which decrease the activity of a G protein-coupled receptor polypeptide, comprising the steps of:

a) contacting a test compound with any G protein-coupled receptor polypeptide encoded by a polynucleotide of claim 1:
b) detecting binding of the test compound to the G protein-coupled receptor polypeptide, wherein a test compound which binds to the polypeptide is identified as a potential therapeutic agent for decreasing the activity of a G protein-coupled receptor polypeptide.

5. A method of screening for agents useful for the treatment of colon cancer which increase the activity of a G protein-coupled receptor polypeptide, comprising the steps of:

a) contacting a test compound with a G protein-coupled receptor polypeptide encoded by any polynucleotide of claim 1; and
b) detecting a G protein-coupled receptor activity of the polypeptide, wherein a test compound which increases the G protein-coupled receptor activity is identified as a potential therapeutic agent for the treatment of colon cancer.

6. A method of screening for agents useful for the treatment of lung or breast cancer which decrease the activity of a G protein-coupled receptor polypeptide, comprising the steps of:

a) contacting a test compound with any polynucleotide of claim 1; and
b) detecting binding of the test compound to the polynucleotide, wherein a test compound which binds to the polynucleotide is identified as a potential therapeutic agent for decreasing the activity of a G protein-coupled receptor polypeptide.

## Patentansprüche

1. Verfahren zum Nachweis eines Polynucleotids, das für ein G-Protein-gekoppeltes Rezeptor-Polypeptid kodiert, in einer biologischen Probe, folgende Schritte umfassend:

I) Hybridisieren eines Polynucleotids, das aus der aus Folgendem bestehenden Gruppe ausgewählt ist:

a) einem Polynucleotid, das für ein G-Protein-gekoppeltes Rezeptor-Polypeptid kodiert, das eine Aminosäuresequenz umfasst, die aus der aus Folgendem bestehenden Gruppe ausgewählt ist:

i. Aminosäuresequenzen, die zu zumindest 50 % mit der in Seq.-ID Nr. 2 gezeigten Aminosäuresequenz identisch sind; und
ii. der in Seq.-ID Nr. 2 gezeigte Aminosäuresequenz,

b) einem Polynucleotid, das die Sequenz aus Seq.-ID Nr. 1 umfasst;
c) einem Polynucleotid, das unter stringenten Bedingungen an ein in (a) und (b) spezifiziertes Polynucleotid hybridisiert und für ein G-Protein-gekoppeltes Rezeptor-Polypeptid kodiert;
d) einem Polynucleotid, dessen Sequenz aufgrund der Degeneration des genetischen Codes von den in (a) bis (c) spezifizierten Polynucleotidsequenzen abweicht und das für ein G-Protein-gekoppeltes Rezeptor-Polypeptid kodiert; und
e) einem Polynucleotid, das ein Fragment oder eine Allel-Variation einer in (a) bis (d) spezifizierten Polynucleotidsequenz darstellt und für ein G-Protein-gekoppeltes Rezeptor-Polypeptid kodiert, an ein Nucleinsäurematerial von einer biologischen Probe, wodurch ein Hybridisierungskomplex gebildet wird; und

II) Nachweisen des Hybridisierungskomplexes.

**2.** Verfahren nach Anspruch 1, worin vor der Hybridisierung das Nucleinsäurematerial der biologischen Probe amplifiziert wird.

**3.** Verfahren zum Nachweis eines Polynucleotids nach Anspruch 1 oder eines G-Protein-gekoppelten Rezeptor-Polypeptids, für das ein solches Polynucleotid kodiert, folgende Schritte umfassend:

a) Kontaktieren einer biologischen Probe mit einem Oligonucleotid, das spezifisch mit dem Polynucleotid wechselwirkt, oder mit einem Antikörper, der spezifisch mit dem G-Protein-gekoppelten Rezeptor-Polypeptid wechselwirkt, und
b) Nachweisen der Wechselwirkung.

**4.** Verfahren zum Screenen auf zur Behandlung von Brust- oder Lungenkrebs nützliche Wirkstoffe, welche die Aktivität eines G-Protein-gekoppelten Rezeptor-Polypeptids verringern, folgende Schritte umfassend:

a) Kontaktieren einer Testverbindung mit einem beliebigen G-Protein-gekoppelten Rezeptor-Polypeptid, für das ein Polynucleotid nach Anspruch 1 kodiert,
b) Nachweisen einer Bindung der Testverbindung an das G-Protein-gekoppelte Rezeptor-Polypeptid, worin eine Testverbindung, die an das Polypeptid bindet, als mögliches Therapeutikum zur Verringerung der Aktivität eines G-Protein-gekoppelten Rezeptor-Polypeptids identifiziert wird.

**5.** Verfahren zum Screenen auf zur Behandlung von Kolonkrebs nützliche Wirkstoffe, welche die Aktivität eines G-Protein-gekoppelten Rezeptor-Polypeptids erhöhen, folgende Schritte umfassend:

a) Kontaktieren einer Testverbindung mit einem G-Protein-gekoppelten Rezeptor-Polypeptid, für das ein beliebiges Polynucleotid nach Anspruch 1 kodiert; und
b) Nachweisen einer G-Protein-gekoppelten Rezeptor-Aktivität des Polypeptids, worin eine Testverbindung, welche die Aktivität des G-Protein-gekoppelten Rezeptors erhöht, als mögliches Therapeutikum zur Behandlung von Kolonkrebs identifiziert wird.

**6.** Verfahren zum Screenen auf zur Behandlung von Lungen- oder Brustkrebs nützliche Wirkstoffe, welche die Aktivität eines G-Protein-gekoppelten Rezeptor-Polypeptids verringern, folgende Schritte umfassend:

a) Kontaktieren einer Testverbindung mit einem beliebigen Polynucleotid nach Anspruch 1; und
b) Nachweisen einer Bindung der Testverbindung an das Polynucleotid, worin eine Testverbindung, welche an das Polynucleotid bindet, als mögliches Therapeutikum zur Verringerung der Aktivität eines G-Protein-gekoppelten Rezeptor-Polypeptids identifiziert wird.

## Revendications

**1.** Méthode pour la détection d'un polynucléotide codant pour un polypeptide récepteur couplé à la protéine G dans un échantillon biologique, comprenant les étapes suivantes :

I) l'hybridation d'un polynucléotide choisi dans le groupe consistant en :

a) un polynucléotide codant pour un polypeptide récepteur couplé à la protéine G, comprenant une séquence d'aminoacides choisie dans le groupe consistant en :

i. des séquences d'aminoacides qui sont identiques à au moins 50 % à la séquence d'aminoacides représentée dans la SEQ ID N° 2 ; et
ii. la séquence d'aminoacides représentée dans la SEQ ID N° 2.

b) un polynucléotide comprenant la séquence de la SEQ ID N° 1 ;
c) un polynucléotide qui s'hybride dans des conditions stringentes à un polynucléotide spécifié en (a) et (b) et qui code pour un polypeptide récepteur couplé à la protéine G ;

d) un polynucléotide dont la séquence s'écarte des séquences polynucléotidiques spécifiées en (a) à (c) en raison de la dégénérescence du code génétique et qui code pour un polypeptide récepteur couplé à la protéine G ; et

e) un polynucléotide qui représente un fragment ou une variation allélique d'une séquence polynucléotidique spécifiée en (a) à (d) et qui code pour un polypeptide récepteur couplé à la protéine G, à une matière du type acide nucléique d'un échantillon biologique, en formant ainsi un complexe d'hybridation ; et

II) la détection dudit complexe d'hybridation.

**2.** Méthode suivant la revendication 1, dans laquelle, avant hybridation, la matière du type acide nucléique de l'échantillon biologique est amplifiée.

**3.** Méthode pour la détection d'un polynucléotide de la revendication 1 ou d'un polypeptide récepteur couplé à la protéine G codé par ce polynucléotide, comprenant les étapes consistant à :

a) mettre en contact un échantillon biologique avec un oligonucléotide qui interagit spécifiquement avec le polynucléotide ou avec un anticorps qui interagit spécifiquement avec le polypeptide récepteur couplé à la protéine G et

b) détecter l'interaction.

**4.** Méthode pour sélectionner des agents utiles pour le traitement du cancer du sein ou du poumon, qui diminuent l'activité d'un polypeptide récepteur couplé à la protéine G, comprenant les étapes consistant à :

a) mettre en contact un composé d'essai avec n'importe quel polypeptide récepteur couplé à la protéine G codé par un polynucléotide de la revendication 1 ;

b) détecter la liaison du composé d'essai au polypeptide récepteur couplé à la protéine G, un composé d'essai qui se lie au polypeptide étant identifié comme agent thérapeutique potentiel pour diminuer l'activité d'un polypeptide récepteur couplé à la protéine G.

**5.** Méthode pour sélectionner des agents utiles pour le traitement du cancer du côlon, qui augmentent l'activité d'un polypeptide récepteur couplé à la protéine G, comprenant les étapes consistant à :

a) mettre en contact un composé d'essai avec un polypeptide récepteur couplé à la protéine G codé par n'importe quel polynucléotide de la revendication 1 ; et

b) détecter une activité de récepteur couplé à la protéine G du polypeptide, un composé d'essai qui augmente l'activité du récepteur couplé à la protéine G étant identifié en tant qu'agent thérapeutique potentiel pour le traitement du cancer du côlon.

**6.** Méthode pour sélectionner des agents utiles pour le traitement du cancer du poumon ou du sein, qui diminuent l'activité d'un polypeptide récepteur couplé à la protéine G, comprenant les étapes consistant à :

a) mettre en contact un composé d'essai avec n'importe quel polynucléotide de la revendication 1 ; et

b) détecter la liaison du composé d'essai au polynucléotide, un composé d'essai qui se lie au polynucléotide étant identifié comme agent thérapeutique potentiel pour diminuer l'activité d'un polypeptide récepteur couplé à la protéine G.

Fig.1

atgacgccca acagcactgg cgaggtgccc agccccattc ccaaggggc tttggggctc

tccctggccc tggcaagcct catcatcacc gcgaacctgc tcctagccct gggcatcgcc

tgggaccgcc gcctgcgcag cccacctgct ggctgcttct tcctgagcct actgctggct

gggctgctca cgggtctggc attgcccaca ttgccagggc tgtggaacca gagtcgccgg

ggttactggt cctgcctcct cgtctacttg gctcccaact tctccttcct ctccctgctt

gccaacctct tgctggtgca cggggagcgc tacatggcag tcctgaggcc actccagccc

cctgggagca ttcggctggc cctgctcctc acctgggctg gtcccctgct ctttgccagt

ctgcccgctc tggggtggaa ccactggacc cctggtgcca actgcagctc ccaggctatc

ttcccagccc cctacctgta cctcgaagtc tatgggctcc tgctgcccgc cgtgggtgct

gctgccttcc tctctgtccg cgtgctggcc actgcccacc gccagctgca ggacatctgc

cggctggagc gggcagtgtg ccgcgatgag ccctccgccc tggcccgggc ccttacctgg

aggcaggcaa gggcacaggc tggagccatg ctgctcttcg ggctgtgctg gggggccctac

gtggccacac tgctcctctc agtcctggcc tatgagcagc gcccgccact ggggcctggg

acactgttgt ccctcctctc cctaggaagt gccagtgcag cggcagtgcc cgtagccatg

gggctgggcg atcagcgcta cacagccccc tggagggcag ccgcccaaag gtgcctgcag

gggctgtggg gaagagcctc ccgggacagt cccggcccca gcattgccta ccacccaagc

agccaaagca gtgtcgacct ggacttgaac

Fig. 2

MTPNSTGEVP SPIPKGALGL SLALASLIIT ANLLLALGIA WDRRLRSPPA GCFFLSLLLA
GLLTGLALPT LPGLWNQSRR GYWSCLLVYL APNFSFLSLL ANLLLVHGER YMAVLRPLQP

PGSIRLALLL TWAGPLLFAS LPALGWNHWT PGANCSSQAI FPAPYLYLEV YGLLLPAVGA
AAFLSVRVLA TAHRQLQDIC RLERAVCRDE PSALARALTW RQARAQAGAM LLFGLCWGPY
VATLLLSVLA YEQRPPLGPC TLLSLLSLGS ASAAAVPVAM GLGDQRYTAP WRAAAQRCLQ

GLWGRASRDS PGPSIAYHPS SQSSVDLDLN

Fig. 3

ACCTGGGCCCCGCTTCTATAGAAGGTATTTTAGCCAAAACTGGACCCGCGGCTATGAGCA

GCGCCCGCCACTGGGGCCTGGACACTGTTGTCCCTCCTCTCCCTAGGAAGTGCCAGTGC

AGCGGCAGTGCCCGTAGCCATGGGGCTGGGCGATCAGCGCTACACAGCCCCCTGGAGGGC

AGCCGCCCAAAGGTGCCTGCAGGGGCTGTGGGAAGAGCCTCCCGGGACAGTCCCGGCCC

CAGCATTGCCTACCACCCAAGCAGCCAAAGCAGTGTCGACCTGGACTTGAACTAAAGGAA

GGGCCTCTGCTGACTCCTACCAGAGCATCCGTCCAGCTCAGCCATCCAGCCTGTCTCTAC

TGGGCCCCACTTCTCTGGATCAGAGACCCTGCCTCTGTTTGACCCCGCACTGACTGAATA
AAGCTCCTCATAGGCCG

Fig. 4

GGCCTGGCCCCGCTGTCCCTACTGGGTGGAGACACCATGTACTTGGTCCACTTGTGCTCT

TCAGCCAGGACACCAGACATGGTCCAAACCGCTGCAGGGCTGGCTGCAGCAACTCCCTGA

CACTCAGGAAGGCCCAGGCTGGGCAGGCAATACCTGCTCCCAACAGCCATGCATGCCGGC

TGCCGCTCCAGGACTCCCCTGTCCCCAGGACCAAGATGACGCCCAACAGCACTGGCGAGG

TGCCCAGCCCCATTCCCAAGGGGGCTTTGGGGCTCTCCCTGGCCCTGGCAAGCCTCATCA

TCACCGCGAACCTGCTCCTAGCCCTGGGCATCGCCTGGGACCGCCGCCTGCGCAGCCCAC

CTGCTGGCTGCTTCTTCCTGAGCCTACTGCTGGCTGGGCTGCTCACGGGTCTGGCATTGC
CCACATTGCCAGGGCTGTGGAACCAGAGTCGCCGGG

Fig. 5

TTTTTAACGGCCAGAGGAGCTTTATTCAGTCAGTGCGGGGTCAAACAGAGGCAGGGTCTC

TGATCCAGAGAAGTGGGGCCCAGTAGAGACAGGCTGGATGGCTGAGCTGGACGGATGCTC

TGGTAGGAGTCAGCAGAGGCCCTTCCTTTAGTTCAAGTCCAGGTCGACACTGCTTTGGCT

GCTTGGGTGGTAGGCAATGCTGGGGCCGGGACTGTCCCGGGAGGCTCTTCCCCACAGCCC

CTGCAGGCACCTTTGGGCGGCTGCCCTCCAGGGGGCTGTGTAGCGCTGATCGCCCAGCCC

CATGGCTACGGGCACTGCCGCTGCACTGGCACTTCCTAGGGAGAGGAGGGACAACAGTGT

CCCAGGCCCCAGTGGCGTCATCTTGGTCCTGNGGACAGGGGAGTCCTGGAGCGGCAGCCG
GCATGCATGGCTGT

Fig. 6

TATGAGAGTCTTTATCTGTTTCACCCCGGTGAGAGCATTTTGAATTTCCTTATTGACGTA

CTTACTTCCCTGATTGGACATTTCCTGGAGCTCATTGTCTGAGACCGTGTGGTCCCCCAG

GACTGGGCCAGTCTCGCCAGGTGAGGAGCAGGGCCAGAAAGAGGAGCAGGGTGTTCATCA
TGGCTCCAATTCTGGAGTCTTTGCACGCCTCGGTCAGCGGC

Fig. 7

BLASTP - alignment of 413 against aageneseq|Y06411|Y06411Human EDG-7 receptor homologue.

This hit is scoring at : 2e-11 (expectation value)
Alignment length (overlap) : 268
Identities : 30 %
Scoring matrix : BLOSUM62 (used to infer consensus pattern)
Database searched : aageneseq

```
Q:      16 GAL-
GLSLALASLIITANLLLALGIAWDRRLRSPPAGCFFLSLLLAGLLTGLALPTLPGL
           GAL GLS:A.:.L::..NLL:...I. ..R R.    C. :::.L:.LLTG.A. ..
L
H:      47 GALRGLSVAASCLVVLENLLVLAAITSHMRSRRWVYYCL-VNITLSDLLTGAAY-
LANVL

           WNQSRRGYWSCLLVYLAPNFSFLSLLAN---
LLLVHGERYMAVLRPLQPPGSIRLALL--
            : :R.   : .  :L.... F.:L.A:   LL.. GER:..::RP:...G:.:.: :

LSGARTFRLAPAQWFLREGLLFTALAASTFSLLFTAGERFATMVRPVAESGATKTSRVYG

           ---
LTWAGPLLFASLPALGWNHWTPGANCSSQAIFPAPYLYLEVYGLLLPAVGAAAFLSV
              L.W. ..L...LP.LGWN  ..  .CSS  :.P   LY : Y L.. .: A.
           FIGLCWLLAALLGMLPLLGWNCLCAFDRCSS--LLP---LYSKRYILFCLVIFAG---
--

RVLATAHRQLQDICRLERAVCRDEPSALARALTWRQARAQAGAMLLFGLCWGPYVATLLL
            VLAT.   .  I RL :A  :..P...AR. . R .:. . .:L.F
:CWGP....LL.
           -
VLATIMGLYGAIFRLVQASGQKAPRPAARRKARRLLKTVLMILLAFLVCWGPLFGLLLA

           SVLA---YEQRPPLGPGTLLSLLSLGSA      271
           .V..   :.Q.   G . :L:L. L.SA
           DVFGSNLWAQEYLRGMDWILALAVLNSA      301
```

Fig. 8

HMMPFAM - alignment of 413 against pfam|hmm|7tm_17 transmembrane receptor (rhodopsin family)

This hit is scoring at : 12.5

Scoring matrix : BLOSUM62 (used to infer consensus pattern)

```
Q:      31 ANLLLALGIAWDRRLRsPPAGCFFLSLLLAGLLTGLALPTLPGLWNQS--RRGYWS--
-C
           .NLL:.L I.  .::LR .P...F.L:L.:A.LL  L.LP. . .:   .  .    :.
C
H:       1
GNlLVilvilrtkklr.tptnifilNLAvADLLfll tlppwalyylvggsedWpfGsalC

           LLVYLAPNFSFLSLLANLLLVHGERYMAVLRPLQPPGSIR------
LALLLTWAGPLLFA
            LV ... .:..: :. L..:. :RY:A::.PL:    . .       :.:LL.W.
.LL.:

klvtaldvvnmyaSillLtaISiDRYlAIvhPlryrrrrtsprrAkvvillvWvlallls

           SLPALGWNHWTPGAN----------CSSQAIFPA----------
PYLYLEVYGLLLPaVG
             P.L      T  ..           C  .   .:             .L. .:.G.LLP
:

lPpllfswvktveegngtlnvnvtvClidfpeestasvstwlrsyvllstlvgFllP.ll

AAAFLSVRVLATAHrqlqdicrleravcrdepsalaraltwRQARAQAGAMLLFGLCWGP
           ..... .R:L.T..                            :.A:. . .:::F LCW
P

vilvcYtrIlrtlr..........................kaaktllvvvvvFvlCWlP

           YVATLLLSVLA    250
           Y...LLL..L.
           yfivllldtlc    222
```

Fig. 9

Genewise analysis of target #413 genewise output

Score 811.05 bits over entire alignment

Scores as bits over a synchronous coding model

413 1
MTPNSTGEVPSPIPKGALGLSLALASLIITANLLLALGIAWDRRLRSPP

MTPNSTGEVPSPIPKGALGLSLALASLIITANLLLALGIAWDRRLRSPP

MTPNSTGEVPSPIPKGALGLSLALASLIITANLLLALGIAWDRRLRSPP

AC055884 -91463
aacaaagggcacacaggtgctcgcgacaaagacccgcgagtgccccacc

tccagcgatcgctcagctgtctctcgtttccatttctgtcgaggtggcc

ggccctcggccctcggtggccgcgacccccgcgcacgcccgcccgccat

413 50
AGCFFLSLLLAGLLTGLALPTLPGLWNQSRRGYWSCLLVYLAPNFSFLS

AGCFFLSLLLAGLLTGLALPTLPGLWNQSRRGYWSCLLVYLAPNFSFLS

AGCFFLSLLLAGLLTGLALPTLPGLWNQSRRGYWSCLLVYLAPNFSFLS

AC055884 -91316
ggtttcacccggccagcgtcatcgctacaccgttttccgttgcatttct

cggtttgtttcgttcgtctcctcgtgaaggggagcgtttatccatcttc

tccccgcaggtggcgtgagcagagggcgtcgtcgcccccccgtccccccc

413 99
LLANLLLVHGERYMAVLRPLQPPGSIRLALLLTWAGPLLFASLPALGWN

LLANLLLVHGERYMAVLRPLQPPGSIRLALLLTWAGPLLFASLPALGWN

LLANLLLVHGERYMAVLRPLQPPGSIRLALLLTWAGPLLFASLPALGWN

AC055884 -91169
ccgactcgcggctaggcacccccgaaccgcccatggccctgaccgcgta

ttcatttagagatcttgctaccggtgtctttcgcgctttcgtcctgga

gtcccgggcggccgacggacgctgctggcgcccgttcgctctgctgggc

Fig. 9 (continued)

413 148
HWTPGANCSSQAIFPAPYLYLEVYGLLLPAVGAAAFLSVRVLATAHRQL

HWTPGANCSSQAIFPAPYLYLEVYGLLLPAVGAAAFLSVRVLATAHRQL

HWTPGANCSSQAIFPAPYLYLEVYGLLLPAVGAAAFLSVRVLATAHRQL

AC055884 -91022
ctacggatatcgatcgctctcggtgccccgggggtctgcgcgagcccc

agccgcaggcacttcccatatatagtttcctgcccttctgttcccagat

cgcttcccccgtccacccgccactgcggccgtttccctccggctcccgg

413 197
QDICRLERAVCRDEPSALARALTWRQARAQAGAMLLFGLCWGPYVATLL

QDICRLERAVCRDEPSALARALTWRQARAQAGAMLLFGLCWGPYVATLL

QDICRLERAVCRDEPSALARALTWRQARAQAGAMLLFGLCWGPYVATLL

AC055884 -90875
cgatccgcggtcggctgcgcgcatacgagcgggacctgcttgctggacc

aatggtagctggaaccctcgctcggacgcacgcttttgtgggcatcctt

gcccggggagcctgcccgcgctcgggagagtacggccggcggccgcagc

413 246
LSVLAYEQRPPLGPGTLLSLLSLGSASAAAVPVAMGLGDQRYTAPWRAA

LSVLAYEQRPPLGPGTLLSLLSLGSASAAAVPVAMGLGDQRYTAPWRAA

LSVLAYEQRPPLGPGTLLSLLSLGSASAAAVPVAMGLGDQRYTAPWRAA

AC055884 -90728
ctgcgtgcccccgcgacttcctcgagaggggcggagcggcctagctagg

tcttcaaagcctgcgcttcttctggcgccctctctgtgaagacccggcc

Fig. 9 (continued)

cacgctggcgaggtgaggccccaatctagagcacgggctgccaccggac

413 295 AQRCLQGLWGRASRDSPGPSIAYHPSSQSSVDLDLN

AQRCLQGLWGRASRDSPGPSIAYHPSSQSSVDLDLN

AQRCLQGLWGRASRDSPGPSIAYHPSSQSSVDLDLN

AC055884 -90581 gcatccgctgagtcgacgcaagtccaacaaggcgta

caggtagtgggccgagcgcgtcaacggaggtatata

cagcgggggaaccgctcccctcccaccactccgcgc

//

Gene 1

Gene 91463 90474

Exon 91463 90474

//

>AC055884.[91463:90472].sp.tr

MTPNSTGEVPSPIPKGALGLSLALASLIITANLLLALGIAWDRRLRSPPAGCFFLSLLLA

GLLTGLALPTLPGLWNQSRRGYWSCLLVYLAPNFSFLSLLANLLLVHGERYMAVLRPLQP

PGSIRLALLLTWAGPLLFASLPALGWNHWTPGANCSSQAIFPAPYLYLEVYGLLLPAVGA

AAFLSVRVLATAHRQLQDICRLERAVCRDEPSALARALTWRQARAQAGAMLLFGLCWGPY

VATLLLSVLAYEQRPPLGPGTLLSLLSLGSASAAAVPVAMGLGDQRYTAPWRAAAQRCLQ

GLWGRASRDSPGPSIAYHPSSQSSVDLDLN

//

Fig. 9 (continued)

>AC055884.[91463:90472].sp

ATGACGCCCAACAGCACTGGCGAGGTGCCCAGCCCCATTCCCAAGGGGGCTTTGGGGCTC

TCCCTGGCCCTGGCAAGCCTCATCATCACCGCGAACCTGCTCCTAGCCCTGGGCATCGCC

TGGGACCGCCGCCTGCGCAGCCCACCTGCTGGCTGCTTCTTCCTGAGCCTACTGCTGGCT

GGGCTGCTCACGGGTCTGGCATTGCCCACATTGCCAGGGCTGTGGAACCAGAGTCGCCGG

GGTTACTGGTCCTGCCTCCTCGTCTACTTGGCTCCCAACTTCTCCTTCCTCTCCCTGCTT

GCCAACCTCTTGCTGGTGCACGGGGAGCGCTACATGGCAGTCCTGAGGCCACTCCAGCCC

CCTGGGAGCATTCGGCTGGCCCTGCTCCTCACCTGGGCTGGTCCCCTGCTCTTTGCCAGT

CTGCCCGCTCTGGGGTGGAACCACTGGACCCCTGGTGCCAACTGCAGCTCCCAGGCTATC

TTCCCAGCCCCCTACCTGTACCTCGAAGTCTATGGGCTCCTGCTGCCCGCCGTGGGTGCT

GCTGCCTTCCTCTCTGTCCGCGTGCTGGCCACTGCCCACCGCCAGCTGCAGGACATCTGC

CGGCTGGAGCGGGCAGTGTGCCGCGATGAGCCCTCCGCCCTGGCCCGGGCCCTTACCTGG

AGGCAGGCAAGGGCACAGGCTGGAGCCATGCTGCTCTTCGGGCTGTGCTGGGGGCCCTAC

GTGGCCACACTGCTCCTCTCAGTCCTGGCCTATGAGCAGCGCCCGCCACTGGGGCCTGGG

ACACTGTTGTCCCTCCTCTCCCTAGGAAGTGCCAGTGCAGCGGCAGTGCCCGTAGCCATG

GGGCTGGGCGATCAGCGCTACACAGCCCCCTGGAGGGCAGCCGCCCAAAGGTGCCTGCAG

GGGCTGTGGGGAAGAGCCTCCCGGGACAGTCCCGGCCCCAGCATTGCCTACCACCCAAGC

AGCCAAAGCAGTGTCGACCTGGACTTGAAC

//

AC055884 GeneWise match 90472 91463 811.05 -
.

413

AC055884 GeneWise cds 91462 904720.00 -
0
BLASTN - alignment of 413_DNA against GENBANK|AC055884|

Fig. 10

MTPNSTGEVP SPIPKGALGL SLALASLIIT ANLLLALGIA WDRRLRSPPA GCFFLSLLLA
GLLTGLALPT LPGLWNQSRR GYWSCLLVYL APNFSFL**SLL ANLLLVHGER YMA**VLRPLQP-

PGSIRLALLL TWAGPLLFAS LPALGWNHWT PGANCSSQAI FPAPYLYLEV YGLLLPAVGA
AAFLSVRVLA TAHRQLQDIC RLERAVCRDE PSALARALTW RQARAQAGAM LLFGLCWGPY
VATLLLSVLA YEQRPPLGPG TLLSLLSLGS ASAAAVPVAM GLGDQRYTAP WRAAAQRCLQ

GLWGRASRDS PGPSIAYHPS SQSSVDLDLN

Fig. 11

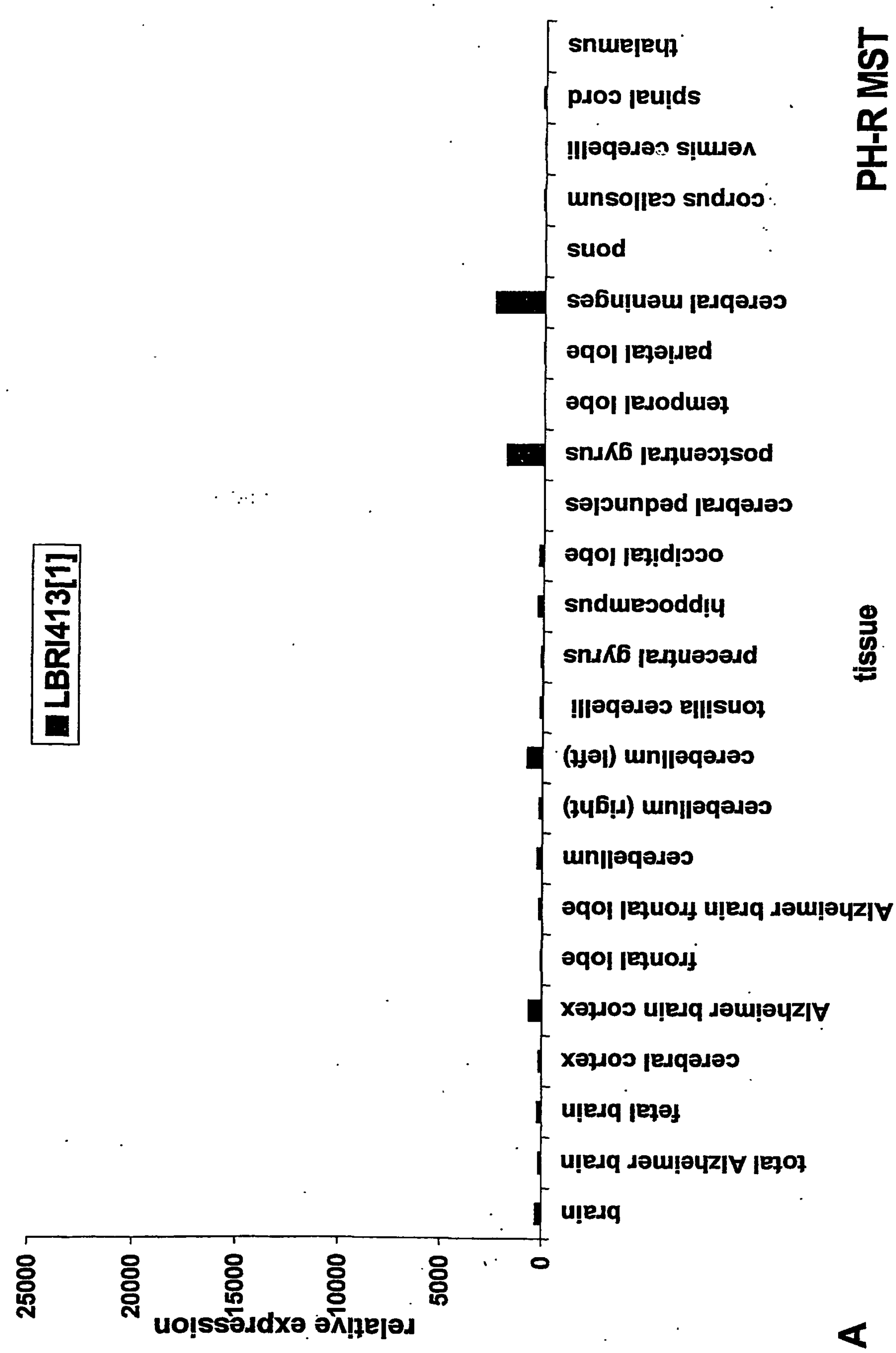
LBRI413[1]
PH-R MST
relative expression
25000
20000
15000
10000
5000
0
tissue
brain
total Alzheimer brain
fetal brain
cerebral cortex
Alzheimer brain cortex
frontal lobe
Alzheimer brain frontal lobe
cerebellum
cerebellum (right)
cerebellum (left)
tonsilla cerebelli
precentral gyrus
hippocampus
occipital lobe
cerebral peduncles
postcentral gyrus
temporal lobe
parietal lobe
cerebral meninges
pons
corpus callosum
vermis cerebelli
spinal cord
thalamus

A

Fig. 12

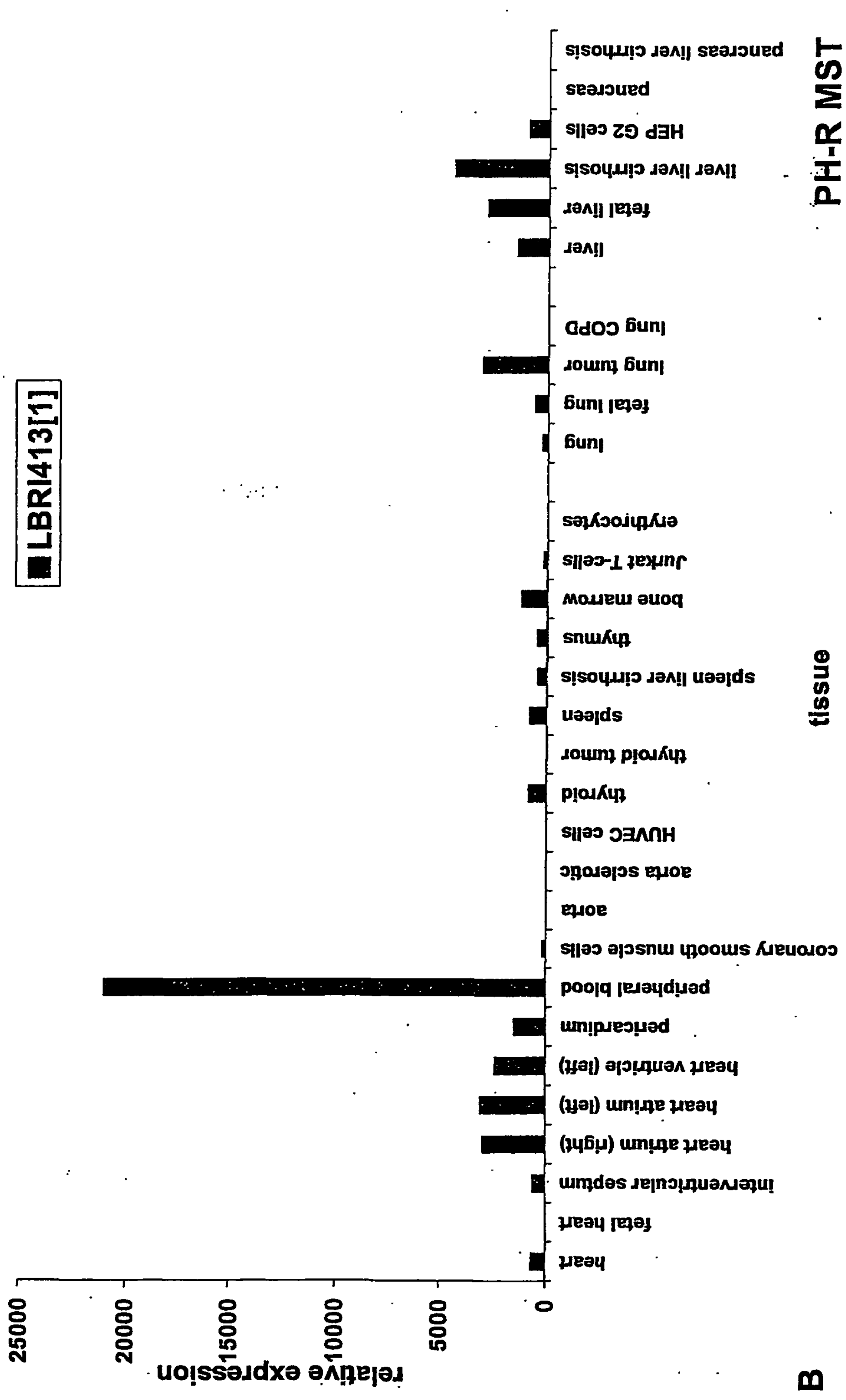
LBRI413[1]
relative expression
25000
20000
15000
10000
5000
0
heart
fetal heart
interventricular septum
heart atrium (right)
heart atrium (left)
heart ventricle (left)
pericardium
peripheral blood
coronary smooth muscle cells
aorta
aorta sclerotic
HUVEC cells
thyroid
thyroid tumor
spleen
spleen liver cirrhosis
thymus
bone marrow
Jurkat T-cells
erythrocytes
lung
fetal lung
lung tumor
lung COPD
liver
fetal liver
liver liver cirrhosis
HEP G2 cells
pancreas
pancreas liver cirrhosis
tissue
PH-R MST
B

Fig. 13

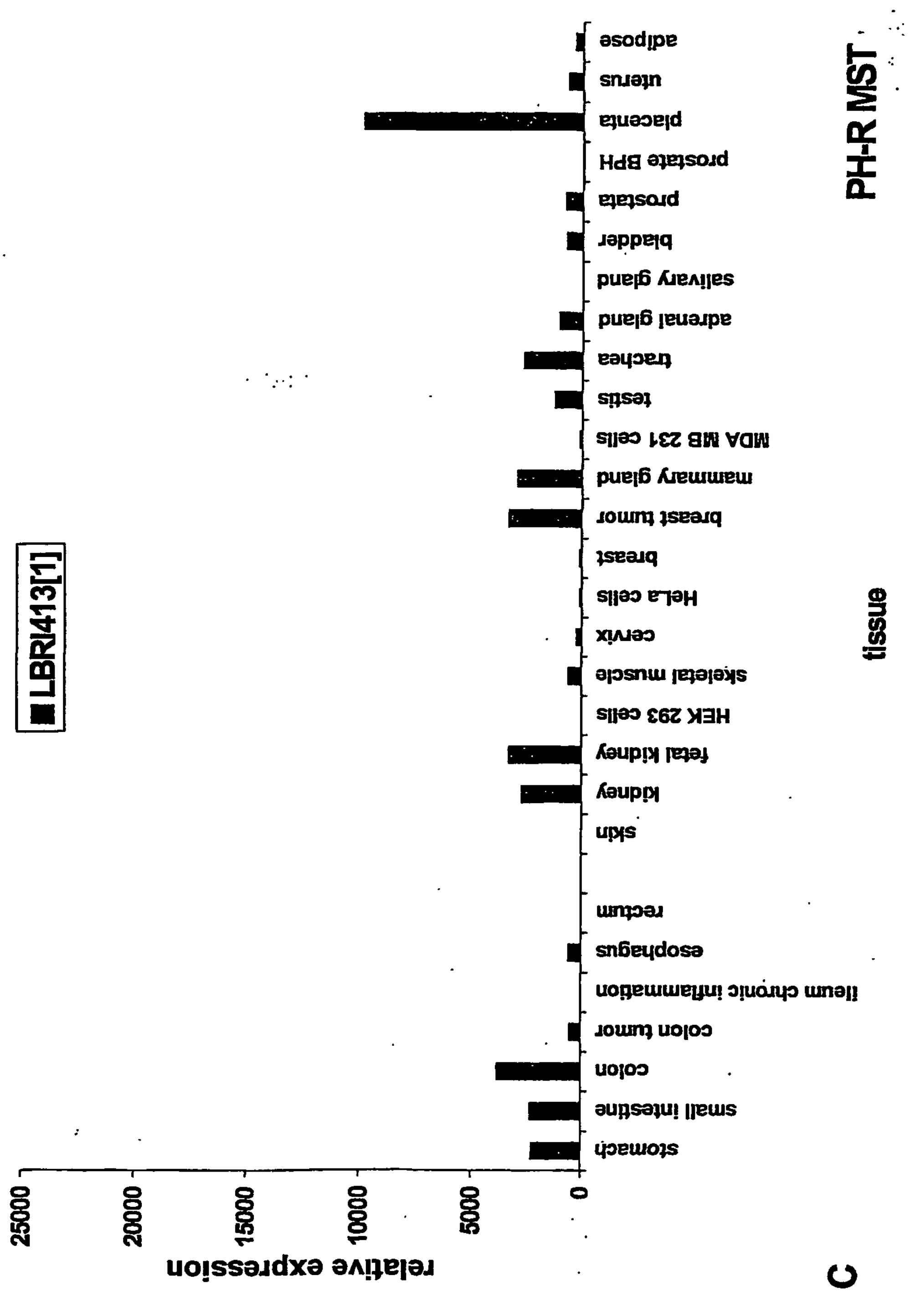
LBRI413[1]
PH-R MST
relative expression
25000
20000
15000
10000
5000
0
stomach
small intestine
colon
colon tumor
ileum chronic inflammation
esophagus
rectum
skin
kidney
fetal kidney
HEK 293 cells
skeletal muscle
cervix
HeLa cells
breast
breast tumor
mammary gland
MDA MB 231 cells
testis
trachea
adrenal gland
salivary gland
bladder
prostata
prostate BPH
placenta
uterus
adipose
tissue
C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 60274233 A
- WO 0064942 A
- GB 2188638 B
- US 5565332 A
- WO 9303151 A
- WO 9413804 A
- US 5641673 A, Haseloff
- EP 321201 A, Gerlach
- US 4843155 A, Chomczynski
- US 5262311 A
- US 5223409 A
- US 5976813 A, Beutel
- US 5283317 A
- WO 9410300 A, Brent
- WO 9201810 A
- US 5705151 A
- US 26941001 P
- US 32380901 P


### Non-patent literature cited in the description


- **LEFKOWITZ.** *Nature,* 1991, vol. 351, 353-354
- **DOHLMAN et al.** *Ann. Rev. Biochem.,* 1991, vol. 60, 653-88
- **JUPPNER et al.** *Science,* 1991, vol. 254, 1024-26
- **LIN et al.** *Science,* 1991, vol. 254, 1022-24
- **NAKANISHI.** *Science,* 1992, vol. 258, 597-603
- **KLEIN et al.** *Science,* 1988, vol. 241, 1467-72
- **KURJAN.** *Ann. Rev. Biochem.,* 1992, vol. 61, 1097-1129
- **JOHNSON et al.** *Endoc. Rev.,* 1989, vol. 10, 317-331
- **BONNER et al.** *J. Mol. Biol.,* 1973, vol. 81, 123
- **SAMBROOK et al.** MOLECULAR CLONING: A LABORATORY MANUAL. 1989, 9.50-9.51
- **BOLTON ; MCCARTHY.** *Proc. Natl. Acad. Sci. U.S.A.,* 1962, vol. 48, 1390
- **SARKAR.** *PCR Methods Applic.,* 1993, vol. 2, 318-322
- **TRIGLIA et al.** *Nucleic Acids Res.,* 1988, vol. 16, 8186
- **LAGERSTROM et al.** *PCR Methods Applic.,* 1991, vol. 1, 111-119
- **PARKER et al.** *Nucleic Acids Res.,* 1991, vol. 19, 3055-3060
- **SAMBROOK ; AUSUBEL et al.** CURRENT PROTOCOLS IN MOLECULAR BIOLOGY. John Wiley & Sons, 1989
- **VAN HEEKE ; SCHUSTER.** *J. Biol. Chem.,* 1989, vol. 264, 5503-5509
- **AUSUBEL ; GRANT et al.** *Methods Enzymol.,* 1987, vol. 153, 516-544
- **TAKAMATSU.** *EMBO J.,* 1987, vol. 6, 307-311
- **CORUZZI et al.** *EMBO J.,* 1984, vol. 3, 1671-1680
- **BROGLIE et al.** *Science,* 1984, vol. 224, 838-843
- **WINTER et al.** *Results Probl. Cell Differ.,* 1991, vol. 17, 85-105
- **HOBBS ; MURRAY.** YEARBOOK OF SCIENCE AND TECHNOLOGY. MCGRAW HILL, 1992, 191-196
- **ENGELHARD et al.** *Proc. Nat. Acad. Sci.,* 1994, vol. 91, 3224-3227
- **LOGAN ; SHENK.** *Proc. Natl. Acad. Sci.,* 1984, vol. 81, 3655-3659
- **SCHARF et al.** *Results Probl. Cell Differ.,* 1994, vol. 20, 125-162
- **WIGLER et al.** *Cell,* 1977, vol. 11, 223-32
- **LOWY et al.** *Cell,* 1980, vol. 22, 817-23
- **WIGLER et al.** *Proc. Natl. Acad. Sci.,* 1980, vol. 77, 3567-70
- **COLBERE-GARAPIN et al.** *J. Mol. Biol.,* 1981, vol. 150, 1-14
- **HARTMAN ; MULLIGAN.** *Proc. Natl. Acad. Sci.,* 1988, vol. 85, 8047-51
- **RHODES et al.** *Methods Mol. Biol.,* 1995, vol. 55, 121-131
- **HAMPTON et al.** SEROLOGICAL METHODS: A LABORATORY MANUAL. APS Press, 1990
- **MADDOX et al.** *J. Exp. Med.,* 1983, vol. 158, 1211-1216
- **PORATH et al.** *Prot. Exp. Purif.,* 1992, vol. 3, 263-281
- **KROLL et al.** *DNA Cell Biol.,* 1993, vol. 12, 441-453
- **CARUTHERS et al.** *Nucl. Acids Res. Symp. Ser.,* 1980, 215-223

- **HORN et al.** *Nucl. Acids Res. Symp. Ser.,* 1980, 225-232
- **MERRIFIELD.** *J. Am. Chem. Soc.,* 1963, vol. 85, 2149-2154
- **ROBERGE et al.** *Science,* 1995, vol. 269, 202-204
- **CREIGHTON.** PROTEINS: STRUCTURES AND MOLECULAR PRINCIPLES. WH Freeman and Co, 1983
- **KOHLER et al.** *Nature,* 1985, vol. 256, 495-497
- **KOZBOR et al.** *J. Immunol. Methods,* 1985, vol. 81, 31-42
- **COTE et al.** *Proc. Natl. Acad. Sci.,* 1983, vol. 80, 2026-2030
- **COLE et al.** *Mol. Cell Biol.,* 1984, vol. 62, 109-120
- **MORRISON et al.** *Proc. Natl. Acad. Sci.,* 1984, vol. 81, 6851-6855
- **NEUBERGER et al.** *Nature,* 1984, vol. 312, 604-608
- **TAKEDA et al.** *Nature,* 1985, vol. 314, 452-454
- **BURTON.** *Proc. Natl. Acad. Sci.,* 1991, vol. 88, 11120-23
- **THIRION et al.** *Eur. J. Cancer Prev.,* 1996, vol. 5, 507-11
- **COLOMA ; MORRISON.** *Nat. Biotechnol.,* 1997, vol. 15, 159-63
- **MALLENDER ; VOSS.** *J. Biol. Chem.,* 1994, vol. 269, 199-206
- **VERHAAR et al.** *Int. J. Cancer,* 1995, vol. 61, 497-501
- **NICHOLLS et al.** *J. Immunol. Meth.,* 1993, vol. 165, 81-91
- **ORLANDI et al.** *Proc. Natl. Acad. Sci.,* 1989, vol. 86, 3833-3837
- **WINTER et al.** *Nature,* 1991, vol. 349, 293-299
- **BROWN.** *Meth. Mol. Biol.,* 1994, vol. 20, 1-8
- **SONVEAUX.** *Meth. Mol. Biol.,* 1994, vol. 26, 1-72
- **UHLMANN et al.** *Chem. Rev.,* 1990, vol. 90, 543-583
- **GEE ; HUBER ; CARR et al.** MOLECULAR AND IMMUNOLOGIC APPROACHES. Futura Publishing Co, 1994
- **AGRAWAL et al.** *Trends Biotechnol.,* 1992, vol. 10, 152-158
- **UHLMANN et al.** *Chem. Rev.,* 1990, vol. 90, 543-584
- **UHLMANN et al.** *Tetrahedron. Lett.,* 1987, vol. 215, 3539-3542
- **CECH.** *Science,* 1987, vol. 236, 1532-1539
- **CECH.** *Ann. Rev. Biochem.,* 1990, vol. 59, 543-568
- **CECH.** *Curr. Opin. Struct. Biol.,* vol. 2, 605-609
- **COUTURE ; STINCHCOMB.** *Trends Genet.,* 1996, vol. 12, 510-515
- **HASELOFF et al.** *Nature,* 1988, vol. 334, 585-591
- **AUSUBEL et al.** CURRENT PROTOCOLS IN MOLECULAR BIOLOGY. John Wiley & Sons, Inc, 1987
- **TEDDER et al.** *Proc. Natl. Acad. Sci. U.S.A.,* 1988, vol. 85, 208-12
- **HEDRICK et al.** *Nature,* vol. 308, 149-53
- **LEE et al.** *Proc. Natl. Acad. Sci. U.S.A.,* 1984, vol. 88, 2825
- **LIANG ; PARDEE.** *Science,* 1992, vol. 257, 967-71
- **LAM.** *Anticancer Drug Des.,* 1997, vol. 12, 145
- **DEWITT et al.** *Proc. Natl. Acad. Sci. U.S.A.,* 1993, vol. 90, 6909
- **ERB et al.** *Proc. Natl. Acad. Sci. U.S.A.,* 1994, vol. 91, 11422
- **ZUCKERMANN et al.** *J. Med. Chem.,* 1994, vol. 37, 2678
- **CHO et al.** *Science,* 1993, vol. 261, 1303
- **CARELL et al.** *Angew. Chem. Int. Ed. Engl.,* 1994, vol. 33, 2059
- **CARELL et al.** *Angew. Chem. Int. Ed. Engl.,* vol. 33, 2061
- **GALLOP et al.** *J. Med. Chem.,* 1994, vol. 37, 1233
- **HOUGHTEN.** *BioTechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc. Natl. Acad. Sci. U.S.A.,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 97, 6378-6382
- **FELICI.** *J. Mol. Biol.,* 1991, vol. 222, 301-310
- **JAYAWICKREME et al.** *Proc. Natl. Acad. Sci. U.S.A.,* 1994, vol. 19, 1614-18
- **CHELSKY.** Strategies for Screening Combinatorial Libraries: Novel and Traditional Approaches. *First Annual Conference of The Society for Biomolecular Screening in Philadelphia,* 07 November 1995
- **SALMON et al.** *Molecular Diversity,* 1996, vol. 2, 57-63
- **MCCONNELL et al.** *Science,* 1992, vol. 257, 1906-1912
- **SJOLANDER ; URBANICZKY.** *Anal. Chem.,* 1991, vol. 63, 2338-2345
- **SZABO et al.** *Curr. Opin. Struct. Biol.,* 1995, vol. 5, 699-705
- **ZERVOS et al.** *Cell,* 1993, vol. 72, 223-232
- **MADURA et al.** *J. Biol. Chem.,* 1993, vol. 268, 12046-12054

- **BARTEL et al.** *BioTechniques,* 1993, vol. 14, 920-924
- **IWABUCHI et al.** *Oncogene,* 1993, vol. 8, 1693-1696
- *Science,* 1989, vol. 246, 181-296
- REMINGTON'S PHARMACEUTICAL SCIENCES. Maack Publishing Co,
- **SENIOR ; SHAPIRO.** Pulmonary Diseases and Disorders. McGraw-Hill, 1998, 659-681
- **BARNES.** *Chest,* 2000, vol. 117, 10S-14S
- **YERXA JOHNSON.** *Drugs of the Future,* 1999, vol. 24, 759-769
- **GERGEN ; WEISS.** *Am. Rev. Respir. Dis.,* 1992, vol. 146, 823-24
- **GOODMAN ; GILMAN'S.** THE PHARMACOLOGIC BASIS OF THERAPEUTICS. MacMillan Publishing Company, 1985
- **CHU ; SHAROM.** *Cell. Immunol.,* 1992, vol. 145, 223-39
- **ALEXANDER et al.** *Lancet,* 1992, vol. 339, 324-28
- **ZAV'YALOV et al.** *Immunol. Lett.,* 1992, vol. 31, 285-88
- **YANG M. ; SRIKAIATKHACHOM A ; ANTONY M. ; CHONG B.H.** *Blood Coagul. Fibrinolysis,* 1996, 127-33
- **ARAI H. ; TSOU C.L. ; CHARO I.F.** *Proc. Natl. Acad. Sci. USA,* 1997, vol. 94, 14495-14499
- **ARAGAY A.M. ; QUICK M.W.** *J. Biol. Chem.,* 1999, vol. 274, 4807-4815
- **DAVIGNON I. ; CATALINA M.D. ; SMITH D. ; MONTGOMERY J. ; CROY J. ; SIEGELMAN M. ; WILKIE T.M.** *Mol. Cell. Biol.,* 2000, vol. 20, 797-804
- **WIESMANN A. ; SPANGRUDE G.J.** *Exp. Hematol.,* 1999, vol. 27, 946-955
- **VAN BROCKLYN J.R ; GRALER M.H. ; BERNHARDT G. ; HOBSON J.P. ; LIPP M. ; SPIEGEL S.** *Blood,* 2000, vol. 95, 2624-2629
- **BRASS L.F.** *J. Clin. Invest.,* 1999, vol. 104, 1663-1665
- **COUGHLIN S.R.** *Proc. Natl. Acad. Sci. USA,* 1999, vol. 96, 11023-11027
- **KOJIMA et al.** *J Hepatol,* January 2000, vol. 32 (1), 43-50
- **PINZANI et al.** *Semin Liver Dis,* 1999, vol. 19 (4), 397-410
- **CAI et al.** *Anticancer Res,* vol. 19 (3B), 2243-7
- **WIRTH et al.** *Eur J Pharmacol,* 15 October 1997, vol. 337 (1), 45-53
- **GATTA et al.** *Ital J Gastroenterol Hepatol,* May 1999, vol. 31 (4), 326-45
- **ALBRECHT et al.** *Drug Targeting,* 1998, vol. 6 (2), 105-117
- **RAMOS et al.** *Pathol Int,* September 1994, vol. 44 (9), 655-61
- **HIDALGO et al.** *J. Autonom. Pharmacol.,* 1998, vol. 18 (1), 31-37
- **FINDEIS et al.** *Trends in Biotechnol.,* 1993, vol. 11, 202-05
- **CHIOU et al.** GENE THERAPEUTICS: METHODS AND APPLICATIONS OF DIRECT GENE TRANSFER. 1994
- **WU ; WU.** *J. Biol. Chem.,* 1988, vol. 263, 621-24
- **WU et al.** *J. Biol. Chem.,* 1994, vol. 269, 542-46
- **ZENKE et al.** *Proc. Natl. Acad. Sci. U.S.A.,* 1990, vol. 87, 3655-59
- **WU et al.** *J. Biol. Chem.,* 1991, vol. 266, 338-42
- **MYERS et al.** *Science,* 1985, vol. 230, 1242
- **COTTON et al.** *Proc. Natl. Acad Sci. USA,* 1985, vol. 85, 4397-4401
- **BUSH et al.** *J. Neurochem.,* 1991, vol. 57, 562-74
- **KNUHTSEN et al.** *Biochem. J.,* 1988, vol. 254, 641-647
- **CUATRECASAS.** *Proc. Natl. Acad. Sci. USA,* 1972, vol. 69, 318-322
- **BRUNS et al.** *Analytical Biochem.,* 1983, vol. 132, 74-81
- **PAUL ; SAID.** *Peptides,* 1986, vol. 7, 147-149
- **CHIRGWIN et al.** *Biochem.,* 1979, vol. 18, 5294-99
- **KLAPDOR et al.** A low cost method to analyze footprint patterns. *J. Neurosci. Methods,* 1997, vol. 75, 49-54
- **HIGUCHI et al.** *BioTechnology,* 1992, vol. 10, 413-17
- **HIGUCHI et al.** *BioTechnology,* 1993, vol. 11, 1026-30
- **HOLLAND et al.** *Proc. Natl. Acad. Sci. U.S.A.,* 1991, vol. 88, 7276-80
- **HEID et al.** *Genome Res.,* 1996, vol. 6, 986-94
- **GIBSON et al.** *Genome Res.,* 1996, vol. 6, 995-1001

SEQUENCE LISTING

<110> Bayer AG

<120> REGULATION OF HUMAN G PROTEIN-COUPLED RECEPTOR

<130> Lio 302

<150> USSN 60/269,410
<151> 2001-02-20

<150> USSN 60/323,809
<151> 2001-09-21

<160> 6

<170> PatentIn version 3.1

<210> 1
<211> 990
<212> DNA
<213> Homo sapiens

<400> 1

atgacgccca acagcactgg cgaggtgccc agccccattc ccaagggggc tttggggctc 60

tccctggccc tggcaagcct catcatcacc gcgaacctgc tcctagccct gggcatcgcc 120

tgggaccgcc gcctgcgcag cccacctgct ggctgcttct tcctgagcct actgctggct 180

gggctgctca cgggtctggc attgcccaca ttgccagggc tgtggaacca gagtcgccgg 240

ggttactggt cctgcctcct cgtctacttg gctcccaact tctccttcct ctccctgctt 300

gccaacctct tgctggtgca cggggagcgc tacatggcag tcctgaggcc actccagccc 360

cctgggagca ttcggctggc cctgctcctc acctgggctg gtcccctgct ctttgccagt 420

ctgcccgctc tggggtggaa ccactggacc cctggtgcca actgcagctc ccaggctatc 480

ttcccagccc cctacctgta cctcgaagtc tatgggctcc tgctgcccgc cgtgggtgct 540

gctgccttcc tctctgtccg cgtgctggcc actgcccacc gccagctgca ggacatctgc 600

cggctggagc gggcagtgtg ccgcgatgag ccctccgccc tggcccgggc ccttacctgg 660

aggcaggcaa gggcacaggc tggagccatg ctgctcttcg ggctgtgctg ggggccctac 720

gtggccacac tgctcctctc agtcctggcc tatgagcagc gcccgccact ggggcctggg 780

acactgttgt ccctcctctc cctaggaagt gccagtgcag cggcagtgcc cgtagccatg 840

gggctgggcg atcagcgcta cacagccccc tggagggcag ccgcccaaag gtgcctgcag 900

gggctgtggg gaagagcctc ccgggacagt cccggcccca gcattgccta ccacccaagc 960

agccaaagca gtgtcgacct ggacttgaac 990

<210> 2
<211> 330
<212> PRT
<213> Homo sapiens

<400> 2

```
Met Thr Pro Asn Ser Thr Gly Glu Val Pro Ser Pro Ile Pro Lys Gly
1               5                   10                  15

Ala Leu Gly Leu Ser Leu Ala Leu Ala Ser Leu Ile Ile Thr Ala Asn
            20                  25                  30

Leu Leu Leu Ala Leu Gly Ile Ala Trp Asp Arg Arg Leu Arg Ser Pro
        35                  40                  45

Pro Ala Gly Cys Phe Phe Leu Ser Leu Leu Leu Ala Gly Leu Leu Thr
    50                  55                  60

Gly Leu Ala Leu Pro Thr Leu Pro Gly Leu Trp Asn Gln Ser Arg Arg
65                  70                  75                  80

Gly Tyr Trp Ser Cys Leu Leu Val Tyr Leu Ala Pro Asn Phe Ser Phe
                85                  90                  95
```

Leu Ser Leu Leu Ala Asn Leu Leu Leu Val His Gly Glu Arg Tyr Met
100 105 110

Ala Val Leu Arg Pro Leu Gln Pro Pro Gly Ser Ile Arg Leu Ala Leu
115 120 125

Leu Leu Thr Trp Ala Gly Pro Leu Leu Phe Ala Ser Leu Pro Ala Leu
130 135 140

Gly Trp Asn His Trp Thr Pro Gly Ala Asn Cys Ser Ser Gln Ala Ile
145 150 155 160

Phe Pro Ala Pro Tyr Leu Tyr Leu Glu Val Tyr Gly Leu Leu Leu Pro
165 170 175

Ala Val Gly Ala Ala Ala Phe Leu Ser Val Arg Val Leu Ala Thr Ala
180 185 190

His Arg Gln Leu Gln Asp Ile Cys Arg Leu Glu Arg Ala Val Cys Arg
195 200 205

Asp Glu Pro Ser Ala Leu Ala Arg Ala Leu Thr Trp Arg Gln Ala Arg
210 215 220

Ala Gln Ala Gly Ala Met Leu Leu Phe Gly Leu Cys Trp Gly Pro Tyr
225 230 235 240

Val Ala Thr Leu Leu Leu Ser Val Leu Ala Tyr Glu Gln Arg Pro Pro
245 250 255

Leu Gly Pro Gly Thr Leu Leu Ser Leu Leu Ser Leu Gly Ser Ala Ser
260 265 270

Ala Ala Ala Val Pro Val Ala Met Gly Leu Gly Asp Gln Arg Tyr Thr
275 280 285

Ala Pro Trp Arg Ala Ala Ala Gln Arg Cys Leu Gln Gly Leu Trp Gly
290 295 300

Arg Ala Ser Arg Asp Ser Pro Gly Pro Ser Ile Ala Tyr His Pro Ser
305 310 315 320

Ser Gln Ser Ser Val Asp Leu Asp Leu Asn
325 330

<210> 3

<211> 437
<212> DNA
<213> Homo sapiens

<400> 3

```
acctgggccc cgcttctata gaaggtattt tagccaaaac tggacccgcg gctatgagca     60
gcgcccgcca ctggggcctg ggacactgtt gtccctcctc tccctaggaa gtgccagtgc    120
agcggcagtg cccgtagcca tggggctggg cgatcagcgc tacacagccc cctggagggc    180
agccgcccaa aggtgcctgc aggggctgtg gggaagagcc tcccgggaca gtcccggccc    240
cagcattgcc taccacccaa gcagccaaag cagtgtcgac ctggacttga actaaaggaa    300
gggcctctgc tgactcctac cagagcatcc gtccagctca gccatccagc ctgtctctac    360
tgggccccac ttctctggat cagagaccct gcctctgttt gaccccgcac tgactgaata    420
aagctcctca taggccg                                                   437
```

<210> 4
<211> 456
<212> DNA
<213> Homo sapiens

<400> 4

```
ggcctggccc cgctgtccct actgggtgga gacaccatgt acttggtcca cttgtgctct     60
tcagccagga caccagacat ggtccaaacc gctgcagggc tggctgcagc aactccctga    120
cactcaggaa ggcccaggct gggcaggcaa tacctgctcc caacagccat gcatgccggc    180
tgccgctcca ggactcccct gtccccagga ccaagatgac gcccaacagc actggcgagg    240
tgcccagccc cattcccaag gggctttgg ggctctccct ggccctggca agcctcatca     300
tcaccgcgaa cctgctccta gccctgggca tcgcctggga ccgccgcctg cgcagcccac    360
ctgctggctg cttcttcctg agcctactgc tggctgggct gctcacgggt ctggcattgc    420
ccacattgcc agggctgtgg aaccagagtc gccggg                              456
```

<210> 5
<211> 434
<212> DNA
<213> Homo sapiens

<220>
<221> misc_feature
<222> (391).. (392)
<223> n=a,t,g or c

<400> 5

tttttaacgg ccagaggagc tttattcagt cagtgcgggg tcaaacagag gcagggtctc 60

tgatccagag aagtggggcc cagtagagac aggctggatg gctgagctgg acggatgctc 120

tggtaggagt cagcagaggc ccttccttta gttcaagtcc aggtcgacac tgctttggct 180

gcttgggtgg taggcaatgc tggggccggg actgtcccgg gaggctcttc cccacagccc 240

ctgcaggcac ctttgggcgg ctgccctcca gggggctgtg tagcgctgat cgcccagccc 300

catggctacg ggcactgccg ctgcactggc acttcctagg gagaggaggg acaacagtgt 360

cccaggcccc agtggcgtca tcttggtcct gnggacaggg gagtcctgga gcggcagccg 420

gcatgcatgg ctgt 434

<210> 6
<211> 221
<212> DNA
<213> Homo sapiens

<400> 6

tatgagagtc tttatctgtt tcaccccggt gagagcattt tgaatttcct tattgacgta 60

cttacttccc tgattggaca tttcctggag ctcattgtct gagaccgtgt ggtcccccag 120

gactgggcca gtctcgccag gtgaggagca gggccagaaa gaggagcagg gtgttcatca 180

tggctccaat tctggagtct ttgcacgcct cggtcagcgg c 221